(19) Europäisches Patentamt
European Patent Office
Office européen des brevets

(11) EP 4 413 984 A1

(12) **EUROPEAN PATENT APPLICATION**
published in accordance with Art. 153(4) EPC

(43) Date of publication:
**14.08.2024 Bulletin 2024/33**

(21) Application number: **22877949.2**

(22) Date of filing: **08.10.2022**

(51) International Patent Classification (IPC):
***A61K 31/40*** *(2006.01)* ***A61P 31/12*** *(2006.01)*
***A61K 31/513*** *(2006.01)* ***A61K 31/675*** *(2006.01)*

(52) Cooperative Patent Classification (CPC):
**A61K 31/40; A61K 31/513; A61K 31/675; A61P 31/12**

(86) International application number:
**PCT/CN2022/123799**

(87) International publication number:
**WO 2023/056933 (13.04.2023 Gazette 2023/15)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **08.10.2021 CN 202111172126**

(71) Applicant: **CHIA TAI TIANQING PHARMACEUTICAL GROUP CO., LTD.**
**Lianyungang,**
**Jiangsu 222062 (CN)**

(72) Inventors:
- **XU, Zhongnan**
  **Lianyungang Jiangsu 222062 (CN)**
- **HUO, Dandan**
  **Lianyungang Jiangsu 222062 (CN)**
- **ZHANG, Hong**
  **Lianyungang Jiangsu 222062 (CN)**
- **YU, Yanan**
  **Lianyungang Jiangsu 222062 (CN)**
- **WANG, Xiang**
  **Lianyungang Jiangsu 222062 (CN)**

(74) Representative: **Potter Clarkson**
**Chapel Quarter**
**Mount Street**
**Nottingham NG1 6HQ (GB)**

# (54) PHARMACEUTICAL COMBINATION CONTAINING CAPSID PROTEIN INHIBITOR AND REVERSE TRANSCRIPTASE INHIBITOR

(57) The present invention belongs to the field of medicinal chemistry, and relates to a pharmaceutical combination containing a capsid protein inhibitor or a pharmaceutically acceptable salt thereof and a reverse transcriptase inhibitor or a pharmaceutically acceptable prodrug thereof, or a pharmaceutically acceptable salt or a solvate thereof. Specifically, the present invention relates to a pharmaceutical combination of a capsid protein inhibitor or a pharmaceutically acceptable salt thereof and entecavir or tenofovir, or a pharmaceutically acceptable prodrug thereof, or a pharmaceutically acceptable salt or a solvate thereof, or the use thereof in the treatment of hepatitis B virus infection. The pharmaceutical combination has a good anti-hepatitis B virus infection effect.

EP 4 413 984 A1

## Description

### CROSS-REFERENCE TO RELATED APPLICATIONS

The present application claims priority and benefit to the Chinese Patent Application No. 202111172126.3 filed with the National Intellectual Property Administration, PRC on October 8, 2021, the disclosure of which is incorporated herein by reference in its entirety.

### TECHNICAL FIELD

The present application belongs to the field of medicinal chemistry, and relates to a pharmaceutical combination comprising a capsid protein inhibitor and a reverse transcriptase inhibitor. In particular, the present application relates to a pharmaceutical combination of a compound of formula I as a capsid protein inhibitor or a pharmaceutically acceptable salt thereof and entecavir or tenofovir, or a pharmaceutically acceptable prodrug thereof, a pharmaceutically acceptable salt thereof or a solvate thereof, or use thereof for treating hepatitis B virus infection.

### BACKGROUND

According to the World Health Organization statistics, about 257 million people worldwide are infected with the hepatitis B virus (HBV). If not well treated, patients with hepatitis will be confronted with long-term fatal diseases such as liver failure, cirrhosis, liver cancer, and the like.

In patients infected with HBV, stable covalently closed circular DNA, i.e., cccDNA, is formed within the host's hepatocyte nucleus, serving as a template for HBV's continuous replication. Subgenomic RNA (sgRNA) and pregenomic RNA (pgRNA) are all formed by transcription of cccDNA. After exiting the nucleus, sgRNA is translated into protein X and three other envelope proteins, while pgRNA is translated into core protein and viral polymerase. Under the action of the polymerase, pgRNA self-assembles with the core protein to form nucleocapsid-encapsulated RNA. Within the nucleocapsid, pgRNA is reverse-transcribed into a negative strand of DNA, from which a positive strand of DNA is further synthesized, forming rcDNA. The nucleocapsid-encapsulated rcDNA, on one hand, re-shells and enters the cell nucleus to further amplify cccDNA and, on the other hand, re-binds to the envelope protein and is released from the cell through the endoplasmic reticulum, forming new HBV In the replication cycle of HBV, the synthesis of nucleocapsid is a key step in the replication process of HBV genome, and the synthesis of viral DNA can only occur specifically within the nucleocapsid. The assembly of the nucleocapsid is an evolutionary constraint process that limits the diversity of HBV, and is very sensitive to even subtle molecular disturbances. For the development of new therapies against different genotypes and drug-resistant strains of hepatitis B virus, the targets acting on the synthesis and degradation processes of the nucleocapsid are very promising.

Conventional therapies currently approved for the treatment of chronic hepatitis B include nucleos(t)ide compounds and interferons. Nucleos(t)ide drugs, such as entecavir and tenofovir, can inhibit HBV DNA replication.

Although patients infected with the hepatitis B virus have many treatment options, there is still a need for more effective therapeutic agents for clinical use.

### SUMMARY

In one aspect, the present application provides a pharmaceutical combination, comprising a capsid protein inhibitor or a pharmaceutically acceptable salt thereof and one or more additional therapeutic agents.

The capsid protein inhibitor or the pharmaceutically acceptable salt thereof described herein is selected from the group consisting of compounds and pharmaceutically acceptable salts thereof in WO2019185016, WO2019165374, WO2019241292, WO2021119081, or WO2020156494. The capsid protein inhibitor or the pharmaceutically acceptable salt thereof described herein is selected from the group consisting of specific compounds and pharmaceutically acceptable salts thereof in WO2019185016, WO2019165374, WO2019241292, WO2021119081, or WO2020156494.

The one or more additional therapeutic agents described herein are selected from the group consisting of nucleotide compounds and interferons. The one or more additional therapeutic agents described herein are selected from the group consisting of reverse transcriptase inhibitors and interferons.

The one or more additional therapeutic agents described herein are selected from the group consisting of reverse transcriptase inhibitors (nucleotide compounds) and interferons (e.g., short-acting interferons or long-acting interferons, such as interferon $\alpha$1b, interferon $\alpha$2a, and interferon $\alpha$2b).

The one or more additional therapeutic agents described herein are selected from the group consisting of reverse transcriptase inhibitors (nucleotide compounds).

In some embodiments, the reverse transcriptase inhibitor is selected from the group consisting of entecavir,

tenofovir, lamivudine, telbivudine, adefovir, clevudine, CMX157, AGX-1009, zidovudine, didanosine, zalcitabine, stavudine, emtricitabine, abacavir, D-D4FC, alovudine, amdoxovir, elvucitabine, delavirdine, efavirenz, nevirapine, capravirine, calanolide A, TMC278, BMS-561390 and DPC-083, and pharmaceutically acceptable prodrugs thereof, pharmaceutically acceptable salts thereof and solvates thereof.

In some embodiments, the reverse transcriptase inhibitor is selected from the group consisting of entecavir and tenofovir, pharmaceutically acceptable prodrugs thereof, pharmaceutically acceptable salts thereof, and solvates thereof. In some embodiments, the reverse transcriptase inhibitor is selected from the group consisting of entecavir, a pharmaceutically acceptable salt thereof, and a solvate thereof. In some embodiments, the reverse transcriptase inhibitor is selected from the group consisting of tenofovir, a pharmaceutically acceptable prodrug thereof, a pharmaceutically acceptable salt thereof, and a solvate thereof. In a further aspect, the present application provides a pharmaceutical combination, comprising the capsid protein inhibitor or the pharmaceutically acceptable salt thereof described herein, as well as

1) entecavir or a pharmaceutically acceptable salt thereof or a solvate thereof; or/and

2) tenofovir or a pharmaceutically acceptable prodrug thereof or a pharmaceutically acceptable salt thereof.

In another aspect, the present application further provides use of the pharmaceutical combination of the present application for preparing a medicament for treating hepatitis B virus infection. The present application further provides a method for treating hepatitis B virus infection, comprising administering to an individual in need an effective amount of the pharmaceutical combination of the present application. The present application further provides a pharmaceutical combination for use in treating hepatitis B virus infection. The present application further provides use of the pharmaceutical combination of the present application for treating hepatitis B virus infection.

In some embodiments of the present application, the pharmaceutical combination described herein comprises the capsid protein inhibitor or the pharmaceutically acceptable salt thereof described herein and entecavir or a pharmaceutically acceptable salt thereof or a hydrate thereof. In some embodiments, the pharmaceutical combination described herein comprises the capsid protein inhibitor or the pharmaceutically acceptable salt thereof described herein and entecavir or a monohydrate thereof. In some embodiments, the pharmaceutical combination described herein comprises the capsid protein inhibitor or the pharmaceutically acceptable salt thereof described herein and entecavir monomaleate monohydrate.

In some embodiments of the present application, the pharmaceutical combination described herein comprises the capsid protein inhibitor or the pharmaceutically acceptable salt thereof described herein and tenofovir or a pharmaceutically acceptable prodrug thereof or a pharmaceutically acceptable salt thereof. In some embodiments, the pharmaceutical combination described herein comprises the capsid protein inhibitor or the pharmaceutically acceptable salt thereof described herein and tenofovir alafenamide or tenofovir disoproxil or a pharmaceutically acceptable salt thereof. In some embodiments, the pharmaceutical combination described herein comprises the capsid protein inhibitor or the pharmaceutically acceptable salt thereof described herein and tenofovir disoproxil fumarate or tenofovir alafenamide fumarate.

In some embodiments of the present application, the capsid protein inhibitor or the pharmaceutically acceptable salt thereof described herein in the pharmaceutical combination can be administered once every day, twice every day, thrice every day, once every two days, once every three days, once every four days, once every five days, once every six days, once every week, once every two weeks, or once every three weeks.

In some embodiments of the present application, the capsid protein inhibitor or the pharmaceutically acceptable salt thereof described herein in the pharmaceutical combination is administered at a dose of 10-500 mg, 10-100 mg, or 10-50 mg (calculated based on the weight of a free compound) each time.

In some embodiments of the present application, each dose of the capsid protein inhibitor or the pharmaceutically acceptable salt thereof described herein in the pharmaceutical combination is a dose of 10-500 mg, 10-100 mg, or 10-50 mg (calculated based on the weight of a free compound).

In some embodiments of the present application, the capsid protein inhibitor or the pharmaceutically acceptable salt thereof described herein in the pharmaceutical combination is administered at a dose of 0.0001-20 mg/kg body weight (calculated based on the weight of a free compound) each time.

In some embodiments of the present application, each dose of the capsid protein inhibitor or the pharmaceutically acceptable salt thereof described herein in the pharmaceutical combination is 0.0001-20 mg/kg body weight (calculated based on the weight of a free compound).

In some embodiments of the present application, the capsid protein inhibitor or the pharmaceutically acceptable salt thereof described herein in the pharmaceutical combination is administered at a dose of 1-2000 mg, 2-1500 mg, 2-1000 mg, 3-800 mg, 3-600 mg, 4-550 mg, 5-500 mg, 5-450 mg, 5-400 mg, 5-480 mg, 5-460 mg, 5-450 mg, 5-430 mg, 6-400 mg, 7-380 mg, 8-360 mg, 9-340 mg, or 10-320 mg each time.

In some embodiments of the present application, each dose of the capsid protein inhibitor or the pharmaceutically acceptable salt thereof described herein in the pharmaceutical combination is 1-2000 mg, 2-1500 mg, 2-1000 mg, 3-800 mg, 3-600 mg, 4-550 mg, 5-500 mg, 5-450 mg, 5-400 mg, 5-480 mg, 5-460 mg, 5-450 mg, 5-430 mg, 6-400 mg, 7-380 mg, 8-360 mg, 9-340 mg, or 10-320 mg.

In some embodiments of the present application, in the pharmaceutical combination, the capsid protein inhibitor or the pharmaceutically acceptable salt thereof described herein is administered at a dose of 1-1500 mg or 10-1000 mg (calculated based on the weight of a free compound) each time, and the average daily dose of tenofovir or the pharmaceutically acceptable prodrug thereof or the pharmaceutically acceptable salt thereof described herein is 0.05 mg to 500 mg (calculated based on the weight of tenofovir).

In some embodiments of the present application, in the pharmaceutical combination, each dose of the capsid protein inhibitor or the pharmaceutically acceptable salt thereof described herein is 1-1500 mg or 10-1000 mg (calculated based on the weight of a free compound), and the average daily dose of tenofovir or the pharmaceutically acceptable prodrug thereof or the pharmaceutically acceptable salt thereof described herein is 0.05 mg to 500 mg (calculated based on the weight of tenofovir).

In some embodiments of the present application, in the pharmaceutical combination, the average daily dose ratio of the capsid protein inhibitor or the pharmaceutically acceptable salt thereof described herein to tenofovir or the pharmaceutically acceptable prodrug thereof or the pharmaceutically acceptable salt thereof described herein (calculated based on the weight of corresponding free compounds, the capsid protein inhibitor compound:tenofovir) is selected from 500:1 to 1:1000. In some embodiments, the average daily dose ratio of the capsid protein inhibitor or the pharmaceutically acceptable salt thereof described herein to tenofovir or the pharmaceutically acceptable prodrug thereof or the pharmaceutically acceptable salt thereof described herein (calculated based on the weight of corresponding free compounds) is selected from the group consisting of 500:1, 450:1, 400:1, 350:1, 300:1, 250:1, 200:1, 150:1, 100:1, 95:1, 90:1, 85:1, 80:1, 75:1, 70:1, 65:1, 60:1, 55:1, 50:1, 45:1, 40:1, 39:1, 38:1, 37:1, 36:1, 35:1, 34:1, 33:1, 32:1, 31:1, 30:1, 29:1, 28:1, 27:1, 26:1, 25:1, 24:1, 23:1, 22:1, 21:1, 20:1, 19:1, 18:1, 17:1, 16:1, 15:1, 10:1, 5:1, 4:1, 3:1, 2:1, 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90, 1:100, 1:150, 1:200, 1:250, 1:300, 1:350, 1:400, 1:450, 1:500, 1:550, 1:600, 1:650, 1:700, 1:750, 1:800, 1:850, 1:900, 1:950 and 1:1000 or from a range formed by any of the above ratios.

In some embodiments of the present application, in the pharmaceutical combination, the average daily dose ratio of the capsid protein inhibitor or the pharmaceutically acceptable salt thereof described herein to tenofovir or the pharmaceutically acceptable prodrug thereof or the pharmaceutically acceptable salt thereof described herein (calculated based on the molar ratio of free compounds) is selected from 100:1 to 1:100. In some embodiments, the average daily dose ratio of the capsid protein inhibitor or the pharmaceutically acceptable salt thereof described herein to tenofovir or the pharmaceutically acceptable prodrug thereof or the pharmaceutically acceptable salt thereof described herein (calculated based on the molar ratio of free compounds, the capsid protein inhibitor compound:tenofovir) is selected from the group consisting of: 100:1, 98:1, 96:1, 94:1, 92:1, 90:1, 88:1, 86:1, 84:1, 82:1, 80:1, 78:1, 76:1, 74:1, 72:1, 70:1, 68:1, 66:1, 64:1, 62:1, 60:1, 58:1, 56:1, 54:1, 52:1, 50:1, 48:1, 46:1, 44:1, 42:1, 40:1, 38:1, 36:1, 34:1, 32:1, 30:1, 28:1, 26:1, 25:1, 24:1, 23:1, 22:1, 21:1, 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:12, 1:14, 1:15, 1:16, 1:17, 1:18, 1:19, 1:20, 1:21, 1:22, 1:23, 1:24, 1:25, 1:26, 1:27, 1:28, 1:29, 1:30, 1:31, 1:32, 1:33, 1:34, 1:35, 1:36, 1:38, 1:40, 1:42, 1:44, 1:46, 1:48, 1:50, 1:52, 1:54, 1:56, 1:58, 1:60, 1:62, 1:64, 1:66, 1:68, 1:70, 1:72, 1:74, 1:76, 1:78, 1:80, 1:82, 1:84, 1:86, 1:88, 1:90, 1:92, 1:94, 1:96, 1:98 and 1:100 or from a range formed by any of the above ratios. In some embodiments of the present application, in the pharmaceutical combination, the average daily dose ratio (calculated based on weight) of the capsid protein inhibitor or the pharmaceutically acceptable salt thereof described herein (with the capsid protein inhibitor compound calculated as a free compound) to tenofovir disoproxil fumarate is selected from the group consisting of 1:300 to 1500:300 and 10:300 to 1000:300. In some embodiments, in the pharmaceutical combination, the average daily dose ratio (calculated based on weight) of the capsid protein inhibitor or the pharmaceutically acceptable salt thereof described herein (with the capsid protein inhibitor compound calculated as a free compound) to tenofovir disoproxil fumarate is selected from the group consisting of 10:300, 20:300, 30:300, 40:300, 50:300, 60:300, 70:300, 80:300, 90:300, 100:300, 110:300, 120:300, 130:300, 140:300, 150:300, 160:300, 170:300, 180:300, 190:300, 200:300, 210:300, 220:300, 230:300, 240:300, 250:300, 260:300, 270:300, 280:300, 290:300, 300:300, 310:300, 320:300, 330:300, 340:300, 350:300, 360:300, 370:300, 380:300, 390:300, 400:300, 410:300, 420:300, 430:300, 440:300, 450:300, 460:300, 470:300, 480:300, 490:300, 500:300, 510:300, 520:300, 530:300, 540:300, 550:300, 560:300, 570:300, 580:300, 590:300, 600:300, 610:300, 620:300, 630:300, 640:300, 650:300, 660:300, 670:300, 680:300, 690:300, 700:300, 710:300, 720:300, 730:300, 740:300, 750:300, 760:300, 770:300, 780:300, 790:300 and 800:300 or from a range formed by any of the above ratios.

In some embodiments of the present application, in the pharmaceutical combination, the average daily dose ratio (calculated based on weight) of the capsid protein inhibitor or the pharmaceutically acceptable salt thereof described herein (with the capsid protein inhibitor compound calculated as a free compound) to tenofovir alafenamide is selected

from the group consisting of 1:25 to 1500:25 and 10:25 to 1000:25. In some embodiments, in the pharmaceutical combination, the average daily dose ratio (calculated based on weight) of the capsid protein inhibitor or the pharmaceutically acceptable salt thereof described herein (with the capsid protein inhibitor compound calculated as a free compound) to tenofovir alafenamide is selected from the group consisting of 10:25, 20:25, 30:25, 40:25, 50:25, 60:25, 70:25, 80:25, 90:25, 100:25, 110:25, 120:25, 130:25, 140:25, 150:25, 160:25, 170:25, 180:25, 190:25, 200:25, 210:25, 220:25, 230:25, 240:25, 250:25, 260:25, 270:25, 280:25, 290:25, 300:25, 310:25, 320:25, 330:25, 340:25, 350:25, 360:25, 370:25, 380:25, 390:25, 400:25, 410:25, 420:25, 430:25, 440:25, 450:25, 460:25, 470:25, 480:25, 490:25, 500:25, 510:25, 520:25, 530:25, 540:25, 550:25, 560:25, 570:25, 580:25, 590:25, 600:25, 610:25, 620:25, 630:25, 640:25, 650:25, 660:25, 670:25, 680:25, 690:25, 700:25, 710:25, 720:25, 730:25, 740:25, 750:25, 760:25, 770:25, 780:25, 790:25 and 800:25 or from a range formed by any of the above ratios.

In some embodiments of the present application, in the pharmaceutical combination, the average daily dose ratio (calculated based on weight) of the capsid protein inhibitor or the pharmaceutically acceptable salt thereof described herein (with the capsid protein inhibitor compound calculated as a free compound) to tenofovir alafenamide fumarate is selected from the group consisting of 1:28 to 1500:28 and 10:28 to 1000:28. In some embodiments, in the pharmaceutical combination, the average daily dose ratio (calculated based on weight) of the capsid protein inhibitor or the pharmaceutically acceptable salt thereof described herein (with the capsid protein inhibitor compound calculated as a free compound) to tenofovir alafenamide fumarate is selected from the group consisting of 10:28, 20:28, 30:28, 40:28, 50:28, 60:28, 70:28, 80:28, 90:28, 100:28, 110:28, 120:28, 130:28, 140:28, 150:28, 160:28, 170:28, 180:28, 190:28, 200:28, 210:28, 220:28, 230:28, 240:28, 280:28, 260:28, 270:28, 280:28, 290:28, 300:28, 310:28, 320:28, 330:28, 340:28, 350:28, 360:28, 370:28, 380:28, 390:28, 400:28, 410:28, 420:28, 430:28, 440:28, 450:28, 460:28, 470:28, 480:28, 490:28, 500:28, 510:28, 520:28, 530:28, 540:28, 550:28, 560:28, 570:28, 580:28, 590:28, 600:28, 610:28, 620:28, 630:28, 640:28, 650:28, 660:28, 670:28, 680:28, 690:28, 700:28, 710:28, 720:28, 730:28, 740:28, 750:28, 760:28, 770:28, 780:28, 790:28 and 800:28 or from a range formed by any of the above ratios.

In some embodiments of the present application, the capsid protein inhibitor or the pharmaceutically acceptable salt thereof and tenofovir or the pharmaceutically acceptable prodrug thereof or the pharmaceutically acceptable salt thereof described herein in the pharmaceutical combination are each administered in a single dose or in multiple doses.

In some embodiments of the present application, in the pharmaceutical combination, the capsid protein inhibitor or the pharmaceutically acceptable salt thereof described herein is administered at a dose of 1-1500 mg, 2-1000 mg, 3-800 mg, 3-600 mg, 4-550 mg, 5-500 mg, 5-450 mg, 5-400 mg, 5-480 mg, 5-460 mg, 5-450 mg, 5-430 mg, 6-400 mg, 7-380 mg, 8-360 mg, 9-340 mg, or 10-320 mg each time, and the average daily dose of entecavir or the pharmaceutically acceptable salt thereof or the solvate thereof described herein is 0.005 mg to 10.0 mg.

In some embodiments of the present application, in the pharmaceutical combination, each dose of the capsid protein inhibitor or the pharmaceutically acceptable salt thereof described herein is 1-1500 mg, 2-1000 mg, 3-800 mg, 3-600 mg, 4-550 mg, 5-500 mg, 5-450 mg, 5-400 mg, 5-480 mg, 5-460 mg, 5-450 mg, 5-430 mg, 6-400 mg, 7-380 mg, 8-360 mg, 9-340 mg, or 10-320 mg each time, and the average daily dose of entecavir or the pharmaceutically acceptable salt thereof or the solvate thereof described herein is 0.005 mg to 10.0 mg.

In some embodiments of the present application, in the pharmaceutical combination, the average daily dose ratio of the capsid protein inhibitor or the pharmaceutically acceptable salt thereof described herein to entecavir or the pharmaceutically acceptable salt thereof or the solvate thereof described herein (calculated based on the weight of free compounds) is selected from 1000:1 to 1:1000. In some embodiments, the average daily dose ratio of the capsid protein inhibitor or the pharmaceutically acceptable salt thereof described herein to entecavir or the pharmaceutically acceptable salt thereof or the solvate thereof described herein (calculated based on the weight of free compounds, the capsid protein inhibitor compound:entecavir) is selected from the group consisting of 1000:1, 950:1, 900:1, 850:1, 800:1, 750:1, 700:1, 650:1, 640:1, 630:1, 620:1, 610:1, 600:1, 550:1, 500:1, 450:1, 400:1, 350:1, 300:1, 250:1, 200:1, 150:1, 100:1, 90:1, 80:1, 70:1, 60:1, 50:1, 40:1, 30:1, 20:1, 10:1, 1:0.01, 1:0.02, 1:0.03, 1:0.04, 1:0.05, 1:0.06, 1:0.07, 1:0.08, 1:0.09, 1:0.1, 1:0.5, 1:0.6, 1:0.7, 1:0.8, 1:0.9, 1:1, 1:10, 1:20, 1:30, 1:40, 1:50, 1:60, 1:70, 1:80, 1:90, 1:100, 1:150, 1:200, 1:250, 1:300, 1:350, 1:400, 1:450, 1:500, 1:550, 1:600, 1:650, 1:700, 1:750, 1:800, 1:850, 1:900, 1:950 and 1:1000 or from a range formed by any of the above ratios.

In some embodiments of the present application, in the pharmaceutical combination, the average daily dose ratio of the capsid protein inhibitor or the pharmaceutically acceptable salt thereof described herein to entecavir or the pharmaceutically acceptable salt thereof or the solvate thereof described herein (calculated based on the molar ratio of free compounds) is selected from 300:1 to 1:100. In some embodiments, the average daily dose ratio of the capsid protein inhibitor or the pharmaceutically acceptable salt thereof described herein to entecavir or the pharmaceutically acceptable salt thereof or the solvate thereof described herein (calculated based on the molar ratio of free compounds, the capsid protein inhibitor compound:entecavir) is selected from the group consisting of: 300:1, 290:1, 280:1, 270:1, 260:1, 250:1, 240:1, 235:1, 230:1, 225:1, 220:1, 215:1, 210:1, 205:1, 200:1, 190:1, 180:1, 170:1, 160:1, 150:1, 140:1, 130:1, 120:1, 110:1, 100:1, 98:1, 96:1, 94:1, 92:1, 90:1, 88:1, 86:1, 84:1, 82:1, 80:1, 78:1, 76:1, 74:1, 72:1, 70:1, 68:1, 66:1, 64:1, 62:1, 60:1, 58:1, 56:1, 54:1, 52:1, 50:1, 48:1, 46:1, 44:1, 42:1, 40:1, 38:1, 36:1, 34:1, 32:1, 30:1, 28:1, 26:1,

24:1, 22:1, 20:1, 18:1, 16:1, 14:1, 12:1, 10:1, 8:1, 6:1, 4:1, 2:1, 1:1, 1:2, 1:4, 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24, 1:25, 1:26, 1:27, 1:28, 1:29, 1:30, 1:32, 1:34, 1:36, 1:38, 1:40, 1:42, 1:44, 1:46, 1:48, 1:50, 1:52, 1:54, 1:56, 1:58, 1:60, 1:62, 1:64, 1:66, 1:68, 1:70, 1:72, 1:74, 1:76, 1:78, 1:80, 1:82, 1:84, 1:86, 1:88, 1:90, 1:92, 1:94, 1:96, 1:98 and 1:100 or from a range formed by any of the above ratios.

In some embodiments of the present application, in the pharmaceutical combination, the average daily dose ratio (calculated based on weight) of the capsid protein inhibitor or the pharmaceutically acceptable salt thereof described herein (with the capsid protein inhibitor compound calculated as a free compound) to entecavir monohydrate is selected from the group consisting of 1:0.5 to 1500:0.5 and 10:0.5 to 1000:0.5. In some embodiments, in the pharmaceutical combination, the average daily dose ratio (calculated based on weight) of the capsid protein inhibitor or the pharmaceutically acceptable salt thereof described herein (with the capsid protein inhibitor compound calculated as a free compound) to entecavir monohydrate is selected from the group consisting of 10:0.5, 20:0.5, 30:0.5, 40:0.5, 50:0.5, 60:0.5, 70:0.5, 80:0.5, 90:0.5, 100:0.5, 110:0.5, 120:0.5, 130:0.5, 140:0.5, 150:0.5, 160:0.5, 170:0.5, 180:0.5, 190:0.5, 200:0.5, 210:0.5, 220:0.5, 230:0.5, 240:0.5, 250:0.5, 260:0.5, 270:0.5, 280:0.5, 290:0.5, 300:0.5, 310:0.5, 320:0.5, 330:0.5, 340:0.5, 350:0.5, 360:0.5, 370:0.5, 380:0.5, 390:0.5, 400:0.5, 410:0.5, 420:0.5, 430:0.5, 440:0.5, 450:0.5, 460:0.5, 470:0.5, 480:0.5, 490:0.5, 500:0.5, 510:0.5, 520:0.5, 530:0.5, 540:0.5, 550:0.5, 560:0.5, 570:0.5, 580:0.5, 590:0.5, 600:0.5, 610:0.5, 620:0.5, 630:0.5, 640:0.5, 650:0.5, 660:0.5, 670:0.5, 680:0.5, 690:0.5, 700:0.5, 710:0.5, 720:0.5, 730:0.5, 740:0.5, 750:0.5, 760:0.5, 770:0.5, 780:0.5, 790:0.5 and 800:0.5 or from a range formed by any of the above ratios.

In some embodiments of the present application, the capsid protein inhibitor or the pharmaceutically acceptable salt thereof and the one or more additional therapeutic agents described herein in the pharmaceutical combination are each administered in a single dose or in multiple doses.

In some embodiments of the present application, the capsid protein inhibitor or the pharmaceutically acceptable salt thereof and entecavir or the pharmaceutically acceptable salt thereof or the solvate thereof described herein in the pharmaceutical combination are each administered in a single dose or in multiple doses.

In some embodiments of the present application, the pharmaceutical combination is a fixed combination. In some embodiments, the fixed combination is in the form of a solid pharmaceutical composition.

In some embodiments, the capsid protein inhibitor or the pharmaceutically acceptable salt thereof and the one or more additional therapeutic agents described herein in the fixed combination are present in the same solid pharmaceutical composition.

In some embodiments, the capsid protein inhibitor or the pharmaceutically acceptable salt thereof and tenofovir or the pharmaceutically acceptable prodrug thereof or the pharmaceutically acceptable salt thereof described herein in the fixed combination are present in the same solid pharmaceutical composition.

In some embodiments, the capsid protein inhibitor or the pharmaceutically acceptable salt thereof and entecavir or the pharmaceutically acceptable salt thereof or the solvate thereof described herein in the fixed combination are present in the same solid pharmaceutical composition.

In some embodiments of the present application, the pharmaceutical combination is a non-fixed combination.

In some embodiments, the capsid protein inhibitor or the pharmaceutically acceptable salt thereof and entecavir or the pharmaceutically acceptable salt thereof or the solvate thereof described herein in the non-fixed combination are each in the form of a solid pharmaceutical composition.

In some embodiments, the capsid protein inhibitor or the pharmaceutically acceptable salt thereof and tenofovir or the pharmaceutically acceptable prodrug thereof or the pharmaceutically acceptable salt thereof described herein in the non-fixed combination are each in the form of a solid pharmaceutical composition.

In some embodiments, the capsid protein inhibitor or the pharmaceutically acceptable salt thereof and tenofovir or the pharmaceutically acceptable prodrug thereof or the pharmaceutically acceptable salt thereof described herein in the non-fixed combination are each in the form of a solid pharmaceutical composition, and the solid pharmaceutical composition of the capsid protein inhibitor or the pharmaceutically acceptable salt thereof and the solid pharmaceutical composition of tenofovir or the pharmaceutically acceptable prodrug thereof or the pharmaceutically acceptable salt thereof described herein are present in the same sachet.

In some embodiments, the capsid protein inhibitor or the pharmaceutically acceptable salt thereof and tenofovir or the pharmaceutically acceptable prodrug thereof or the pharmaceutically acceptable salt thereof described herein in the non-fixed combination are each in the form of a solid pharmaceutical composition, and the solid pharmaceutical composition of the capsid protein inhibitor or the pharmaceutically acceptable salt thereof and the solid pharmaceutical composition of tenofovir or the pharmaceutically acceptable prodrug thereof or the pharmaceutically acceptable salt thereof described herein are not present in the same sachet.

In some embodiments, the capsid protein inhibitor or the pharmaceutically acceptable salt thereof and entecavir or the pharmaceutically acceptable salt thereof or the solvate thereof described herein in the non-fixed combination are each in the form of a solid pharmaceutical composition, and the solid pharmaceutical composition of the capsid protein inhibitor or the pharmaceutically acceptable salt thereof and the solid pharmaceutical composition of entecavir or the pharmaceutically acceptable salt thereof or the solvate thereof described herein are present in the same sachet.

In some embodiments, the capsid protein inhibitor or the pharmaceutically acceptable salt thereof and entecavir or the pharmaceutically acceptable salt thereof or the solvate thereof described herein in the non-fixed combination are each in the form of a solid pharmaceutical composition, and the solid pharmaceutical composition of the capsid protein inhibitor or the pharmaceutically acceptable salt thereof and the solid pharmaceutical composition of entecavir or the pharmaceutically acceptable salt thereof or the solvate thereof described herein are not present in the same sachet.

In some embodiments of the present application, the solid pharmaceutical composition is selected from the group consisting of a tablet and a capsule.

In another aspect, the present application further provides use of the pharmaceutical combination of the present application for preparing a medicament for treating hepatitis B virus infection. The pharmaceutical combination is as described above.

In another aspect, the present application further provides a method for treating hepatitis B virus infection, comprising administering to an individual in need an effective amount of the pharmaceutical combination of the present application. The pharmaceutical combination is as described above.

In another aspect, the present application further provides the pharmaceutical combination of the present application for use in treating hepatitis B virus infection. The pharmaceutical combination is as described above.

In another aspect, the present application further provides use of the pharmaceutical combination of the present application for treating hepatitis B virus infection. The pharmaceutical combination is as described above.

In some embodiments of the present application, the present application further provides a kit for use in treating hepatitis B virus infection, comprising: (a) a first pharmaceutical composition comprising the capsid protein inhibitor or the pharmaceutically acceptable salt thereof described herein as an active ingredient; and (b) a second pharmaceutical composition comprising a reverse transcriptase inhibitor or a pharmaceutically acceptable prodrug thereof, a pharmaceutically acceptable salt thereof or a solvate thereof as an active ingredient; and optionally (c) a package insert for combined use of the capsid protein inhibitor or the pharmaceutically acceptable salt thereof described herein and the reverse transcriptase inhibitor or the pharmaceutically acceptable prodrug thereof, the pharmaceutically acceptable salt thereof or the solvate thereof.

In some embodiments of the present application, the reverse transcriptase inhibitor or the pharmaceutically acceptable prodrug thereof, the pharmaceutically acceptable salt thereof or the solvate thereof is as described above.

In some embodiments of the present application, the present application provides use of the combination comprising both the capsid protein inhibitor or the pharmaceutically acceptable salt thereof and tenofovir or the pharmaceutically acceptable prodrug thereof or the pharmaceutically acceptable salt thereof described herein as active ingredients for preparing a medicament for treating hepatitis B virus infection, wherein the capsid protein inhibitor or the pharmaceutically acceptable salt thereof and tenofovir or the pharmaceutically acceptable prodrug thereof or the pharmaceutically acceptable salt thereof described herein are each prepared into a pharmaceutical composition.

In some embodiments of the present application, the present application further provides a kit for use in treating hepatitis B virus infection, comprising: (a) a first pharmaceutical composition comprising the capsid protein inhibitor or the pharmaceutically acceptable salt thereof described herein as an active ingredient; and (b) a second pharmaceutical composition comprising tenofovir or a pharmaceutically acceptable prodrug thereof or a pharmaceutically acceptable salt thereof as an active ingredient; and optionally (c) a package insert for combined use of the capsid protein inhibitor or the pharmaceutically acceptable salt thereof described herein and tenofovir or the pharmaceutically acceptable prodrug thereof or the pharmaceutically acceptable salt thereof.

In some embodiments of the present application, the present application provides use of the combination comprising the capsid protein inhibitor or the pharmaceutically acceptable salt thereof and entecavir or the pharmaceutically acceptable salt thereof or the solvate thereof described herein for preparing a medicament for treating hepatitis B virus infection, wherein the capsid protein inhibitor or the pharmaceutically acceptable salt thereof and entecavir or the pharmaceutically acceptable salt thereof or the solvate thereof described herein are each prepared into a pharmaceutical composition.

In some embodiments of the present application, the present application further provides a kit for use in treating hepatitis B virus infection, comprising: (a) a first pharmaceutical composition comprising the capsid protein inhibitor or the pharmaceutically acceptable salt thereof described herein as an active ingredient; and (b) a second pharmaceutical composition comprising entecavir or a pharmaceutically acceptable salt thereof or a solvate thereof as an active ingredient; and optionally (c) a package insert for combined use of the capsid protein inhibitor or the pharmaceutically acceptable salt thereof described herein and entecavir or the pharmaceutically acceptable salt thereof or the solvate thereof.

In some embodiments of the present application, the capsid protein inhibitor or the pharmaceutically acceptable salt thereof described herein is selected from a compound of formula I or a pharmaceutically acceptable salt thereof:

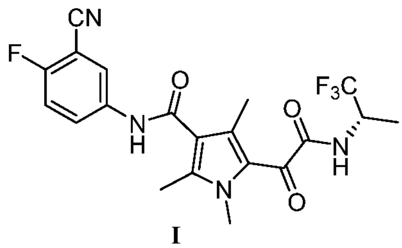

I

In another aspect, the present application provides a pharmaceutical combination, comprising a compound of formula I or a pharmaceutically acceptable salt thereof and a reverse transcriptase inhibitor or a pharmaceutically acceptable prodrug thereof, a pharmaceutically acceptable salt thereof or a solvate thereof:

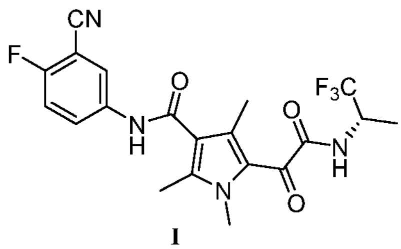

I

In some embodiments, the reverse transcriptase inhibitor or the pharmaceutically acceptable prodrug thereof, the pharmaceutically acceptable salt thereof or the solvate thereof is selected from the group consisting of entecavir or a pharmaceutically acceptable salt thereof or a solvate thereof, and tenofovir or a pharmaceutically acceptable prodrug thereof or a pharmaceutically acceptable salt thereof.

In a further aspect, the present application provides a pharmaceutical combination, comprising a compound of formula I or a pharmaceutically acceptable salt thereof, and entecavir or a pharmaceutically acceptable salt thereof or a solvate thereof.

In a further aspect, the present application provides a pharmaceutical combination, comprising a compound of formula I or a pharmaceutically acceptable salt thereof, and tenofovir or a pharmaceutically acceptable prodrug thereof or a pharmaceutically acceptable salt thereof.

In another aspect, the present application further provides use of the pharmaceutical combination of the present application for preparing a medicament for treating hepatitis B virus infection. The present application further provides a method for treating hepatitis B virus infection, comprising administering to an individual in need an effective amount of the pharmaceutical combination of the present application. The present application further provides a pharmaceutical combination for use in treating hepatitis B virus infection. The present application further provides use of the pharmaceutical combination of the present application for treating hepatitis B virus infection.

In some embodiments of the present application, the entecavir solvate is selected from an entecavir hydrate. In some embodiments, the entecavir hydrate is selected from entecavir hemihydrate to dihydrate. In some embodiments, the entecavir hydrate is selected from entecavir monohydrate.

In some embodiments of the present application, the pharmaceutically acceptable salt of entecavir is selected from a maleate. In some embodiments of the present application, the pharmaceutically acceptable salt of entecavir is selected from a monomaleate.

In some embodiments of the present application, entecavir or the pharmaceutically acceptable salt thereof or the solvate thereof is selected from the group consisting of entecavir monomaleate, entecavir monohydrate, and entecavir monomaleate monohydrate.

In some embodiments of the present application, the pharmaceutical combination described herein comprises a compound of formula I or a pharmaceutically acceptable salt thereof and entecavir or a pharmaceutically acceptable salt thereof or a hydrate thereof. In some embodiments, the pharmaceutical combination described herein comprises a compound of formula I or a pharmaceutically acceptable salt thereof and entecavir or a monohydrate thereof. In some embodiments, the pharmaceutical combination described herein comprises a compound of formula I or a pharmaceutically acceptable salt thereof and entecavir monomaleate monohydrate.

In some embodiments of the present application, the pharmaceutically acceptable prodrug of tenofovir comprises tenofovir disoproxil, tenofovir alafenamide, or tenofovir amibufenamide.

In some embodiments of the present application, the pharmaceutically acceptable salt of tenofovir or the pharmaceutically acceptable prodrug thereof is selected from the group consisting of a fumarate, an orotate, a disulfonate, a phosphate, a succinate, and an aspartate. In some embodiments of the present application, the pharmaceutically acceptable salt of tenofovir or the pharmaceutically acceptable prodrug thereof is selected from a fumarate.

In some embodiments of the present application, tenofovir or the pharmaceutically acceptable prodrug thereof or the pharmaceutically acceptable salt thereof is selected from the group consisting of tenofovir, tenofovir alafenamide fumarate, tenofovir disoproxil fumarate, tenofovir disoproxil orotate, tenofovir disoproxil hemidisulfonate, tenofovir disoproxil phosphate, tenofovir disoproxil succinate, tenofovir disoproxil aspartate, and tenofovir amibufenamide fumarate. In some embodiments of the present application, tenofovir or the pharmaceutically acceptable prodrug thereof or the pharmaceutically acceptable salt thereof is selected from the group consisting of tenofovir disoproxil fumarate and tenofovir alafenamide fumarate. In some embodiments of the present application, tenofovir or the pharmaceutically acceptable prodrug thereof or the pharmaceutically acceptable salt thereof is selected from tenofovir disoproxil fumarate.

In some embodiments of the present application, tenofovir or the pharmaceutically acceptable prodrug thereof or the pharmaceutically acceptable salt thereof is selected from the group consisting of the following structures:

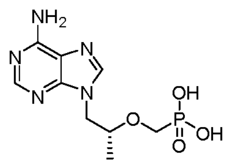,
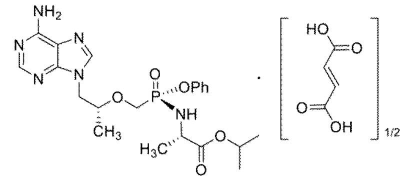,
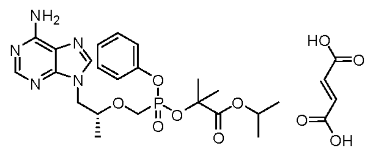,
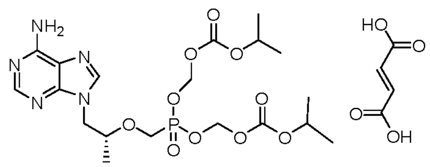,
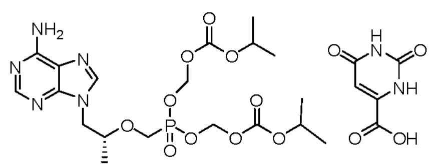,
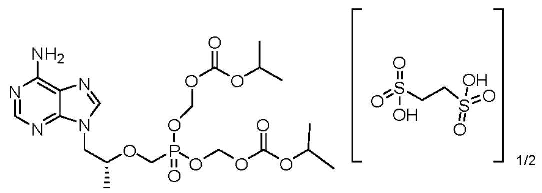,
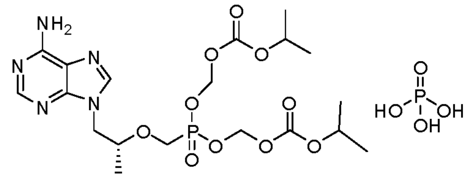,
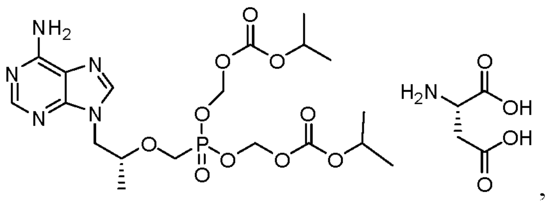, and
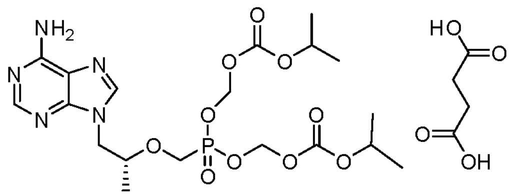.

In some embodiments of the present application, the pharmaceutical combination described herein comprises a compound of formula I or a pharmaceutically acceptable salt thereof and tenofovir or a pharmaceutically acceptable prodrug thereof or a pharmaceutically acceptable salt thereof. In some embodiments, the pharmaceutical combination described herein comprises a compound of formula I or a pharmaceutically acceptable salt thereof and tenofovir alafenamide or tenofovir disoproxil or a pharmaceutically acceptable salt thereof. In some embodiments, the pharmaceutical combination described herein comprises a compound of formula I or a pharmaceutically acceptable salt thereof and tenofovir disoproxil fumarate or tenofovir alafenamide fumarate.

In some embodiments of the present application, the compound of formula I or the pharmaceutically acceptable salt thereof in the pharmaceutical combination can be administered once every day, twice every day, thrice every day, once every two days, once every three days, once every four days, once every five days, once every six days, once every week, once every two weeks, or once every three weeks.

In some embodiments of the present application, the compound of formula I or the pharmaceutically acceptable salt thereof in the pharmaceutical combination is administered at a dose of 10 mg or 50 mg (calculated based on the weight of the compound of formula I) each time.

In some embodiments of the present application, the compound of formula I or the pharmaceutically acceptable salt thereof in the pharmaceutical combination is selected from the group consisting of doses of 10 mg and 50 mg (calculated based on the weight of the compound of formula I).

In some embodiments of the present application, the compound of formula I or the pharmaceutically acceptable salt thereof in the pharmaceutical combination is administered at a dose of 0.0001-20 mg/kg body weight (calculated based on the weight of the compound of formula I) each time.

In some embodiments of the present application, the dose of the compound of formula I or the pharmaceutically acceptable salt thereof in the pharmaceutical combination is 0.0001-20 mg/kg body weight (calculated based on the weight of the compound of formula I) per administration.

In some embodiments of the present application, the compound of formula I or the pharmaceutically acceptable salt thereof in the pharmaceutical combination is administered at a dose of 1-1500 mg, 2-1000 mg, 3-800 mg, 3-600 mg, 4-550 mg, 5-500 mg, 5-450 mg, 5-400 mg, 5-480 mg, 5-460 mg, 5-450 mg, 5-430 mg, 6-400 mg, 7-380 mg, 8-360 mg, 9-340 mg, or 10-320 mg each time.

In some embodiments of the present application, the dose of the compound of formula I or the pharmaceutically acceptable salt thereof in the pharmaceutical combination is 1-1500 mg, 2-1000 mg, 3-800 mg, 3-600 mg, 4-550 mg, 5-500 mg, 5-450 mg, 5-400 mg, 5-480 mg, 5-460 mg, 5-450 mg, 5-430 mg, 6-400 mg, 7-380 mg, 8-360 mg, 9-340 mg, or 10-320 mg per administration.

In some embodiments of the present application, tenofovir or the pharmaceutically acceptable prodrug thereof or the pharmaceutically acceptable salt thereof in the pharmaceutical combination can be administered once every day, twice every day, or once every two days, or once every three days, or once every four days, or once every five days, or once every six days, or once every seven days. In some embodiments of the present application, tenofovir or the pharmaceutically acceptable prodrug thereof or the pharmaceutically acceptable salt thereof in the pharmaceutical combination can be administered once every day.

In some embodiments of the present application, for patients with impaired renal function, tenofovir or the pharmaceutically acceptable prodrug thereof or the pharmaceutically acceptable salt thereof is administered once every 24 hours for those with a creatinine clearance rate (mL/min) $\geq$ 50, once every 48 hours for those with a creatinine clearance rate (mL/min) $\geq$ 30-49, and once every 72-96 hours for those with a creatinine clearance rate (mL/min) $\geq$ 10-29; and for hemodialysis patients, tenofovir or the pharmaceutically acceptable prodrug thereof or the pharmaceutically acceptable salt thereof is administered once every 7 days or after a total of about 12 hours of dialysis.

In some embodiments of the present application, tenofovir disoproxil fumarate is administered at a dose of 300 mg each time. In some embodiments of the present application, tenofovir alafenamide is administered at a dose of 25 mg each time, or tenofovir alafenamide fumarate is administered at a dose of 28 mg each time.

In some embodiments of the present application, the dose of tenofovir disoproxil fumarate is 300 mg per administration. In some embodiments of the present application, the dose of tenofovir alafenamide is 25 mg per administration, or the dose of tenofovir alafenamide fumarate is 28 mg per administration.

In some embodiments of the present application, the average daily dose of tenofovir or the pharmaceutically acceptable prodrug thereof or the pharmaceutically acceptable salt thereof in the pharmaceutical combination is 0.05 mg to 500 mg (calculated based on the weight of tenofovir). In some embodiments of the present application, the average daily dose of tenofovir or the pharmaceutically acceptable prodrug thereof or the pharmaceutically acceptable salt thereof in the pharmaceutical combination is 0.1 mg to 400 mg (calculated based on the weight of tenofovir). In some embodiments of the present application, the average daily dose of tenofovir or the pharmaceutically acceptable prodrug thereof or the pharmaceutically acceptable salt thereof in the pharmaceutical combination is 1 mg to 300 mg (calculated based on the weight of tenofovir). In some embodiments of the present application, the average daily dose of tenofovir or the pharmaceutically acceptable prodrug thereof or the pharmaceutically acceptable salt thereof in the pharmaceutical combination is 5 mg to 200 mg (calculated based on the weight of tenofovir). In some embodiments of the present application, the average daily dose of tenofovir or the pharmaceutically acceptable prodrug thereof or the pharmaceutically acceptable salt thereof in the pharmaceutical combination is 8 mg to 190 mg (calculated based on the weight of tenofovir). In some embodiments of the present application, the average daily dose of tenofovir or the pharmaceutically acceptable prodrug thereof or the pharmaceutically acceptable salt thereof in the pharmaceutical combination is 10 mg to 185 mg (calculated based on the weight of tenofovir). In some embodiments of the present application, the average daily dose of tenofovir or the pharmaceutically acceptable prodrug thereof or the pharmaceutically acceptable salt thereof in the pharmaceutical combination is 14 mg to 140 mg (calculated based on the weight of tenofovir).

In some embodiments of the present application, entecavir or the pharmaceutically acceptable salt thereof or the solvate thereof in the pharmaceutical combination can be administered once every day, twice every day, or once every two days.

In some embodiments of the present application, entecavir or the pharmaceutically acceptable salt thereof or the solvate thereof in the pharmaceutical combination is administered at a dose of 0.5 mg or 1.0 mg (calculated based on the weight of entecavir) each time.

In some embodiments of the present application, the dose of entecavir or the pharmaceutically acceptable salt thereof or the solvate thereof in the pharmaceutical combination is a dose of 0.5 mg or 1.0 mg (calculated based on the weight of entecavir) per administration.

In some embodiments of the present application, the average daily dose of entecavir or the pharmaceutically acceptable salt thereof or the solvate thereof in the pharmaceutical combination is 0.005 mg to 10.0 mg. In some embodiments of the present application, the average daily dose of entecavir or the pharmaceutically acceptable salt thereof or the solvate thereof in the pharmaceutical combination is 0.05 mg to 5.0 mg. In some embodiments of the present application, the average daily dose of entecavir or the pharmaceutically acceptable salt thereof or the solvate thereof in the pharmaceutical combination is 0.10 mg to 2.0 mg. In some embodiments of the present application, the average daily dose of entecavir or the pharmaceutically acceptable salt thereof or the solvate thereof in the pharmaceutical combination is 0.25 mg to 2.0 mg. In some embodiments of the present application, the average daily dose of entecavir or the pharmaceutically acceptable salt thereof or the solvate thereof in the pharmaceutical combination is 0.5 mg to 1.0 mg.

In some embodiments of the present application, in the pharmaceutical combination, the compound of formula I or the pharmaceutically acceptable salt thereof is administered at a dose of 1-1500 mg or 10-1000 mg (calculated based on the weight of the compound of formula I) each time, and the average daily dose of tenofovir or the pharmaceutically acceptable prodrug thereof or the pharmaceutically acceptable salt thereof is 0.05 mg to 500 mg (calculated based on the weight of tenofovir).

In some embodiments of the present application, in the pharmaceutical combination, the dose of the compound of formula **I** or the pharmaceutically acceptable salt thereof is 1-1500 mg or 10-1000 mg (calculated based on the weight of the compound of formula **I**) per administration, and the average daily dose of tenofovir or the pharmaceutically acceptable prodrug thereof or the pharmaceutically acceptable salt thereof is 0.05 mg to 500 mg (calculated based on the weight of tenofovir).

In some embodiments of the present application, in the pharmaceutical combination, the average daily dose ratio of the compound of formula **I** or the pharmaceutically acceptable salt thereof to tenofovir or the pharmaceutically acceptable prodrug thereof or the pharmaceutically acceptable salt thereof (calculated based on the weight of corresponding free compounds, the compound of formula **I**:tenofovir) is selected from 500:1 to 1:1000. In some embodiments, the average daily dose ratio of the compound of formula **I** or the pharmaceutically acceptable salt thereof to tenofovir or the pharmaceutically acceptable prodrug thereof or the pharmaceutically acceptable salt thereof (calculated based on the weight of corresponding free compounds, the compound of formula **I**:tenofovir) is selected from the group consisting of 500:1, 450:1, 400:1, 350:1, 300:1, 250:1, 200:1, 150:1, 100:1, 95:1, 90:1, 85:1, 80:1, 75:1, 70:1, 65:1, 60:1, 55:1, 50:1, 45:1, 40:1, 39:1, 38:1, 37:1, 36:1, 35:1, 34:1, 33:1, 32:1, 31:1, 30:1, 29:1, 28:1, 27:1, 26:1, 25:1, 24:1, 23:1, 22:1, 21:1, 20:1, 19:1, 18:1, 17:1, 16:1, 15:1, 10:1, 5:1, 4:1, 3:1, 2:1, 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90, 1:100, 1:150, 1:200, 1:250, 1:300, 1:350, 1:400, 1:450, 1:500, 1:550, 1:600, 1:650, 1:700, 1:750, 1:800, 1:850, 1:900, 1:950 and 1:1000 or from a range formed by any of the above ratios.

In some embodiments of the present application, in the pharmaceutical combination, the average daily dose ratio of the compound of formula **I** or the pharmaceutically acceptable salt thereof to tenofovir or the pharmaceutically acceptable prodrug thereof or the pharmaceutically acceptable salt thereof (calculated based on the molar ratio of free compounds) is selected from 100:1 to 1:100. In some embodiments, the average daily dose ratio of the compound of formula **I** or the pharmaceutically acceptable salt thereof to tenofovir or the pharmaceutically acceptable prodrug thereof or the pharmaceutically acceptable salt thereof (calculated based on the molar ratio of free compounds, the compound of formula I:tenofovir) is selected from the group consisting of: 100:1, 98:1, 96:1, 94:1, 92:1, 90:1, 88:1, 86:1, 84:1, 82:1, 80:1, 78:1, 76:1, 74:1, 72:1, 70:1, 68:1, 66:1, 64:1, 62:1, 60:1, 58:1, 56:1, 54:1, 52:1, 50:1, 48:1, 46:1, 44:1, 42:1, 40:1, 38:1, 36:1, 34:1, 32:1, 30:1, 28:1, 26:1, 25:1, 24:1, 23:1, 22:1, 21:1, 20:1, 19:1, 18:1, 17:1, 16:1, 15:1, 14:1, 13:1, 12:1, 11:1, 10:1, 9:1, 8:1, 7:1, 6:1, 5:1, 4:1, 3:1, 2:1, 1:1, 1:2, 1:3, 1:4, 1:5, 1:6, 1:7, 1:8, 1:9, 1:10, 1:12, 1:14, 1:15, 1:16, 1:17, 1:18, 1:19, 1:20, 1:21, 1:22, 1:23, 1:24, 1:25, 1:26, 1:27, 1:28, 1:29, 1:30, 1:31, 1:32, 1:33, 1:34, 1:35, 1:36, 1:38, 1:40, 1:42, 1:44, 1:46, 1:48, 1:50, 1:52, 1:54, 1:56, 1:58, 1:60, 1:62, 1:64, 1:66, 1:68, 1:70, 1:72, 1:74, 1:76, 1:78, 1:80, 1:82, 1:84, 1:86, 1:88, 1:90, 1:92, 1:94, 1:96, 1:98 and 1:100 or from a range formed by any of the above ratios.

In some embodiments of the present application, in the pharmaceutical combination, the average daily dose ratio (calculated based on weight) of the compound of formula **I** or the pharmaceutically acceptable salt thereof (with the compound of formula **I** calculated as a free compound) to tenofovir disoproxil fumarate is selected from the group consisting of 1:300 to 1500:300 and 10:300 to 1000:300. In some embodiments, in the pharmaceutical combination, the average daily dose ratio (calculated based on weight) of the compound of formula **I** or the pharmaceutically acceptable salt thereof (with the compound of formula **I** calculated as a free compound) to tenofovir disoproxil fumarate is selected from the group consisting of 10:300, 20:300, 30:300, 40:300, 50:300, 60:300, 70:300, 80:300, 90:300, 100:300, 110:300, 120:300, 130:300, 140:300, 150:300, 160:300, 170:300, 180:300, 190:300, 200:300, 210:300, 220:300, 230:300, 240:300, 250:300, 260:300, 270:300, 280:300, 290:300, 300:300, 310:300, 320:300, 330:300, 340:300, 350:300,

360:300, 370:300, 380:300, 390:300, 400:300, 410:300, 420:300, 430:300, 440:300, 450:300, 460:300, 470:300, 480:300, 490:300, 500:300, 510:300, 520:300, 530:300, 540:300, 550:300, 560:300, 570:300, 580:300, 590:300, 600:300, 610:300, 620:300, 630:300, 640:300, 650:300, 660:300, 670:300, 680:300, 690:300, 700:300, 710:300, 720:300, 730:300, 740:300, 750:300, 760:300, 770:300, 780:300, 790:300 and 800:300 or from a range formed by any of the above ratios.

In some embodiments of the present application, in the pharmaceutical combination, the average daily dose ratio (calculated based on weight) of the compound of formula **I** or the pharmaceutically acceptable salt thereof (with the compound of formula **I** calculated as a free compound) to tenofovir alafenamide is selected from the group consisting of 1:25 to 1500:25 and 10:25 to 1000:25. In some embodiments, in the pharmaceutical combination, the average daily dose ratio (calculated based on weight) of the compound of formula **I** or the pharmaceutically acceptable salt thereof (with the compound of formula **I** calculated as a free compound) to tenofovir alafenamide is selected from the group consisting of 10:25, 20:25, 30:25, 40:25, 50:25, 60:25, 70:25, 80:25, 90:25, 100:25, 110:25, 120:25, 130:25, 140:25, 150:25, 160:25, 170:25, 180:25, 190:25, 200:25, 210:25, 220:25, 230:25, 240:25, 250:25, 260:25, 270:25, 280:25, 290:25, 300:25, 310:25, 320:25, 330:25, 340:25, 350:25, 360:25, 370:25, 380:25, 390:25, 400:25, 410:25, 420:25, 430:25, 440:25, 450:25, 460:25, 470:25, 480:25, 490:25, 500:25, 510:25, 520:25, 530:25, 540:25, 550:25, 560:25, 570:25, 580:25, 590:25, 600:25, 610:25, 620:25, 630:25, 640:25, 650:25, 660:25, 670:25, 680:25, 690:25, 700:25, 710:25, 720:25, 730:25, 740:25, 750:25, 760:25, 770:25, 780:25, 790:25 and 800:25 or from a range formed by any of the above ratios.

In some embodiments of the present application, in the pharmaceutical combination, the average daily dose ratio (calculated based on weight) of the compound of formula **I** or the pharmaceutically acceptable salt thereof (with the compound of formula **I** calculated as a free compound) to tenofovir alafenamide fumarate is selected from the group consisting of 1:28 to 1500:28 and 10:28 to 1000:28. In some embodiments, in the pharmaceutical combination, the average daily dose ratio (calculated based on weight) of the compound of formula **I** or the pharmaceutically acceptable salt thereof (with the compound of formula **I** calculated as a free compound) to tenofovir alafenamide fumarate is selected from the group consisting of 10:28, 20:28, 30:28, 40:28, 50:28, 60:28, 70:28, 80:28, 90:28, 100:28, 110:28, 120:28, 130:28, 140:28, 150:28, 160:28, 170:28, 180:28, 190:28, 200:28, 210:28, 220:28, 230:28, 240:28, 280:28, 260:28, 270:28, 280:28, 290:28, 300:28, 310:28, 320:28, 330:28, 340:28, 350:28, 360:28, 370:28, 380:28, 390:28, 400:28, 410:28, 420:28, 430:28, 440:28, 450:28, 460:28, 470:28, 480:28, 490:28, 500:28, 510:28, 520:28, 530:28, 540:28, 550:28, 560:28, 570:28, 580:28, 590:28, 600:28, 610:28, 620:28, 630:28, 640:28, 650:28, 660:28, 670:28, 680:28, 690:28, 700:28, 710:28, 720:28, 730:28, 740:28, 750:28, 760:28, 770:28, 780:28, 790:28 and 800:28 or from a range formed by any of the above ratios.

In some embodiments of the present application, the compound of formula **I** or the pharmaceutically acceptable salt thereof and tenofovir or the pharmaceutically acceptable prodrug thereof or the pharmaceutically acceptable salt thereof in the pharmaceutical combination are each administered in a single dose or in multiple doses.

In some embodiments of the present application, in the pharmaceutical combination, the compound of formula **I** or the pharmaceutically acceptable salt thereof is administered at a dose of 1-1500 mg, 2-1000 mg, 3-800 mg, 3-600 mg, 4-550 mg, 5-500 mg, 5-450 mg, 5-400 mg, 5-480 mg, 5-460 mg, 5-450 mg, 5-430 mg, 6-400 mg, 7-380 mg, 8-360 mg, 9-340 mg, or 10-320 mg each time, and the average daily dose of entecavir or the pharmaceutically acceptable salt thereof or the solvate thereof is 0.005 mg to 10.0 mg.

In some embodiments of the present application, in the pharmaceutical combination, the dose of the compound of formula **I** or the pharmaceutically acceptable salt thereof is 1-1500 mg, 2-1000 mg, 3-800 mg, 3-600 mg, 4-550 mg, 5-500 mg, 5-450 mg, 5-400 mg, 5-480 mg, 5-460 mg, 5-450 mg, 5-430 mg, 6-400 mg, 7-380 mg, 8-360 mg, 9-340 mg, or 10-320 mg per administration, and the average daily dose of entecavir or the pharmaceutically acceptable salt thereof or the solvate thereof is 0.005 mg to 10.0 mg.

In some embodiments of the present application, in the pharmaceutical combination, the average daily dose ratio of the compound of formula **I** or the pharmaceutically acceptable salt thereof to entecavir or the pharmaceutically acceptable salt thereof or the solvate thereof (calculated based on the weight of free compounds) is selected from 1000:1 to 1:1000. In some embodiments, the average daily dose ratio of the compound of formula **I** or the pharmaceutically acceptable salt thereof to entecavir or the pharmaceutically acceptable salt thereof or the solvate thereof (calculated based on the weight of free compounds, the compound of formula **I**:entecavir) is selected from the group consisting of 1000:1, 950:1, 900:1, 850:1, 800:1, 750:1, 700:1, 650:1, 640:1, 630:1, 620:1, 610:1, 600:1, 550:1, 500:1, 450:1, 400:1, 350:1, 300:1, 250:1, 200:1, 150:1, 100:1, 90:1, 80:1, 70:1, 60:1, 50:1, 40:1, 30:1, 20:1, 10:1, 1:0.01, 1:0.02, 1:0.03, 1:0.04, 1:0.05, 1:0.06, 1:0.07, 1:0.08, 1:0.09, 1:0.1, 1:0.5, 1:0.6, 1:0.7, 1:0.8, 1:0.9, 1:1, 1:10, 1:20, 1:30, 1:40, 1:50, 1:60, 1:70, 1:80, 1:90, 1:100, 1:150, 1:200, 1:250, 1:300, 1:350, 1:400, 1:450, 1:500, 1:550, 1:600, 1:650, 1:700, 1:750, 1:800, 1:850, 1:900, 1:950 and 1:1000 or from a range formed by any of the above ratios.

In some embodiments of the present application, in the pharmaceutical combination, the average daily dose ratio of the compound of formula **I** or the pharmaceutically acceptable salt thereof to entecavir or the pharmaceutically acceptable salt thereof or the solvate thereof (calculated based on the molar ratio of free compounds) is selected from

300:1 to 1:100. In some embodiments, the average daily dose ratio of the compound of formula **I** or the pharmaceutically acceptable salt thereof to entecavir or the pharmaceutically acceptable salt thereof or the solvate thereof (calculated based on the molar ratio of free compounds, the compound of formula **I**:entecavir) is selected from the group consisting of: 300:1, 290:1, 280:1, 270:1, 260:1, 250:1, 240:1, 235:1, 230:1, 225:1, 220:1, 215:1, 210:1, 205:1, 200:1, 190:1, 180:1, 170:1, 160:1, 150:1, 140:1, 130:1, 120:1, 110:1, 100:1, 98:1, 96:1, 94:1, 92:1, 90:1, 88:1, 86:1, 84:1, 82:1, 80:1, 78:1, 76:1, 74:1, 72:1, 70:1, 68:1, 66:1, 64:1, 62:1, 60:1, 58:1, 56:1, 54:1, 52:1, 50:1, 48:1, 46:1, 44:1, 42:1, 40:1, 38:1, 36:1, 34:1, 32:1, 30:1, 28:1, 26:1, 24:1, 22:1, 20:1, 18:1, 16:1, 14:1, 12:1, 10:1,8:1,6:1,4:1,2:1, 1:1, 1:2, 1:4, 1:5, 1:6, 1:7, 1:8, 1:9, 1:10, 1:11, 1:12, 1:13, 1:14, 1:15, 1:16, 1:17, 1:18, 1:19, 1:20, 1:21, 1:22, 1:23, 1:24, 1:25, 1:26, 1:27, 1:28, 1:29, 1:30, 1:32, 1:34, 1:36, 1:38, 1:40, 1:42, 1:44, 1:46, 1:48, 1:50, 1:52, 1:54, 1:56, 1:58, 1:60, 1:62, 1:64, 1:66, 1:68, 1:70, 1:72, 1:74, 1:76, 1:78, 1:80, 1:82, 1:84, 1:86, 1:88, 1:90, 1:92, 1:94, 1:96, 1:98 and 1:100 or from a range formed by any of the above ratios.

In some embodiments of the present application, in the pharmaceutical combination, the average daily dose ratio (calculated based on weight) of the compound of formula **I** or the pharmaceutically acceptable salt thereof (with the compound of formula **I** calculated as a free compound) to entecavir monohydrate is selected from the group consisting of 1:0.5 to 1500:0.5 and 10:0.5 to 1000:0.5. In some embodiments, in the pharmaceutical combination, the average daily dose ratio (calculated based on weight) of the compound of formula **I** or the pharmaceutically acceptable salt thereof (with the compound of formula **I** calculated as a free compound) to entecavir monohydrate is selected from the group consisting of 10:0.5, 20:0.5, 30:0.5, 40:0.5, 50:0.5, 60:0.5, 70:0.5, 80:0.5, 90:0.5, 100:0.5, 110:0.5, 120:0.5, 130:0.5, 140:0.5, 150:0.5, 160:0.5, 170:0.5, 180:0.5, 190:0.5, 200:0.5, 210:0.5, 220:0.5, 230:0.5, 240:0.5, 250:0.5, 260:0.5, 270:0.5, 280:0.5, 290:0.5, 300:0.5, 310:0.5, 320:0.5, 330:0.5, 340:0.5, 350:0.5, 360:0.5, 370:0.5, 380:0.5, 390:0.5, 400:0.5, 410:0.5, 420:0.5, 430:0.5, 440:0.5, 450:0.5, 460:0.5, 470:0.5, 480:0.5, 490:0.5, 500:0.5, 510:0.5, 520:0.5, 530:0.5, 540:0.5, 550:0.5, 560:0.5, 570:0.5, 580:0.5, 590:0.5, 600:0.5, 610:0.5, 620:0.5, 630:0.5, 640:0.5, 650:0.5, 660:0.5, 670:0.5, 680:0.5, 690:0.5, 700:0.5, 710:0.5, 720:0.5, 730:0.5, 740:0.5, 750:0.5, 760:0.5, 770:0.5, 780:0.5, 790:0.5 and 800:0.5 or from a range formed by any of the above ratios.

In some embodiments of the present application, the compound of formula **I** or the pharmaceutically acceptable salt thereof and entecavir or the pharmaceutically acceptable salt thereof or the solvate thereof in the pharmaceutical combination are each administered in a single dose or in multiple doses.

In some embodiments of the present application, the pharmaceutical combination is a fixed combination. In some embodiments, the fixed combination is in the form of a solid pharmaceutical composition.

In some embodiments, the compound of formula **I** or the pharmaceutically acceptable salt thereof and tenofovir or the pharmaceutically acceptable prodrug thereof or the pharmaceutically acceptable salt thereof in the fixed combination are present in the same solid pharmaceutical composition.

In some embodiments, the compound of formula **I** or the pharmaceutically acceptable salt thereof and entecavir or the pharmaceutically acceptable salt thereof or the solvate thereof in the fixed combination are present in the same solid pharmaceutical composition.

In some embodiments of the present application, the pharmaceutical combination is a non-fixed combination. In some embodiments, the compound of formula **I** or the pharmaceutically acceptable salt thereof and entecavir or the pharmaceutically acceptable salt thereof or the solvate thereof in the non-fixed combination are each in the form of a solid pharmaceutical composition.

In some embodiments, the compound of formula **I** or the pharmaceutically acceptable salt thereof and tenofovir or the pharmaceutically acceptable prodrug thereof or the pharmaceutically acceptable salt thereof in the non-fixed combination are each in the form of a solid pharmaceutical composition, and the solid pharmaceutical composition of the compound of formula **I** or the pharmaceutically acceptable salt thereof and the solid pharmaceutical composition of tenofovir or the pharmaceutically acceptable prodrug thereof or the pharmaceutically acceptable salt thereof are present in the same sachet. In some embodiments, the compound of formula **I** or the pharmaceutically acceptable salt thereof and tenofovir or the pharmaceutically acceptable prodrug thereof or the pharmaceutically acceptable salt thereof in the non-fixed combination are each in the form of a solid pharmaceutical composition, and the solid pharmaceutical composition of the compound of formula **I** or the pharmaceutically acceptable salt thereof and the solid pharmaceutical composition of tenofovir or the pharmaceutically acceptable prodrug thereof or the pharmaceutically acceptable salt thereof are not present in the same sachet.

In some embodiments, the compound of formula **I** or the pharmaceutically acceptable salt thereof and entecavir or the pharmaceutically acceptable salt thereof or the solvate thereof in the non-fixed combination are each in the form of a solid pharmaceutical composition, and the solid pharmaceutical composition of the compound of formula **I** or the pharmaceutically acceptable salt thereof and the solid pharmaceutical composition of entecavir or the pharmaceutically acceptable salt thereof or the solvate thereof are present in the same sachet. In some embodiments, the compound of formula **I** or the pharmaceutically acceptable salt thereof and entecavir or the pharmaceutically acceptable salt thereof or the solvate thereof in the non-fixed combination are each in the form of a solid pharmaceutical composition, and the solid pharmaceutical composition of the compound of formula **I** or the pharmaceutically acceptable salt thereof and the

solid pharmaceutical composition of entecavir or the pharmaceutically acceptable salt thereof or the solvate thereof are not present in the same sachet.

In some embodiments of the present application, the solid pharmaceutical composition is selected from the group consisting of a tablet and a capsule.

In another aspect, the present application further provides use of the pharmaceutical combination of the present application for preparing a medicament for treating hepatitis B virus infection. The pharmaceutical combination is as described above.

In another aspect, the present application further provides a method for treating hepatitis B virus infection, comprising administering to an individual in need an effective amount of the pharmaceutical combination of the present application. The pharmaceutical combination is as described above.

In another aspect, the present application further provides the pharmaceutical combination of the present application for use in treating hepatitis B virus infection. The pharmaceutical combination is as described above.

In another aspect, the present application further provides use of the pharmaceutical combination of the present application for treating hepatitis B virus infection. The pharmaceutical combination is as described above.

In some embodiments of the present application, the present application further provides a kit for use in treating hepatitis B virus infection, comprising: (a) a first pharmaceutical composition comprising a compound of formula I or a pharmaceutically acceptable salt thereof as an active ingredient; and (b) a second pharmaceutical composition comprising a reverse transcriptase inhibitor or a pharmaceutically acceptable prodrug thereof, a pharmaceutically acceptable salt thereof or a solvate thereof as an active ingredient; and optionally (c) a package insert for combined use of the compound of formula **I** or the pharmaceutically acceptable salt thereof and the reverse transcriptase inhibitor or the pharmaceutically acceptable prodrug thereof, the pharmaceutically acceptable salt thereof or the solvate thereof.

In some embodiments of the present application, the reverse transcriptase inhibitor or the pharmaceutically acceptable prodrug thereof, the pharmaceutically acceptable salt thereof or the solvate thereof is as described above.

In some embodiments of the present application, the present application provides use of the combination comprising both the compound of formula **I** or the pharmaceutically acceptable salt thereof and tenofovir or the pharmaceutically acceptable prodrug thereof or the pharmaceutically acceptable salt thereof as active ingredients for preparing a medicament for treating hepatitis B virus infection, wherein the compound of formula **I** or the pharmaceutically acceptable salt thereof and tenofovir or the pharmaceutically acceptable prodrug thereof or the pharmaceutically acceptable salt thereof are each prepared into a pharmaceutical composition.

In some embodiments of the present application, the present application further provides a kit for use in treating hepatitis B virus infection, comprising: (a) a first pharmaceutical composition comprising a compound of formula **I** or a pharmaceutically acceptable salt thereof as an active ingredient; and (b) a second pharmaceutical composition comprising tenofovir or a pharmaceutically acceptable prodrug thereof or a pharmaceutically acceptable salt thereof as an active ingredient; and optionally (c) a package insert for combined use of the compound of formula **I** or the pharmaceutically acceptable salt thereof and tenofovir or the pharmaceutically acceptable prodrug thereof or the pharmaceutically acceptable salt thereof.

In some embodiments of the present application, the present application provides use of the combination comprising the compound of formula **I** or the pharmaceutically acceptable salt thereof and entecavir or the pharmaceutically acceptable salt thereof or the solvate thereof for preparing a medicament for treating hepatitis B virus infection, wherein the compound of formula **I** or the pharmaceutically acceptable salt thereof and entecavir or the pharmaceutically acceptable salt thereof or the solvate thereof are each prepared into a pharmaceutical composition. In some embodiments of the present application, the present application further provides a kit for use in treating hepatitis B virus infection, comprising: (a) a first pharmaceutical composition comprising a compound of formula **I** or a pharmaceutically acceptable salt thereof as an active ingredient; and (b) a second pharmaceutical composition comprising entecavir or a pharmaceutically acceptable salt thereof or a solvate thereof as an active ingredient; and optionally (c) a package insert for combined use of the compound of formula I or the pharmaceutically acceptable salt thereof and entecavir or the pharmaceutically acceptable salt thereof or the solvate thereof.

Exemplary specific compounds (1-60) or pharmaceutically acceptable salts thereof in WO2019185016:

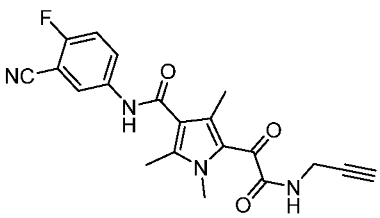 , 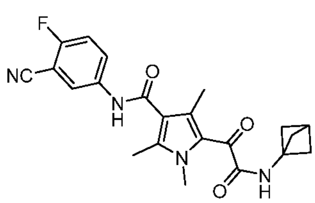 , 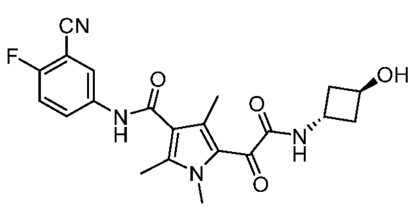 ,

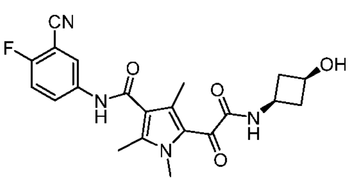, 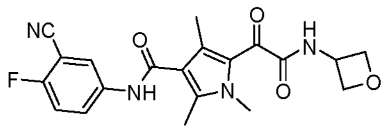, 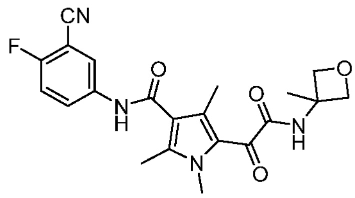,

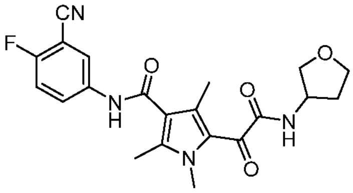, 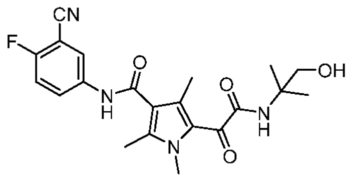, 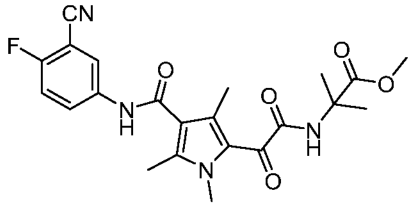,

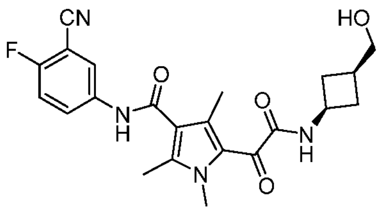, 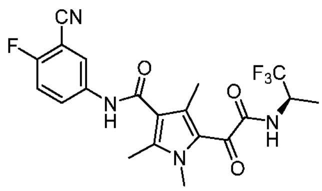, 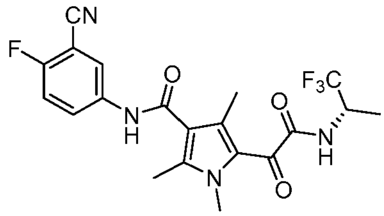,

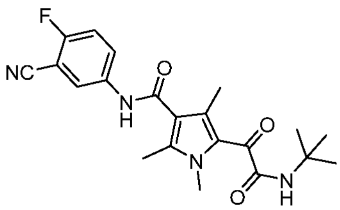, 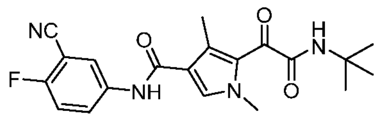, 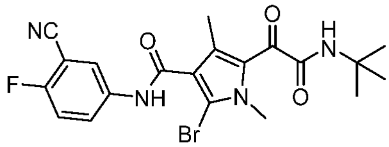,

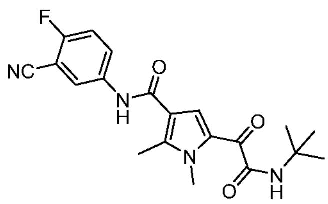, 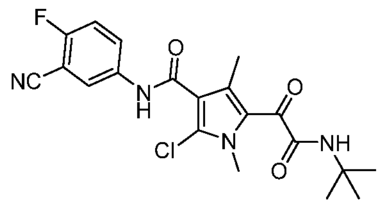, 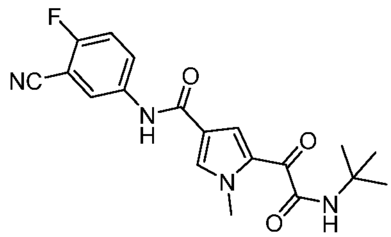,

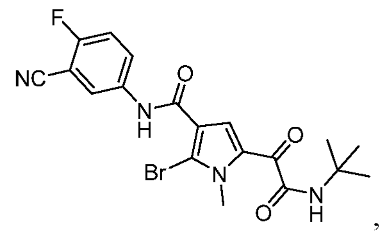, 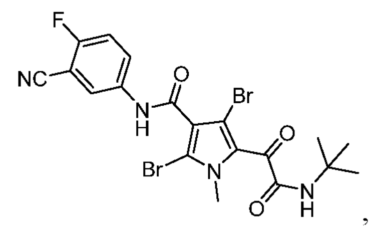, 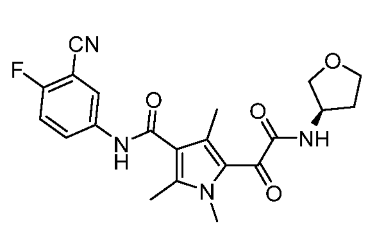,

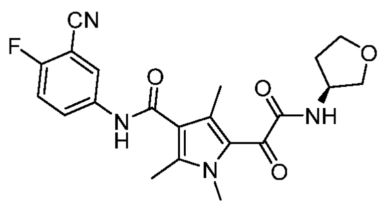, 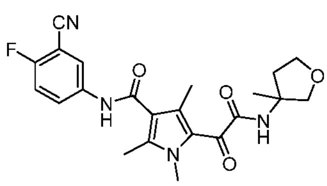, 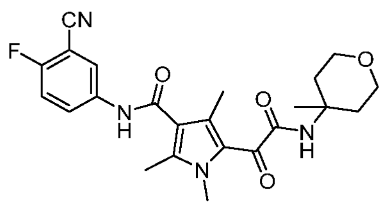,

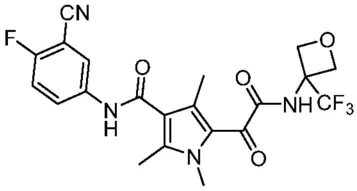,
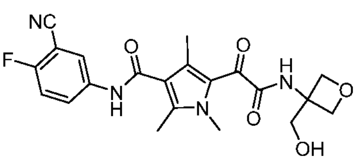,
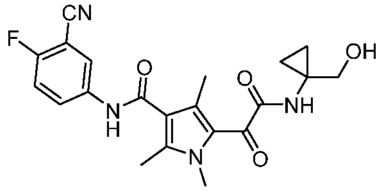,

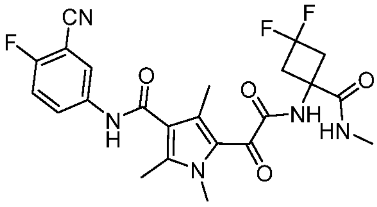,
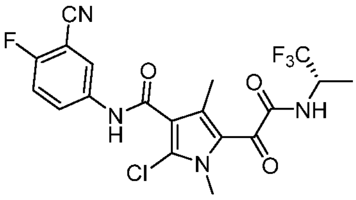,
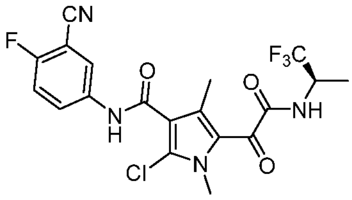,

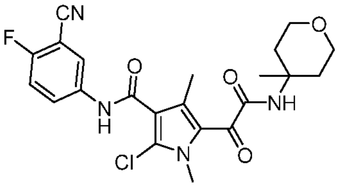,
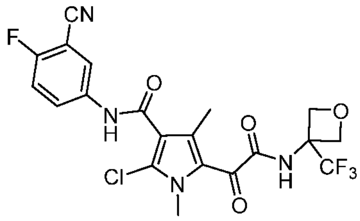,
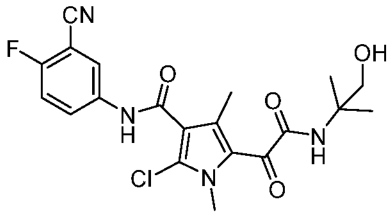,

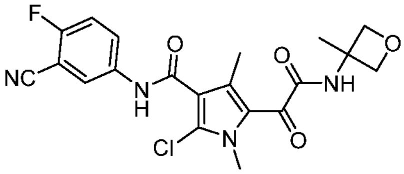,
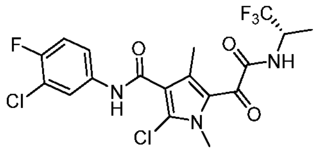,
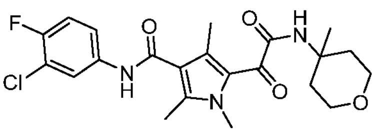,

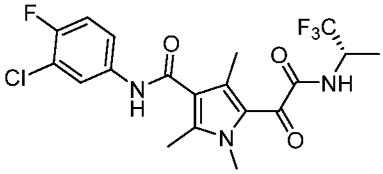,
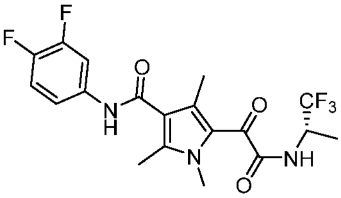,
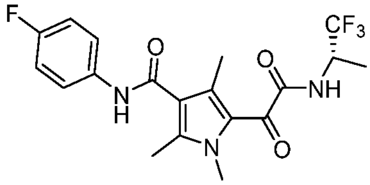,

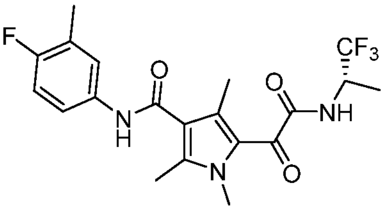,
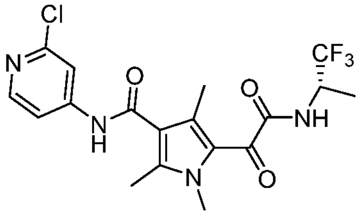,
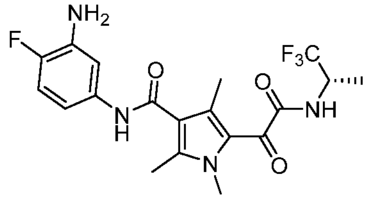,

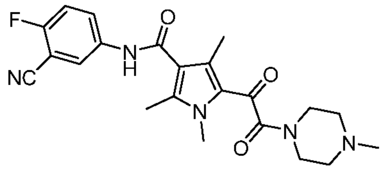,
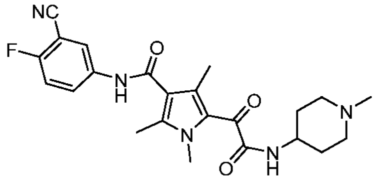,
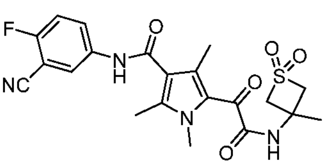,

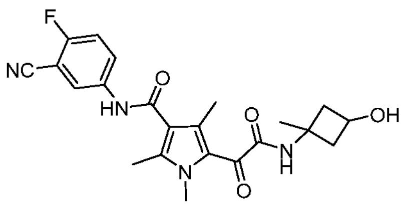, 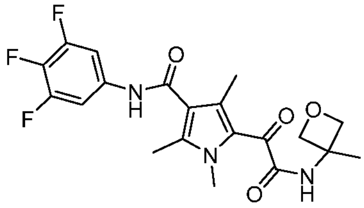, 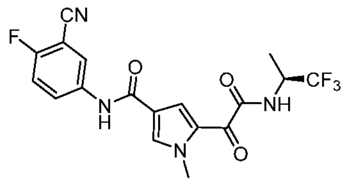,

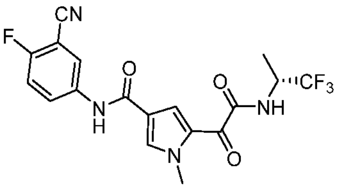, 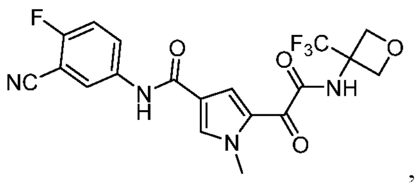, 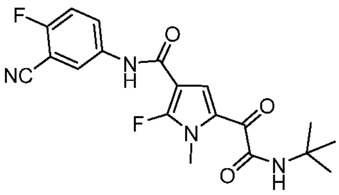,

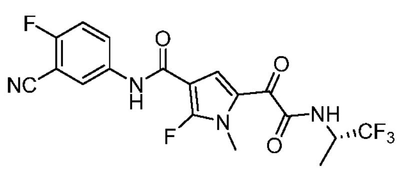, 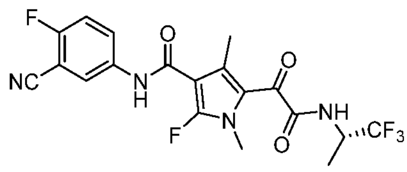, 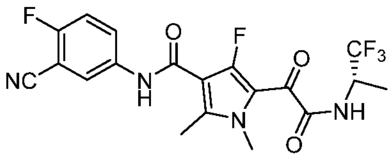,

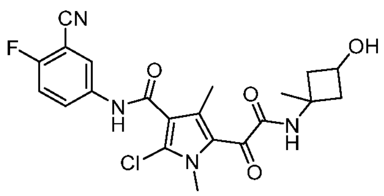, 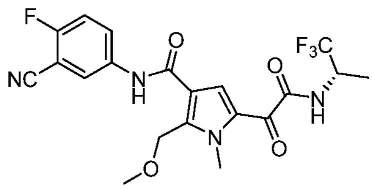, 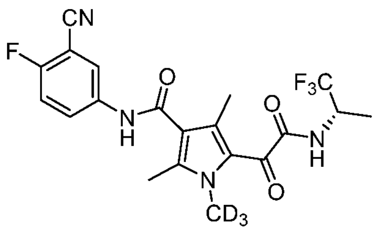,

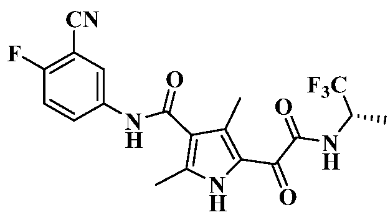, 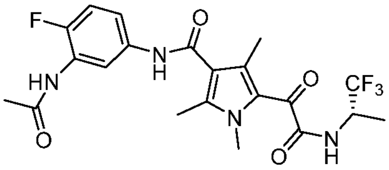, and 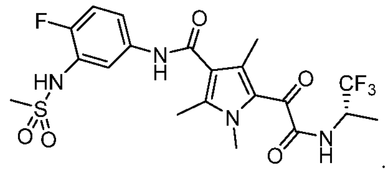.

Exemplary specific compounds (1-56) or pharmaceutically acceptable salts thereof in WO2019165374:

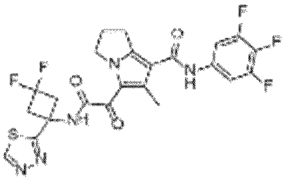

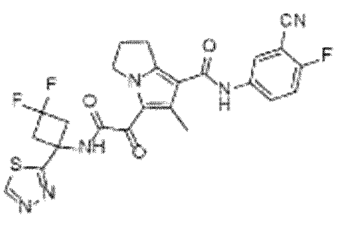

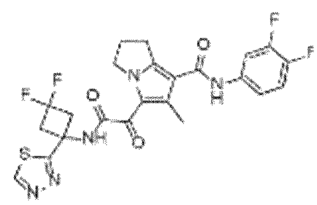

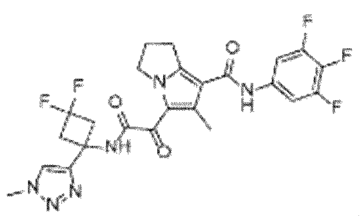

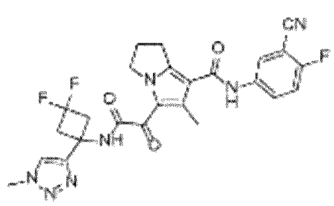

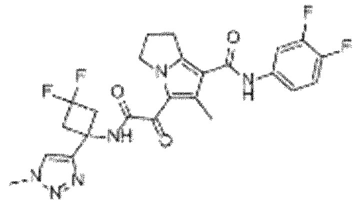

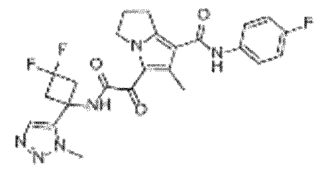
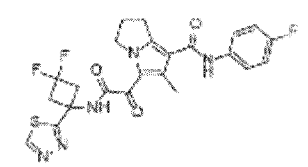
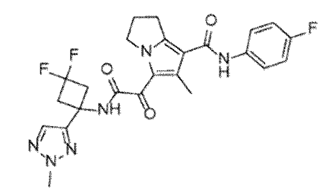

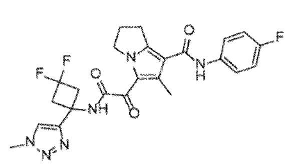
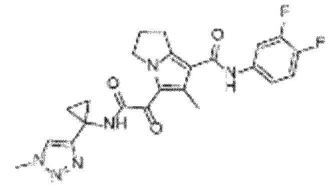
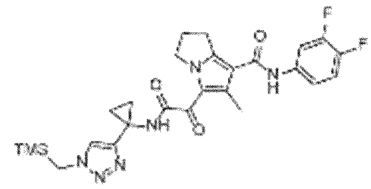

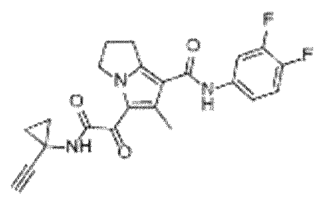
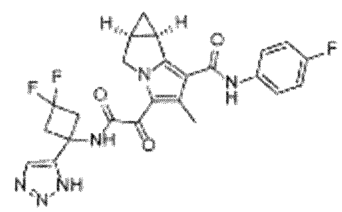
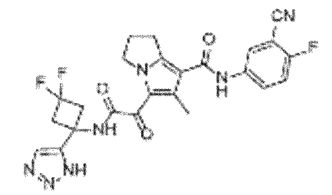

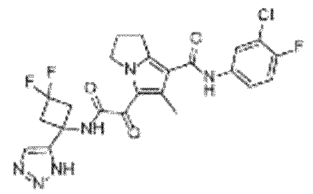
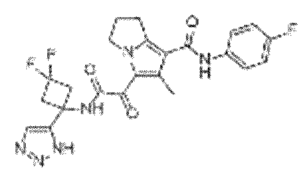
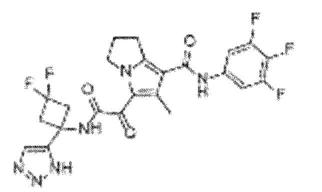

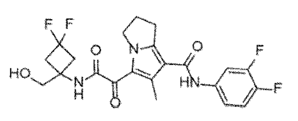
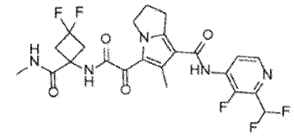
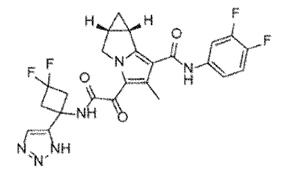

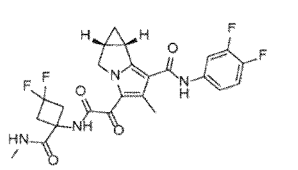
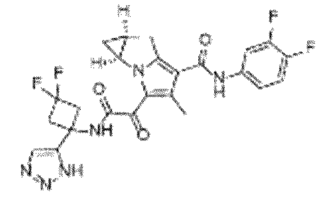
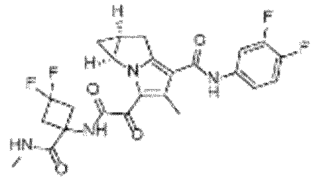

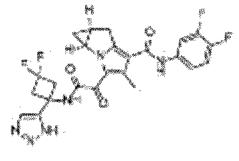
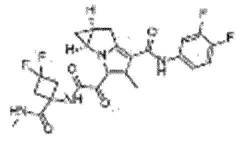
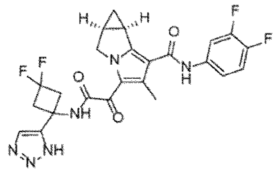

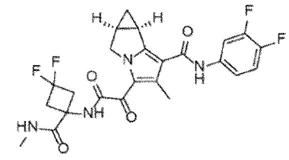 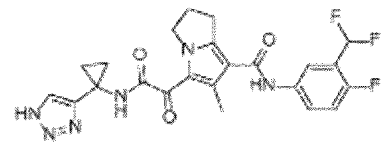 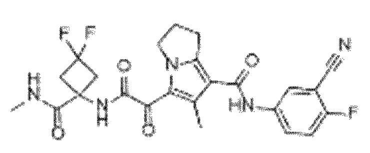

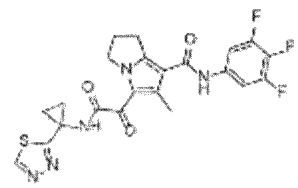 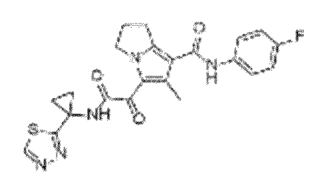 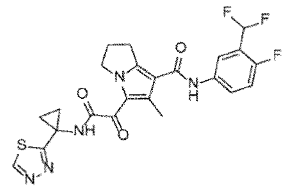

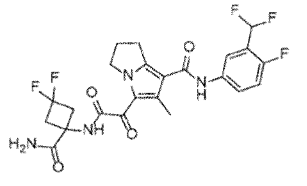 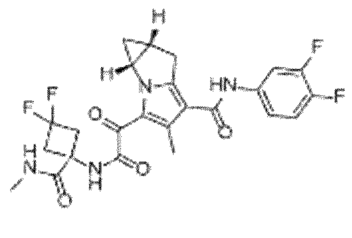 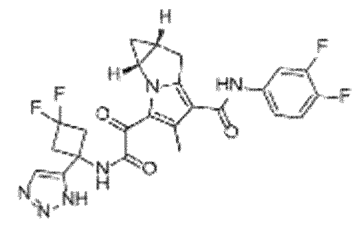

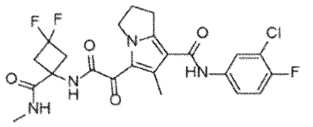 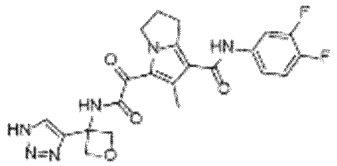 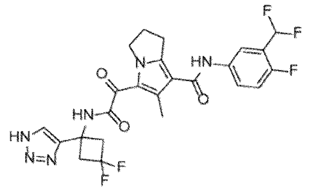

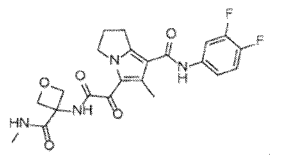 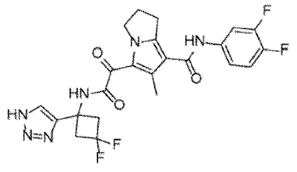 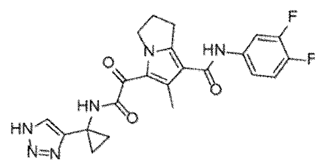

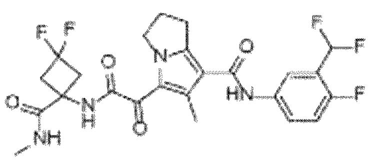 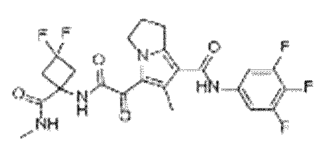 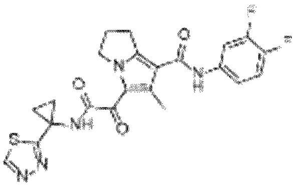

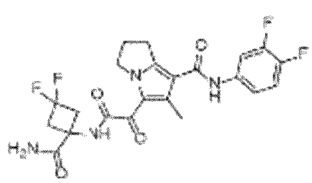 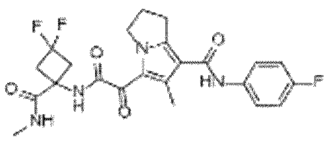 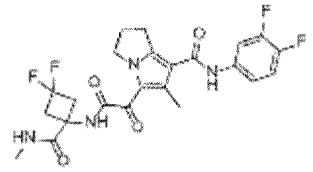

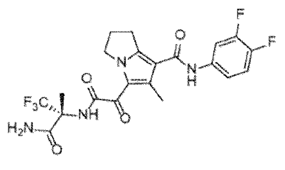

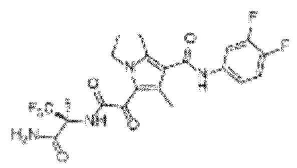

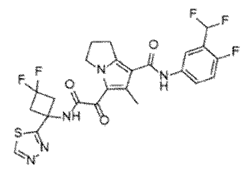

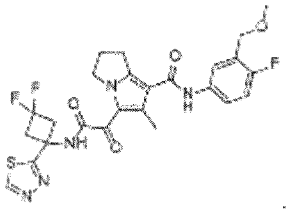

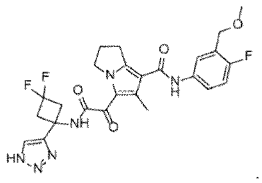

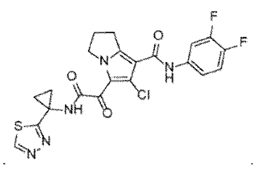

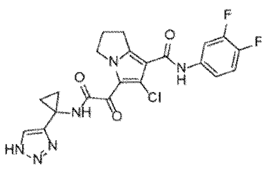

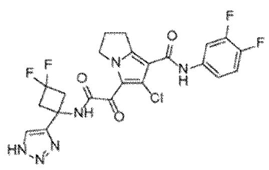

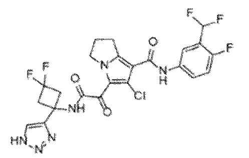

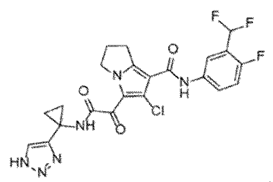

.

Exemplary specific compounds (compounds 1-759) or pharmaceutically acceptable salts thereof in WO2019241292:

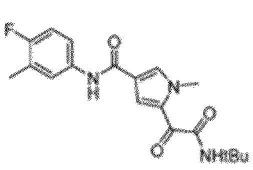
,
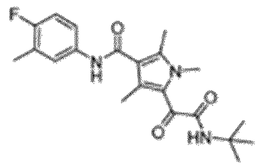
,
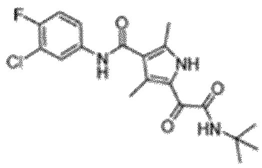
,

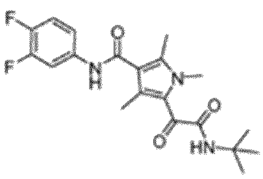
,
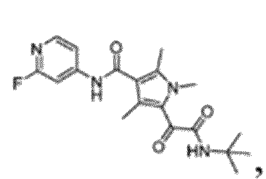
,
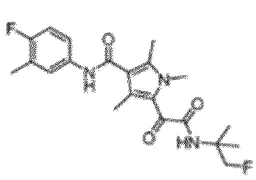
,

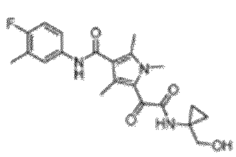
,
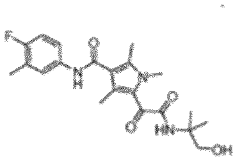
,
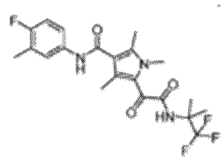
,

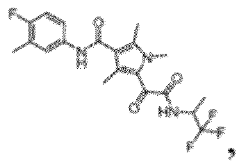,
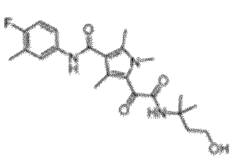,
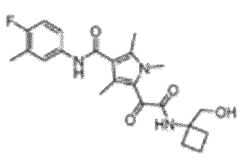,

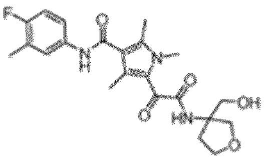,
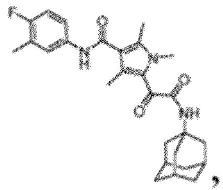,
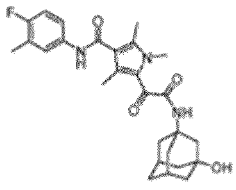,

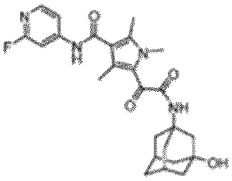,
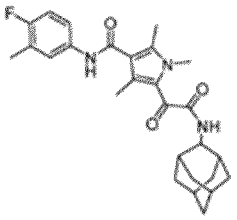,
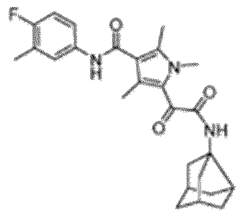,

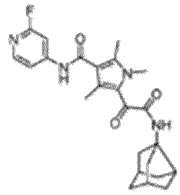,
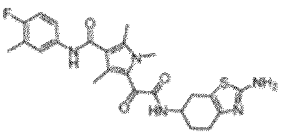,
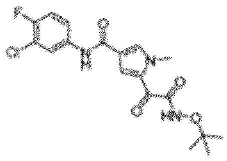,

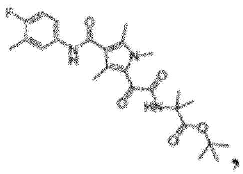,
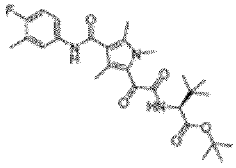,

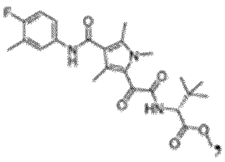,
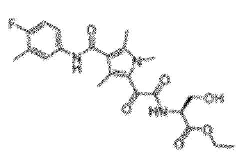,
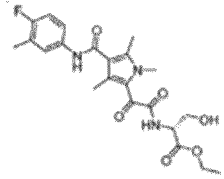,

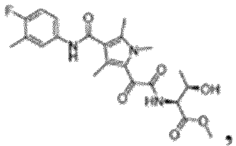,
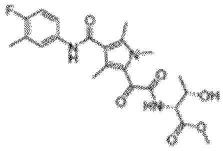,
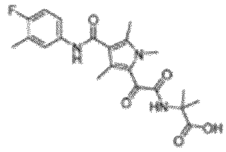,

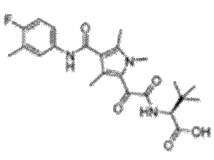, 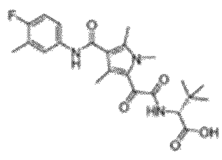, 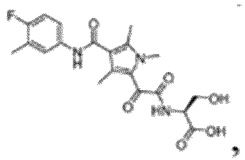,

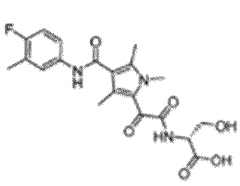, 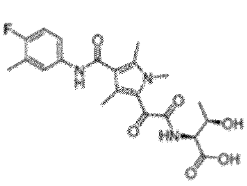, 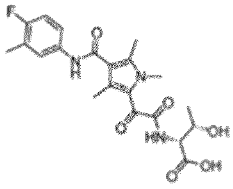,

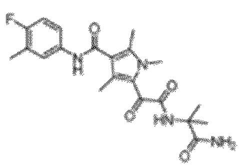, 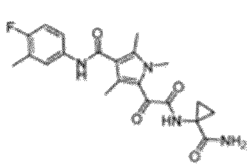, 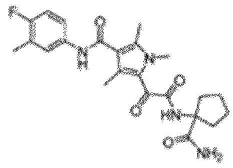,

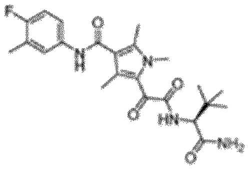, 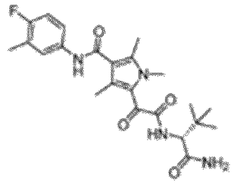, 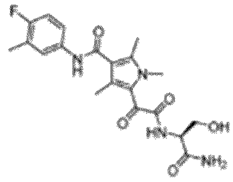,

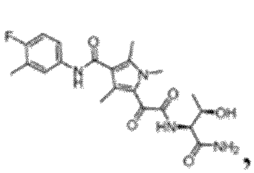, 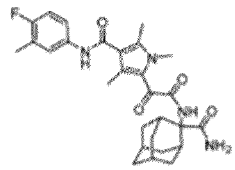, 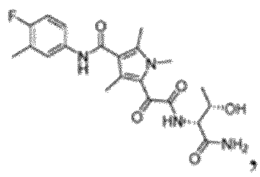,

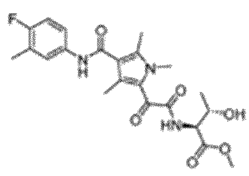, 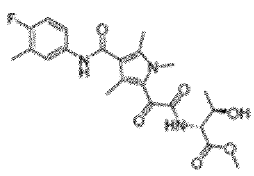, 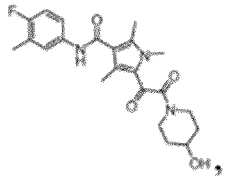,

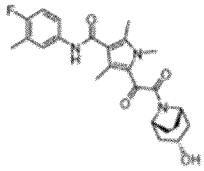, 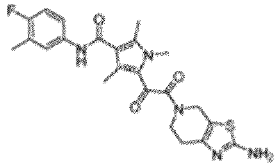, 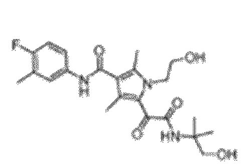,

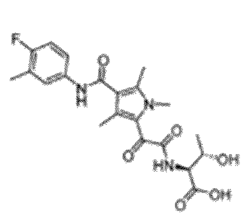 , 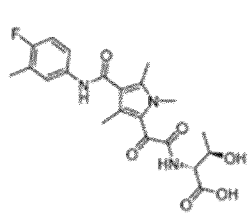 , 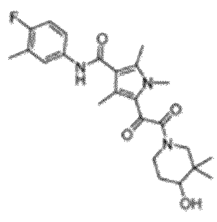 ,

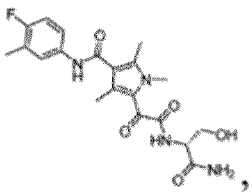 , 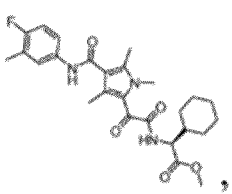 , 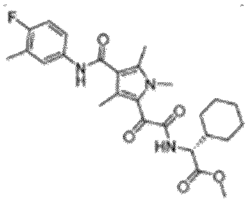 ,

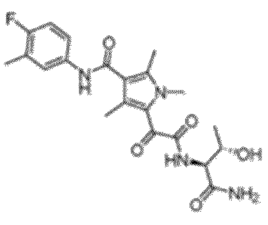 , 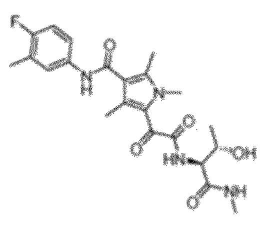 , 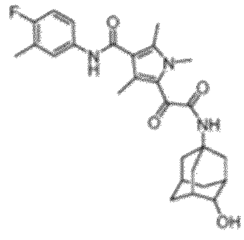 ,

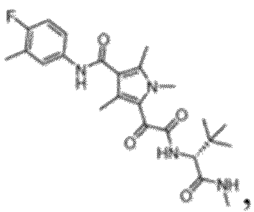 , 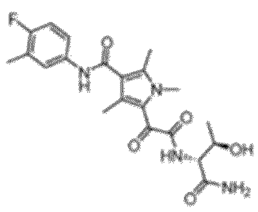 , 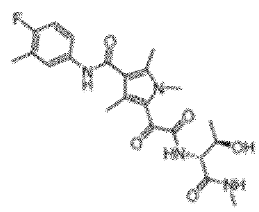 ,

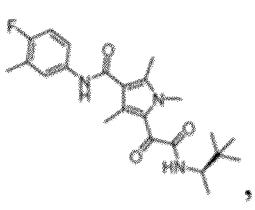 , 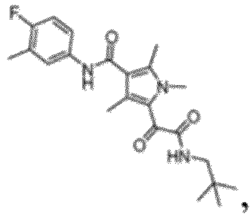 , 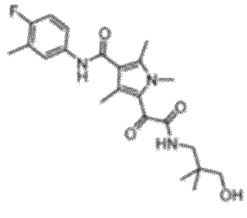 ,

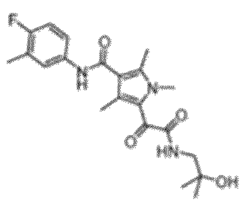 , 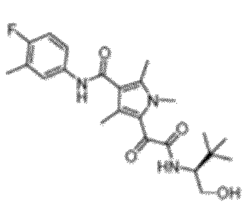 , 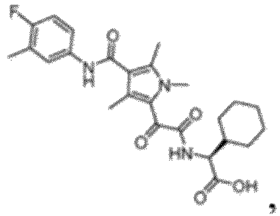 ,

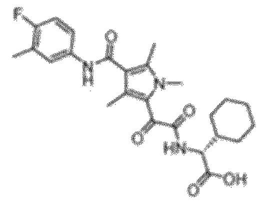, 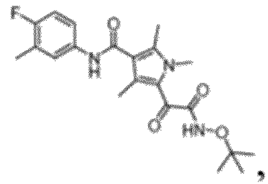, 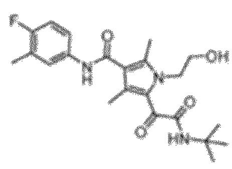,

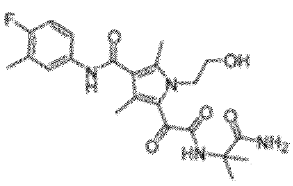, 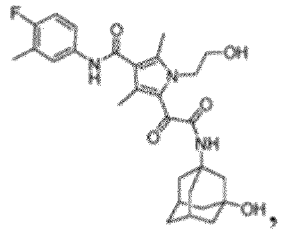, 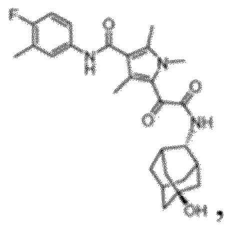,

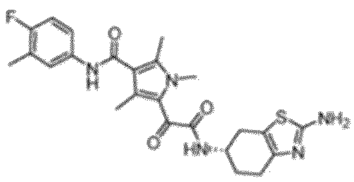, 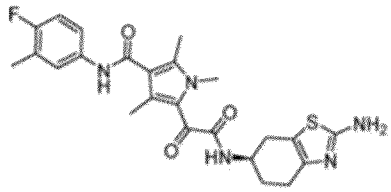,

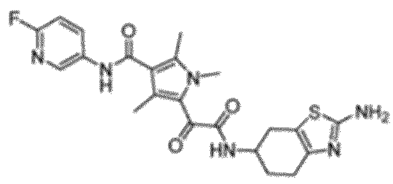, 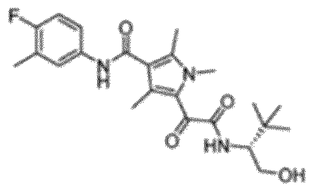,

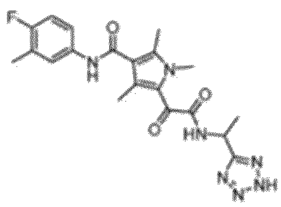, 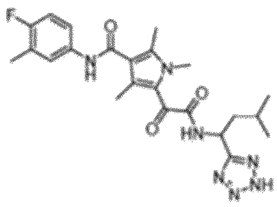, 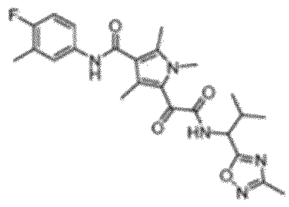,

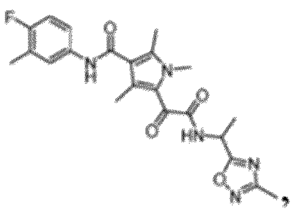, 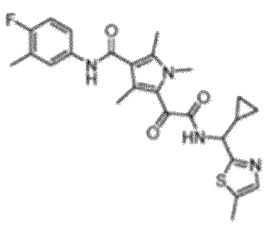, 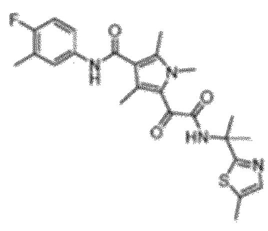,

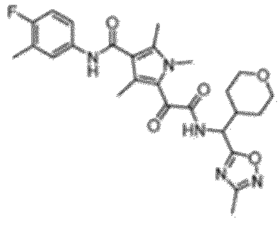,
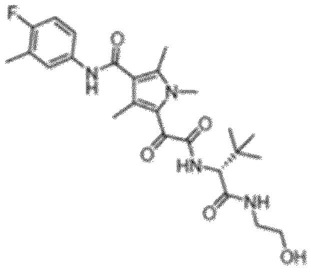,
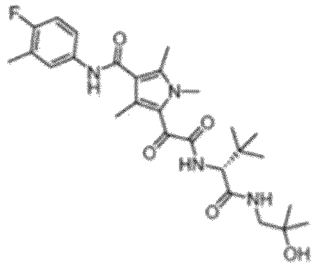,

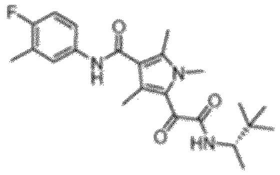,
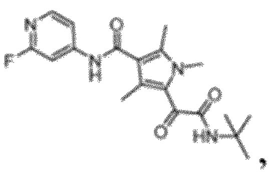,
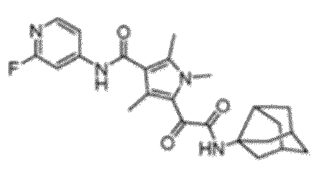,

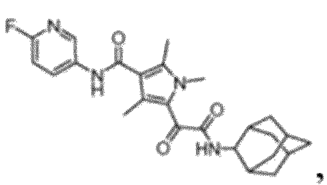,
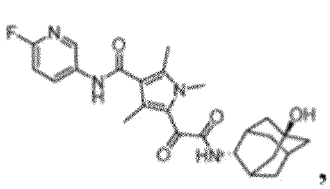,
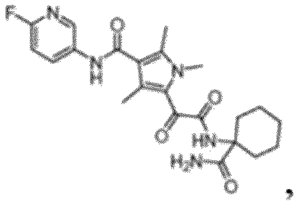,

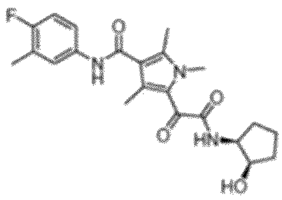,
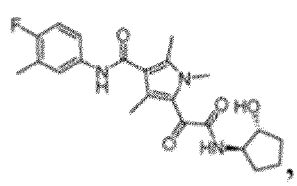,
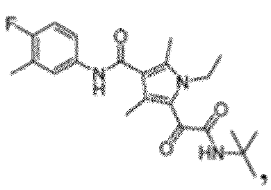,

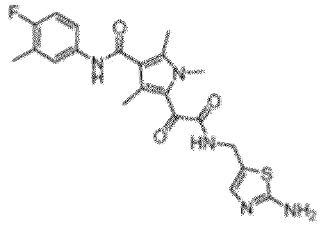,
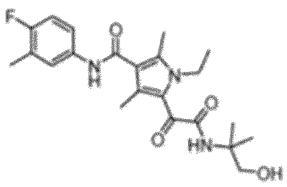,
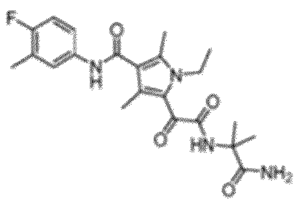,

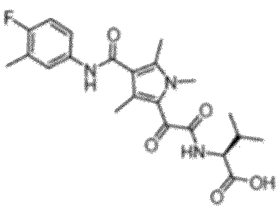,
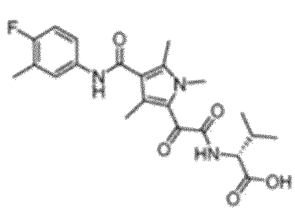,
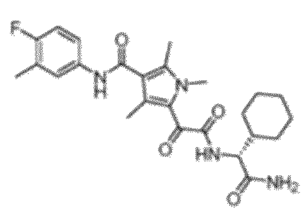,

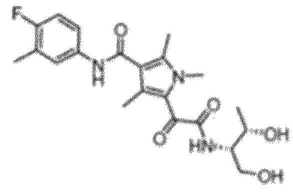, 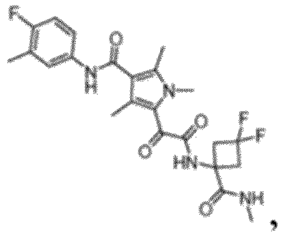, 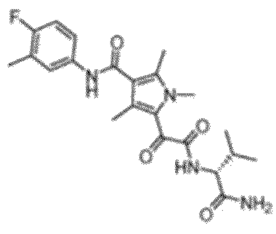,

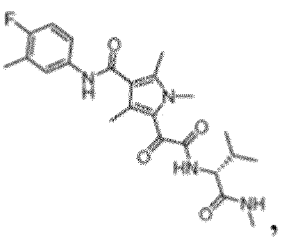, 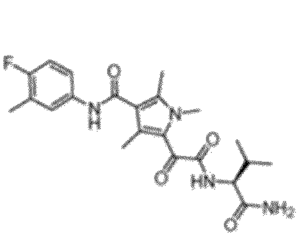, 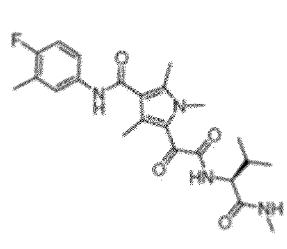,

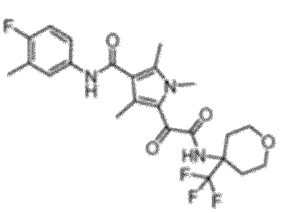, 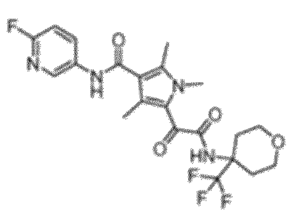, 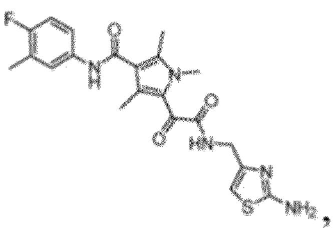,

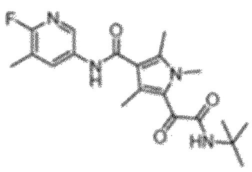, 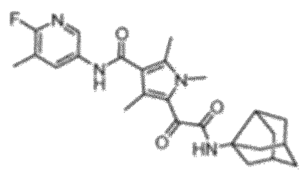, 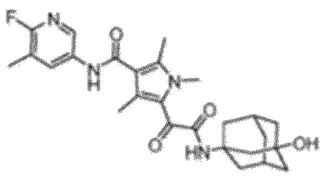,

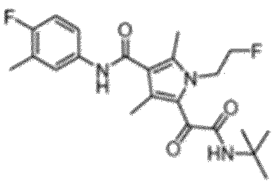, 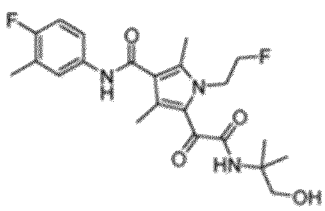, 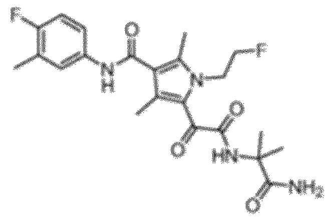,

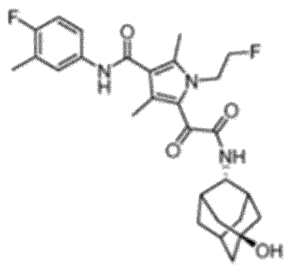, 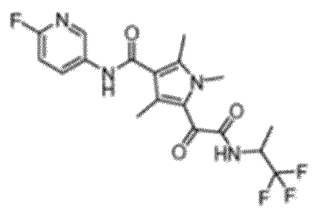, 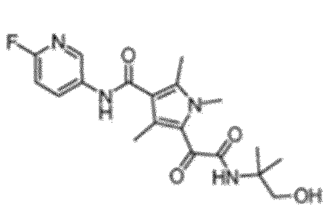,

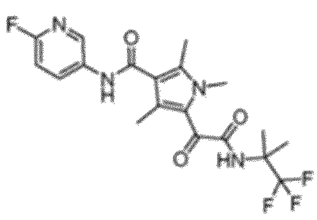,
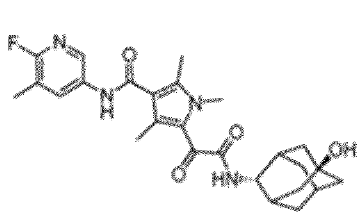,
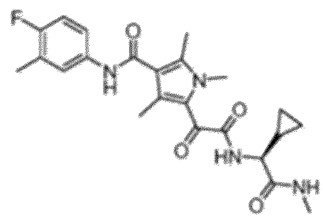,

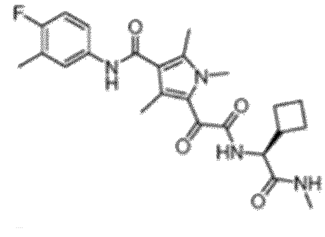,
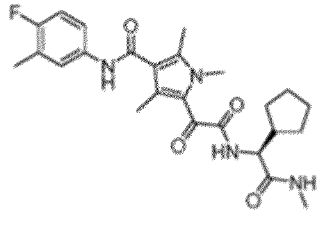,
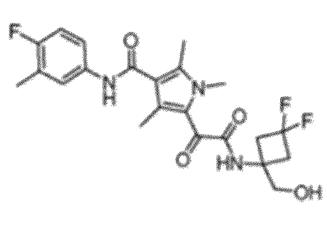,

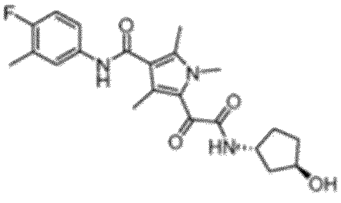,
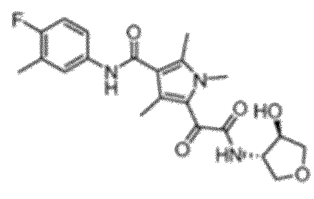,
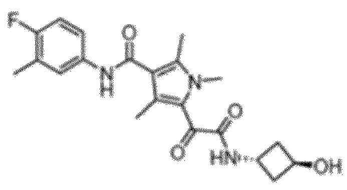,

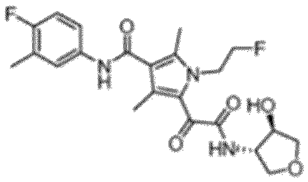,
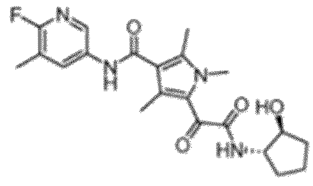,
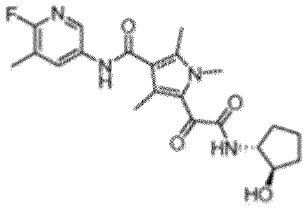,

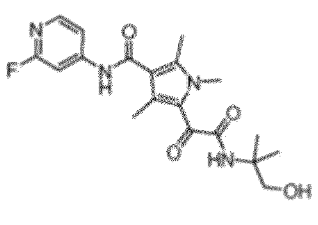,
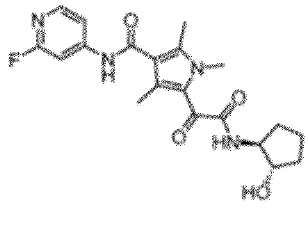,
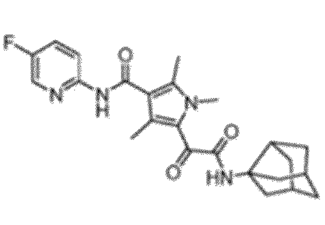,

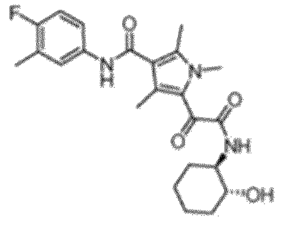,
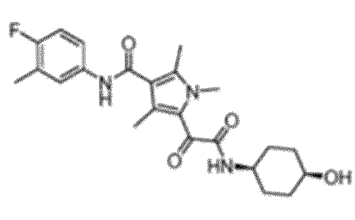,

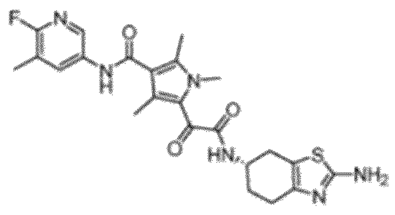,
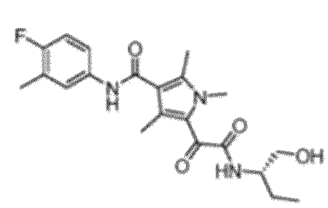,

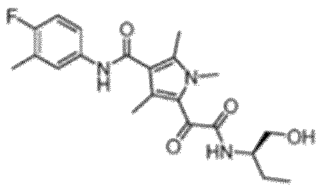,
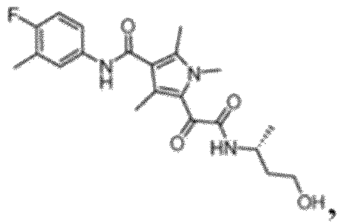,
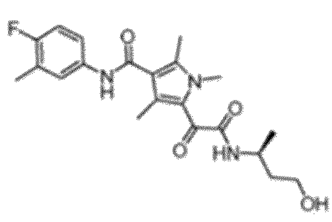,

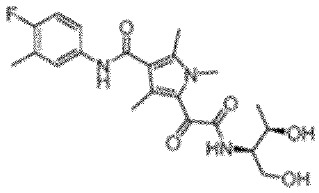,
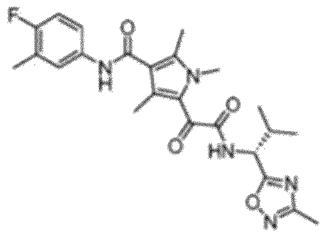,
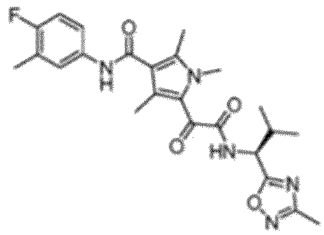,

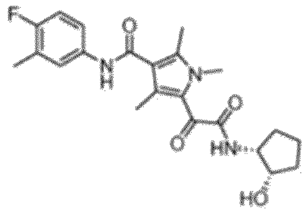,
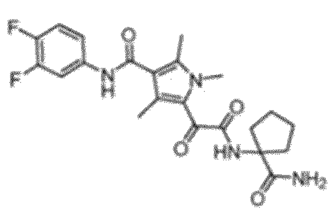,
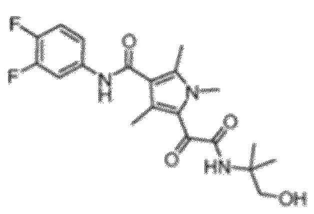,

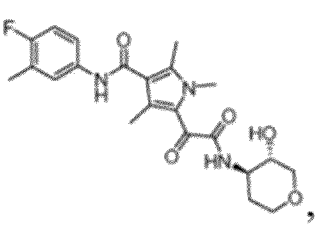,
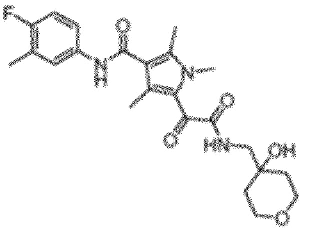,
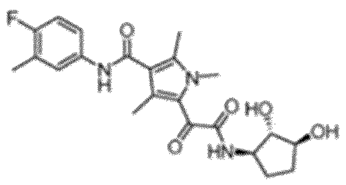,

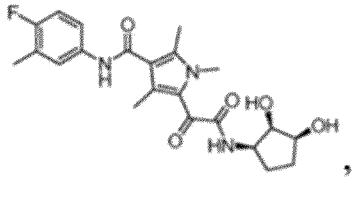,
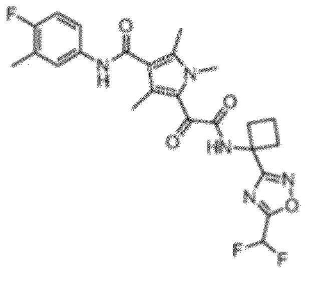,

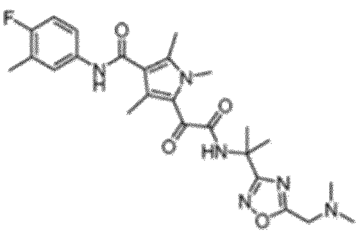,
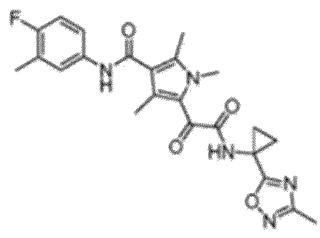,

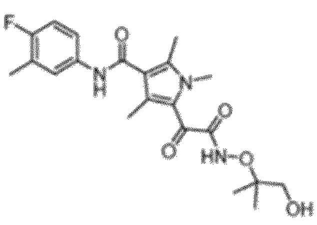,
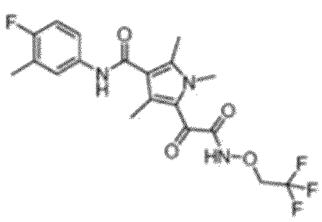,
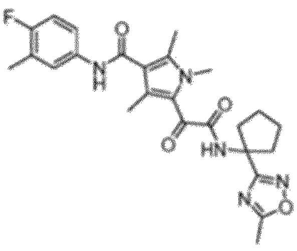,

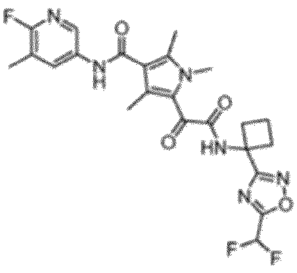,
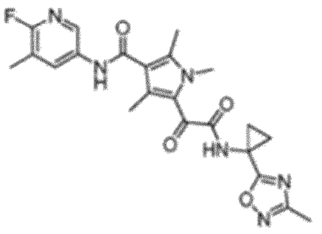,
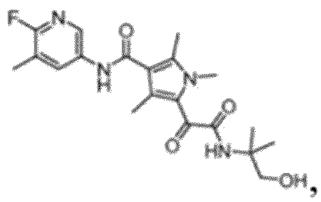,

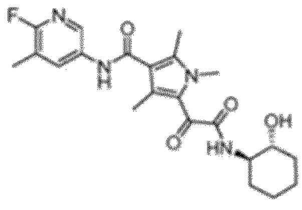,
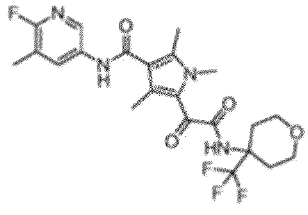,
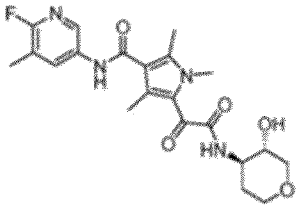,

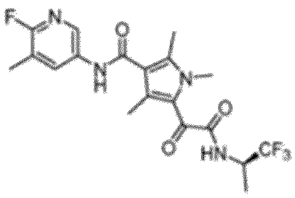,
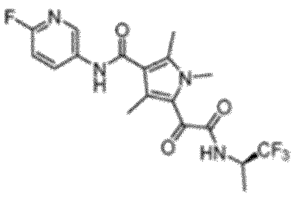,
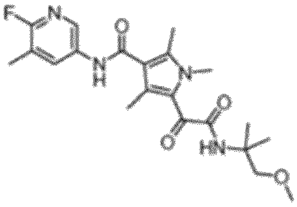,

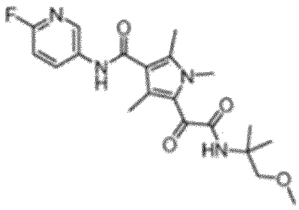,
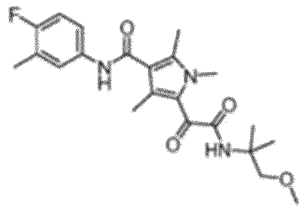,
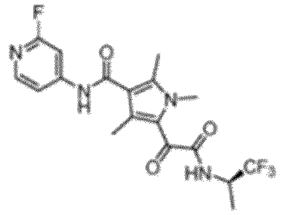,

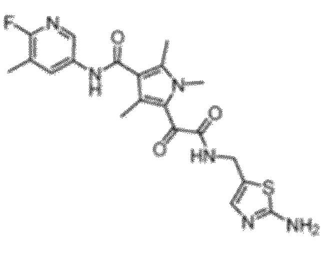,
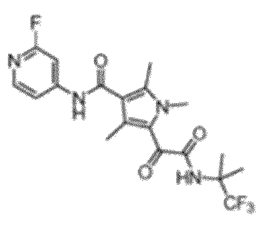,
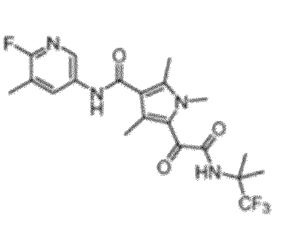,

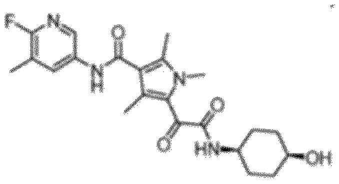,
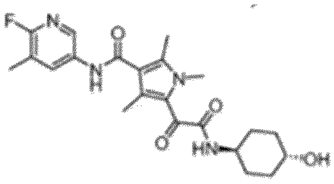,

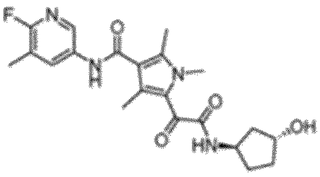,
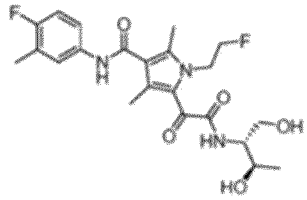,
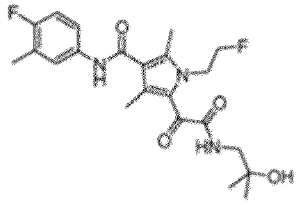,

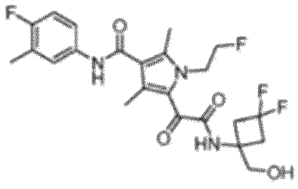,
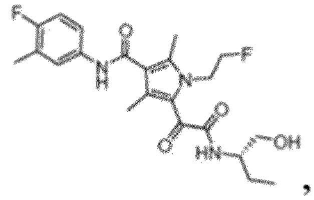,
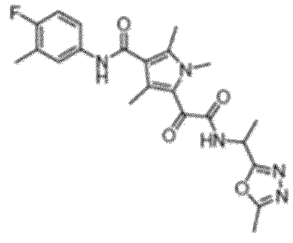,

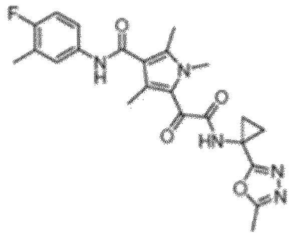,
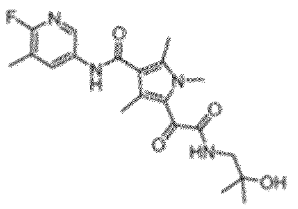,
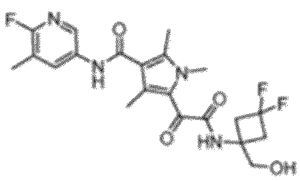,

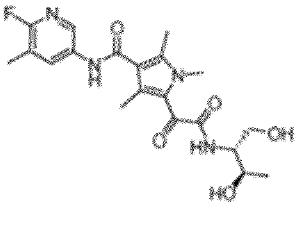,
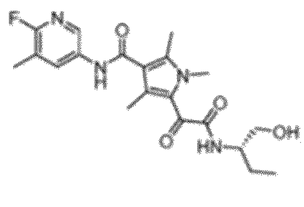,
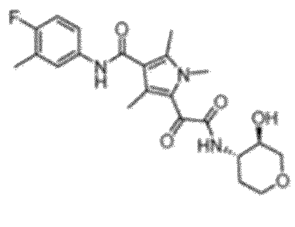,

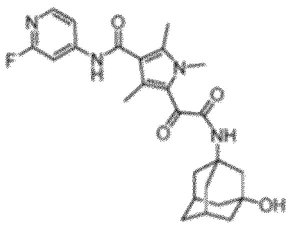,
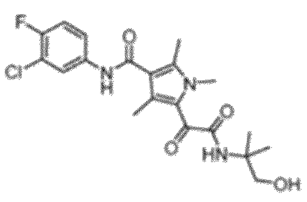,
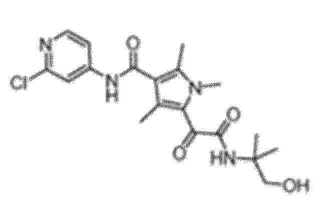,

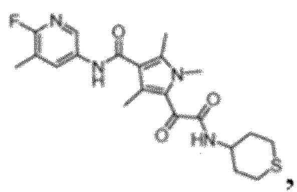, 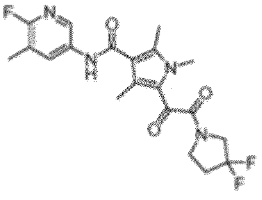, 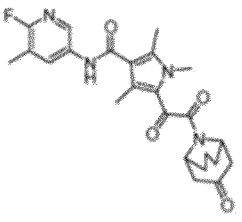,

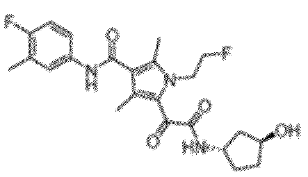, 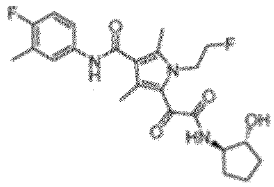, 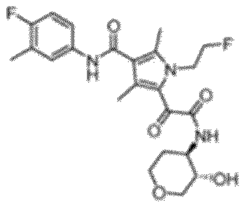,

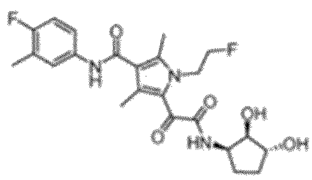, 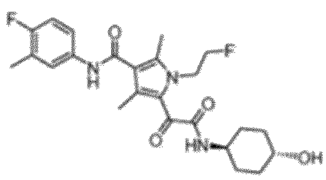, 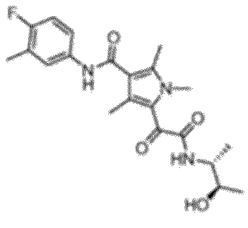,

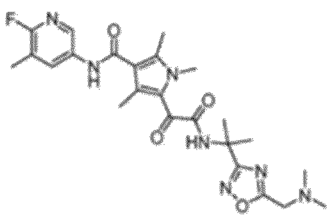, 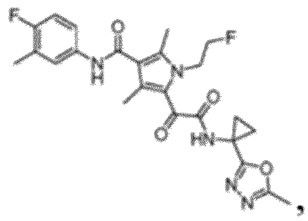, 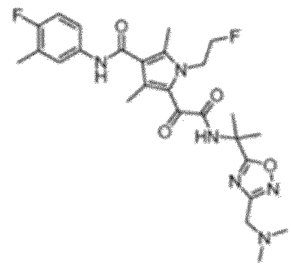,

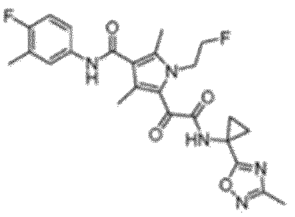, 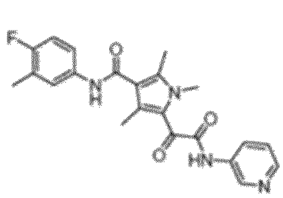, 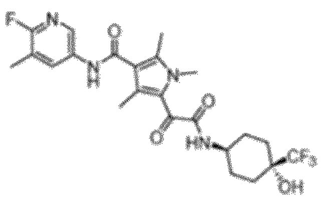,

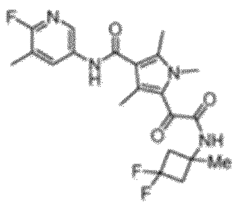, 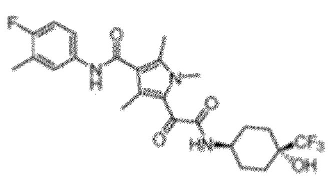, 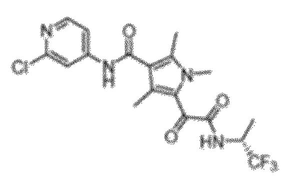,

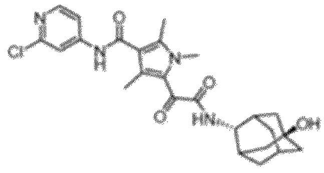, 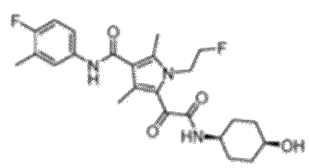,

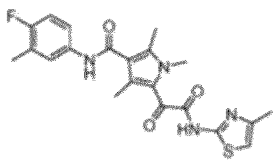, 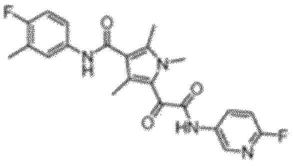,

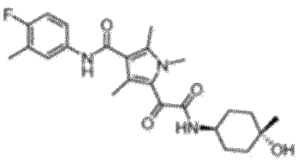, 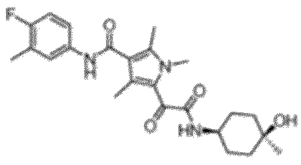,

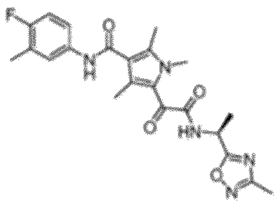, 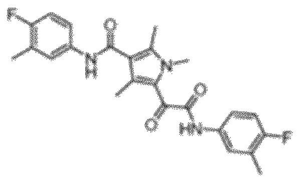, 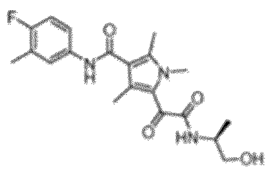,

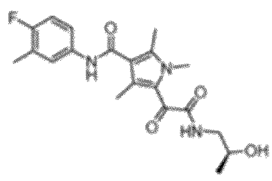, 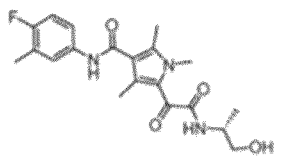, 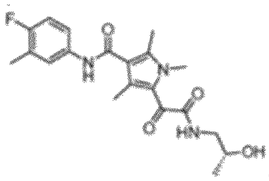,

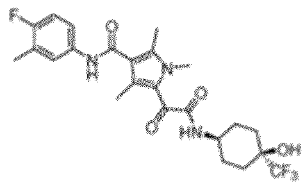, 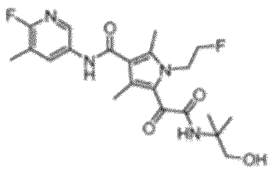, 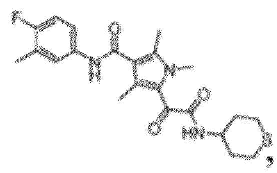,

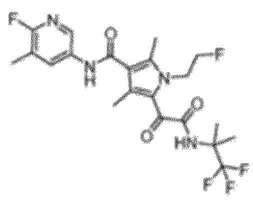, 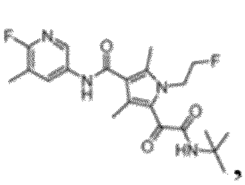, 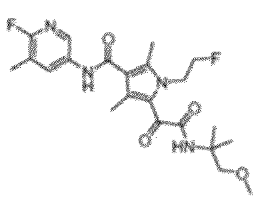,

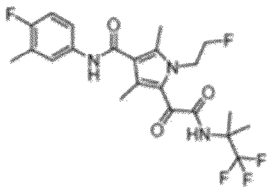,
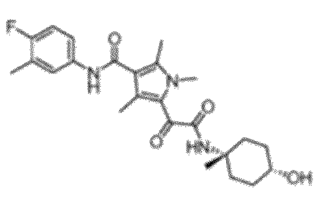,
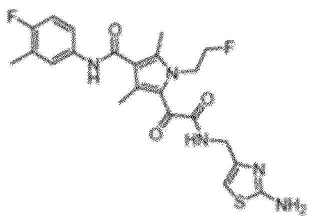,

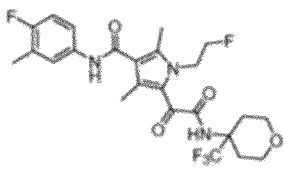,
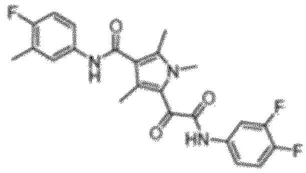,

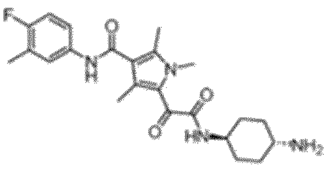,
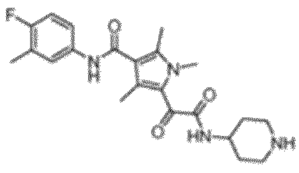,
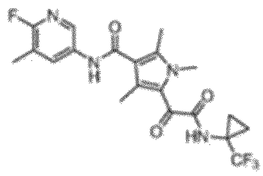,

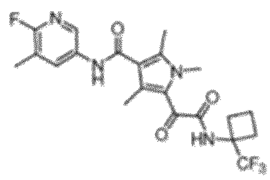,
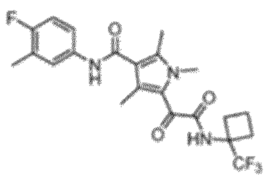,
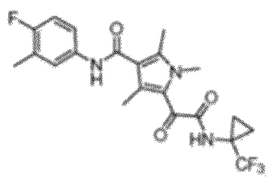,

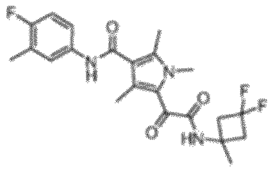,
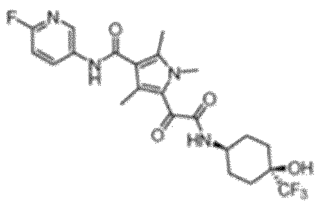,

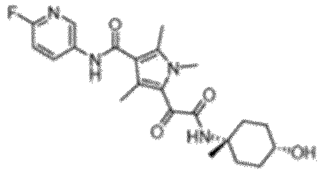,
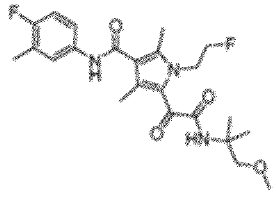,
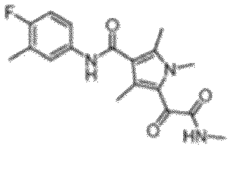,

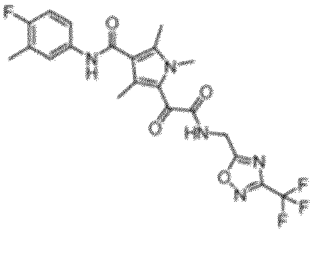,
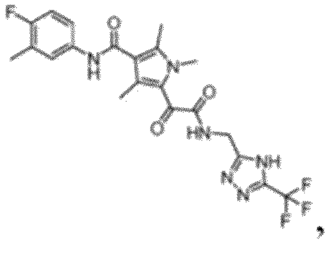,

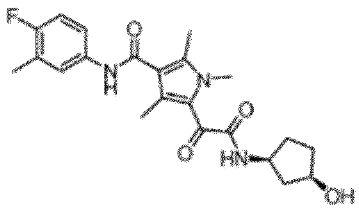,
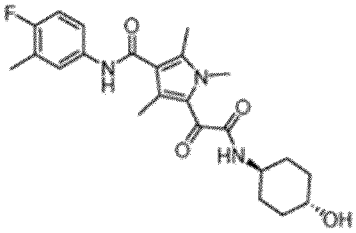,
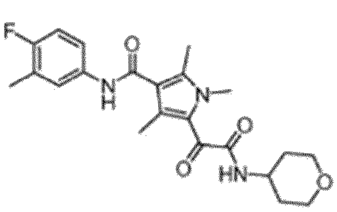,

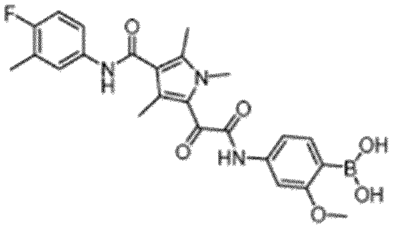,
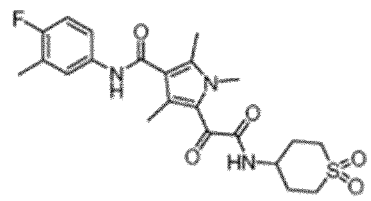,

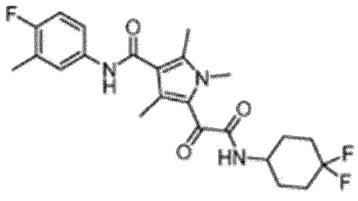,
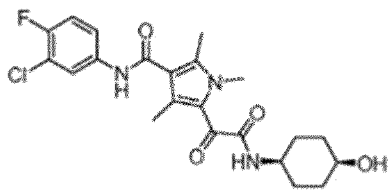,

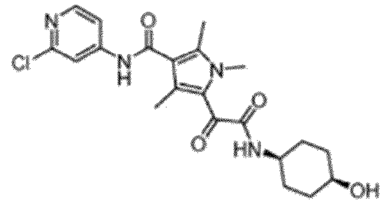,
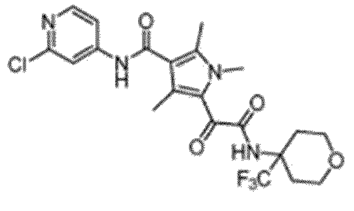,

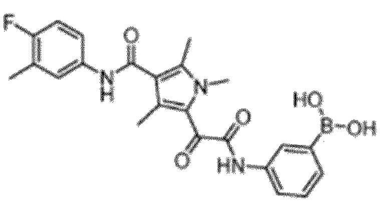,
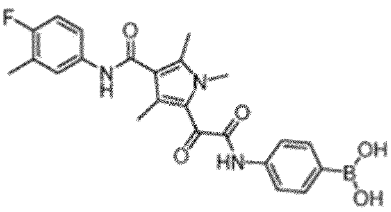,

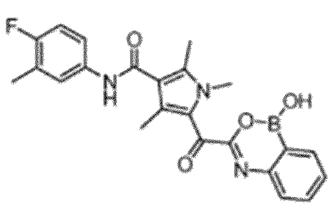,
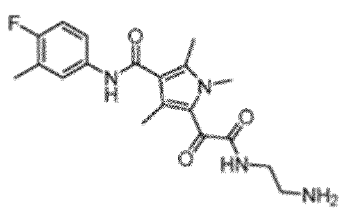,
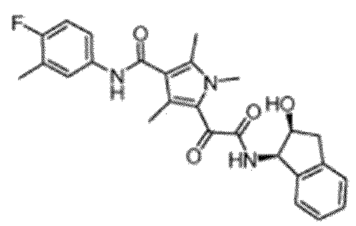,

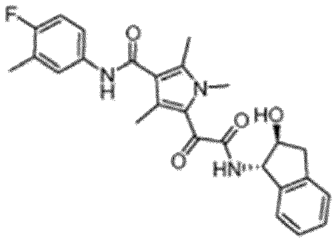,

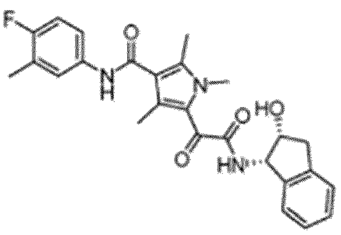,

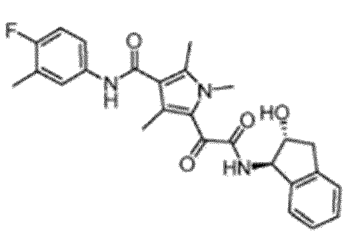,

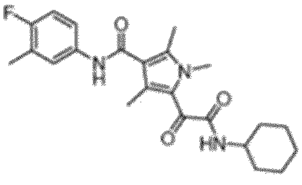,

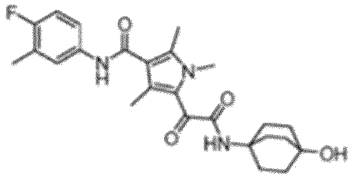,

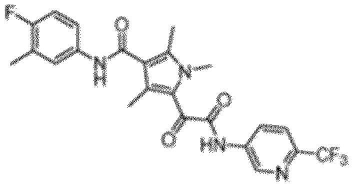,

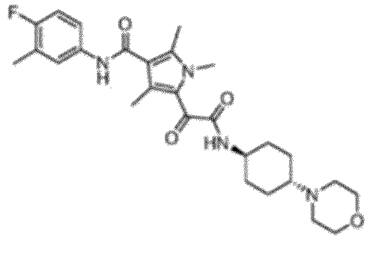,

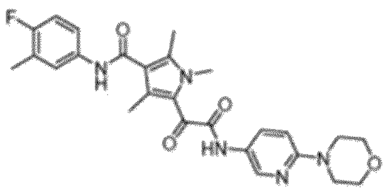,

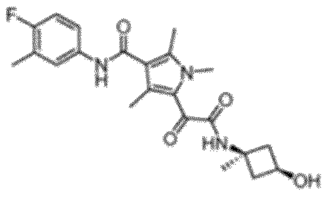,

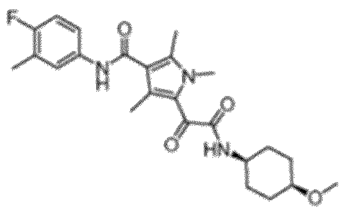,

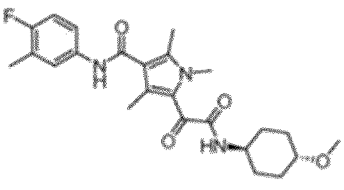,

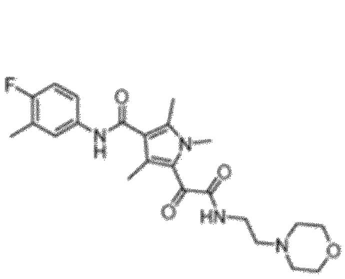,

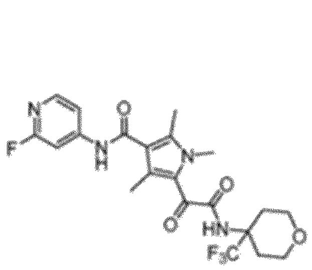,

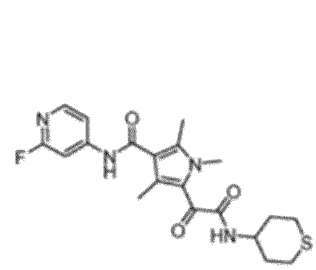,

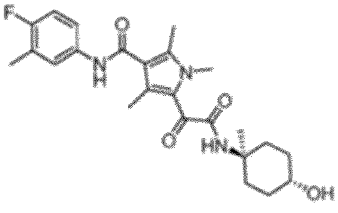,
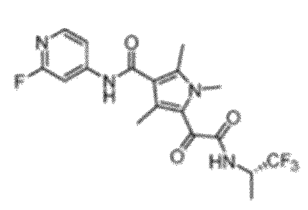,
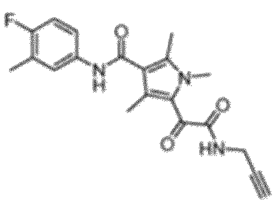,

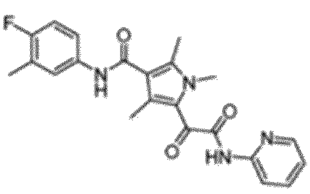,
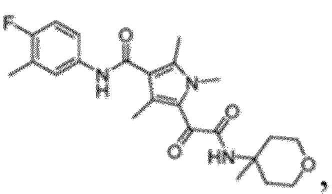,
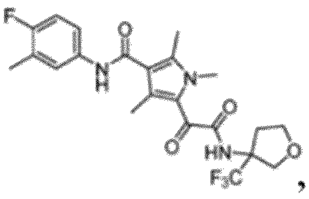,

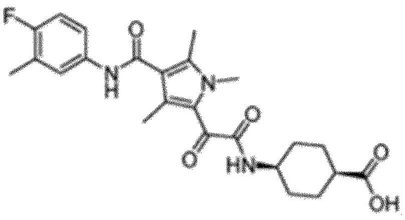,
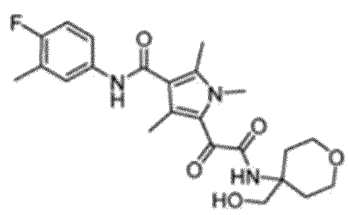,

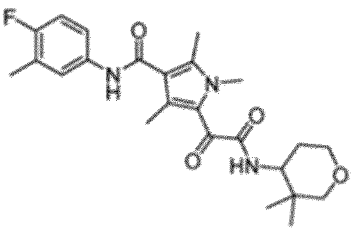,
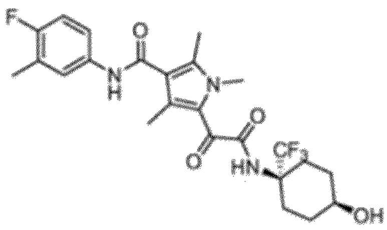,

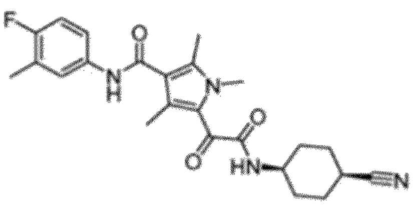,
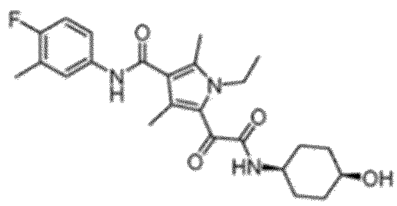,

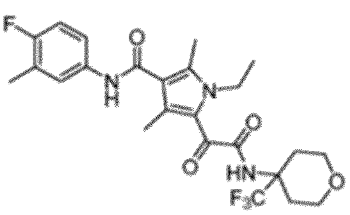,
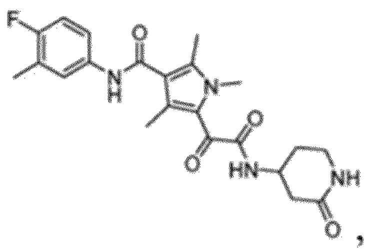,

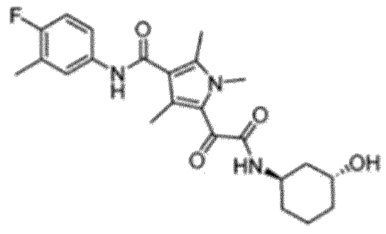,

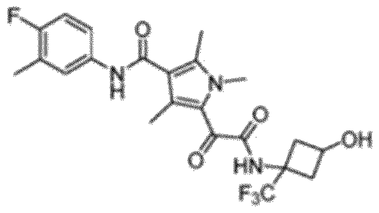,

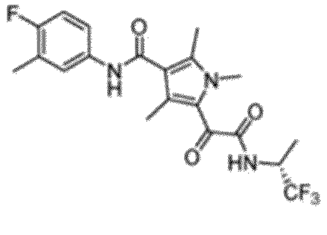,

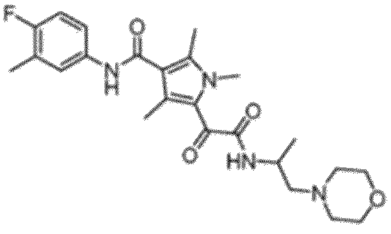,

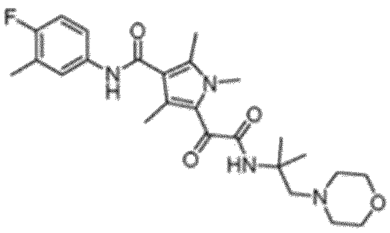,

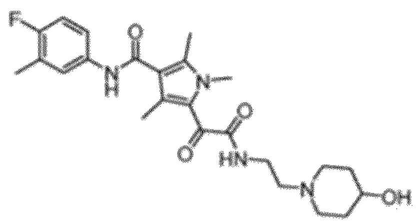,

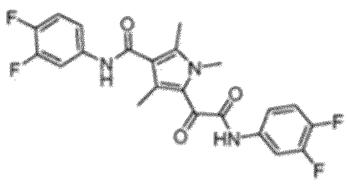,

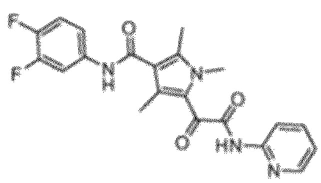,

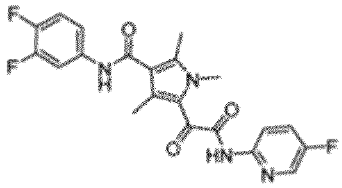,

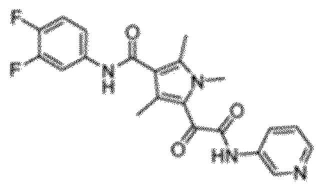,

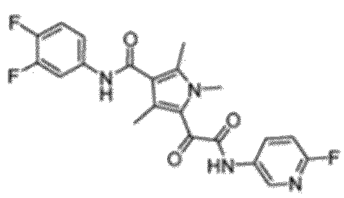,

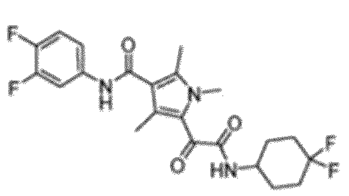,

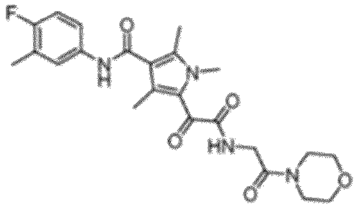,

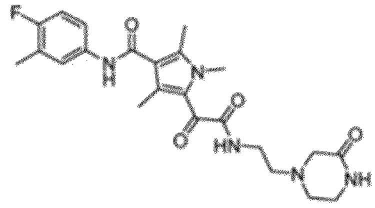,

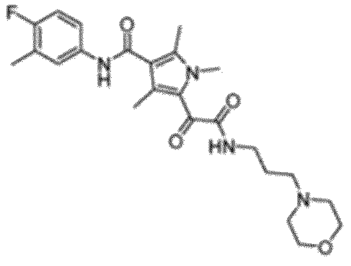 , 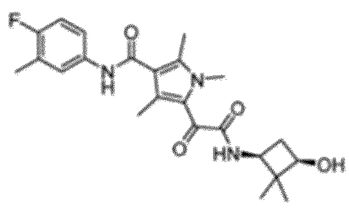 ,

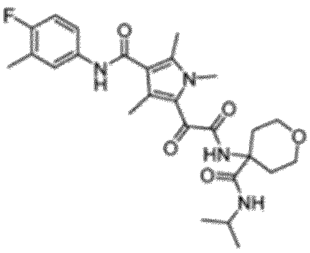 , 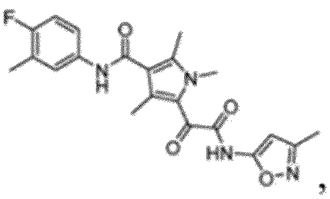 , 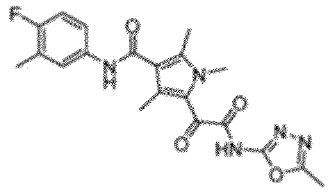 ,

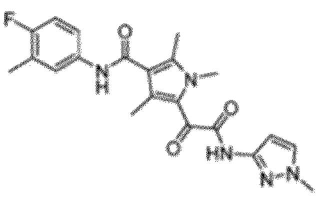 , 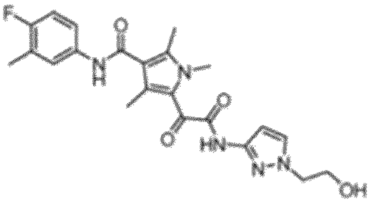 ,

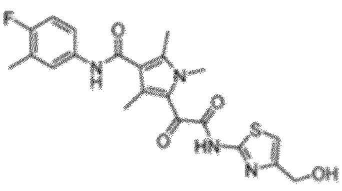 , 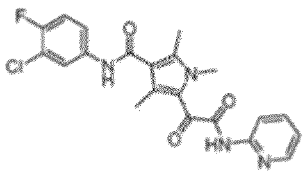 ,

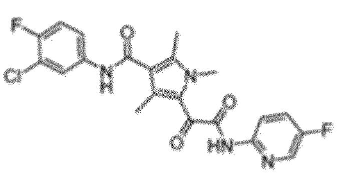 , 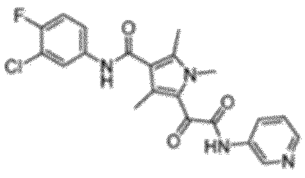 ,

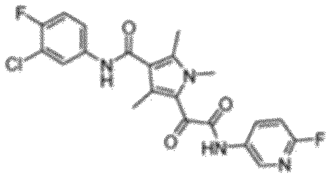 , 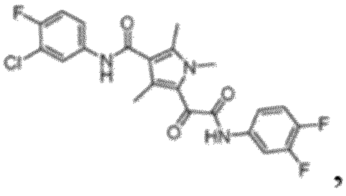 ,

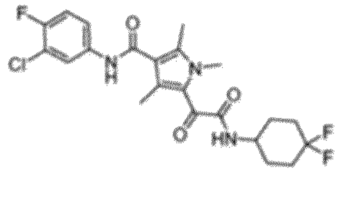 , 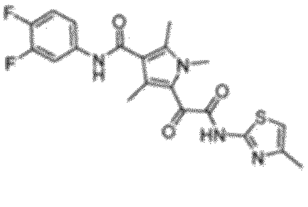 ,

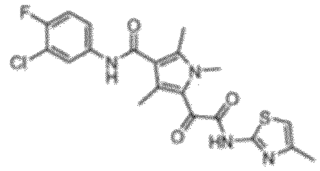 ,

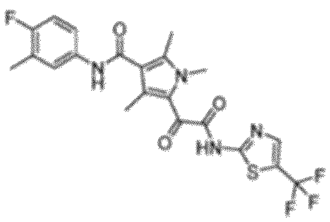 ,

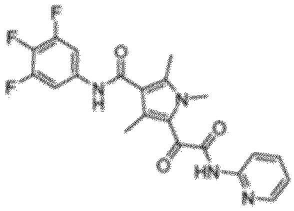 ,

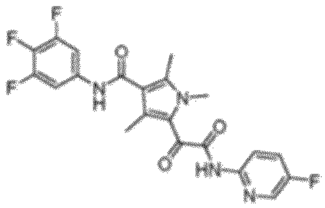 ,

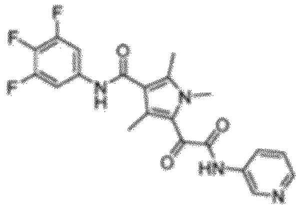 ,

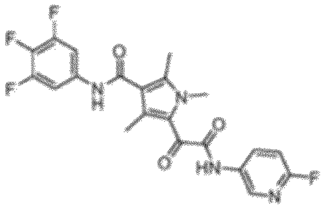 ,

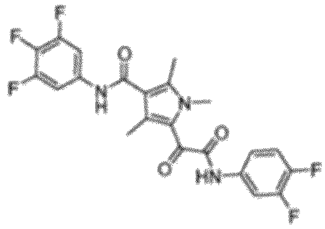 ,

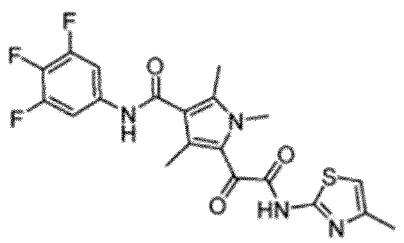 ,

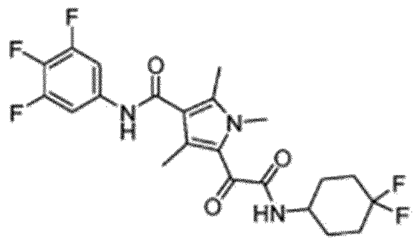 ,

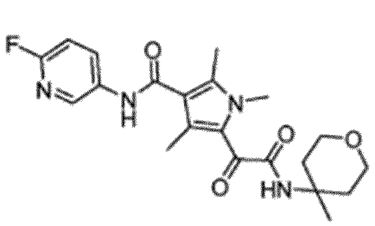 ,

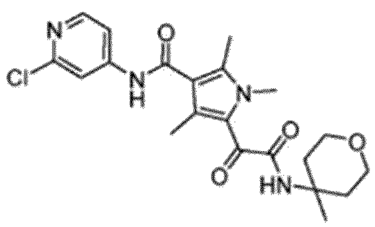 ,

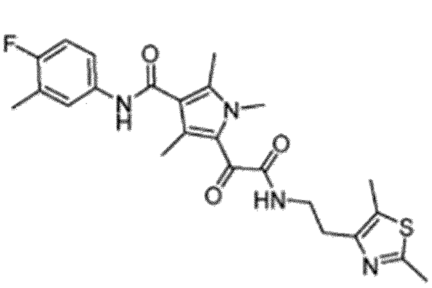 ,

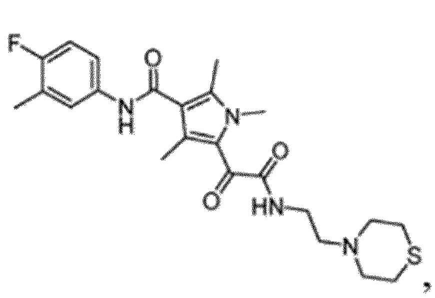 ,

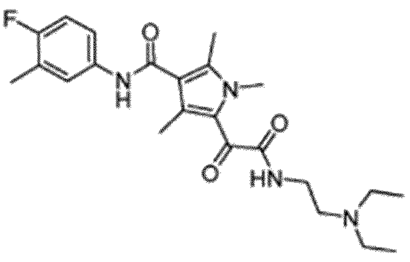,

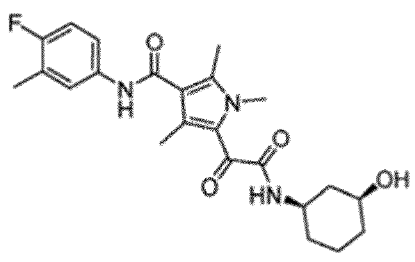,

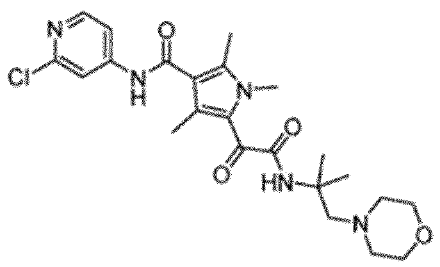,

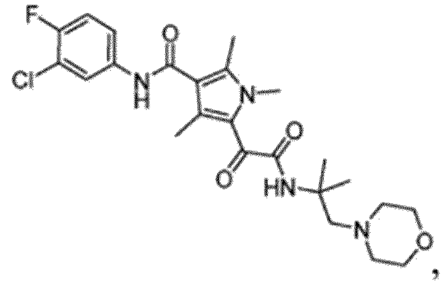,

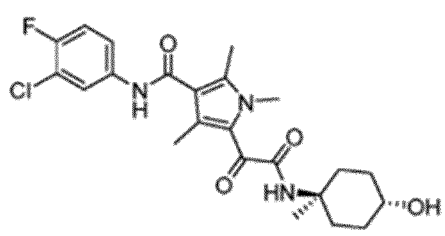,

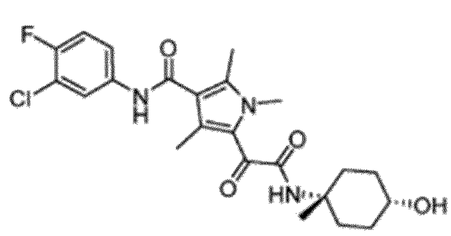,

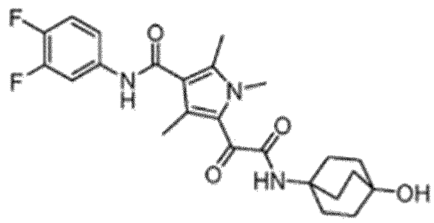,

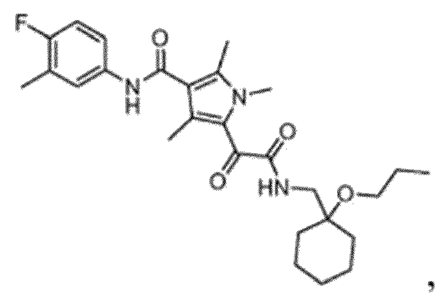,

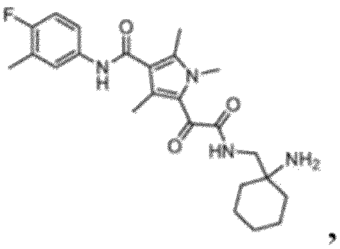,

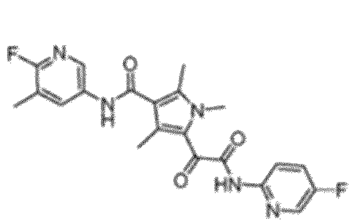,

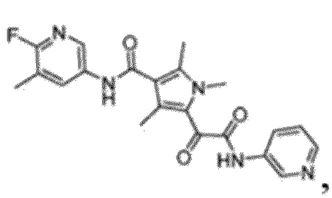,

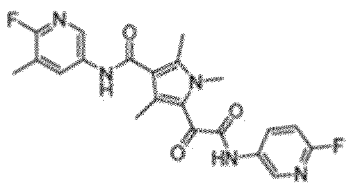,

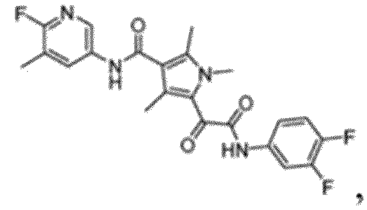 , 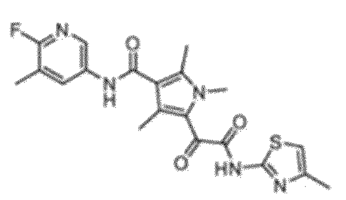 ,

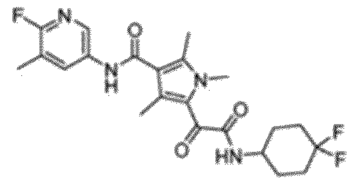 , 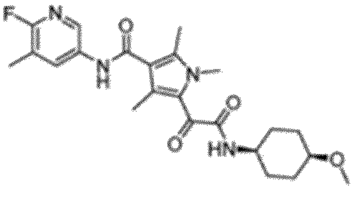 ,

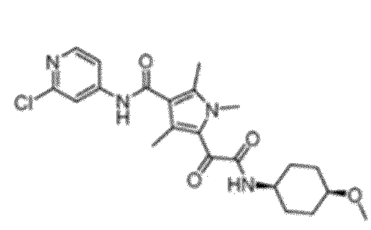 , 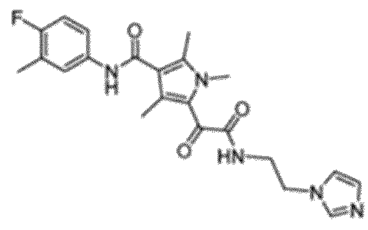 ,

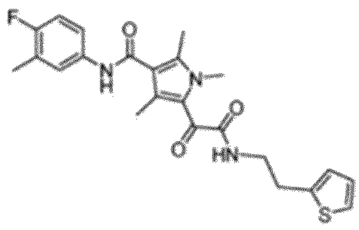 , 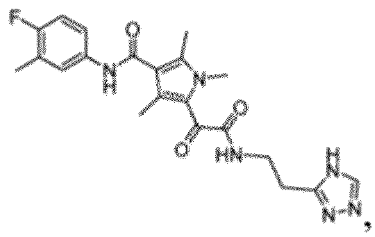 ,

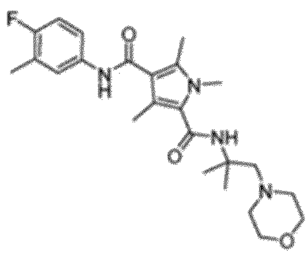 , 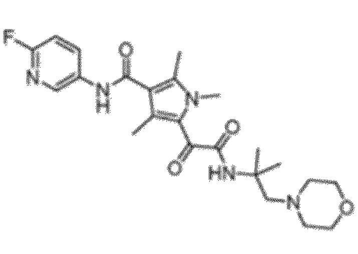 ,

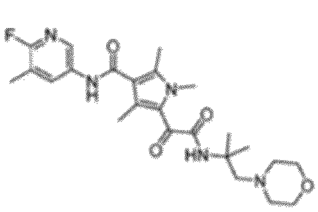 , 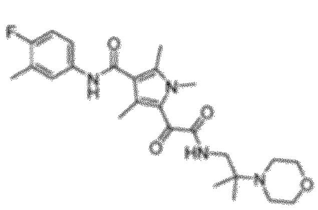 ,

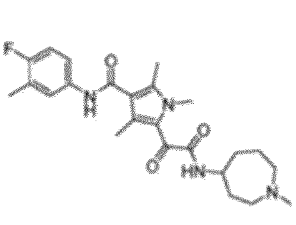 , 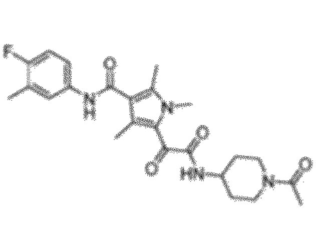 ,

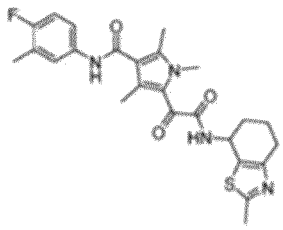,
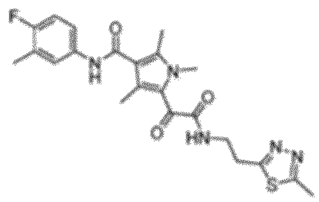,

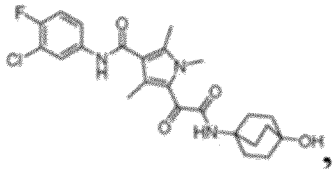,
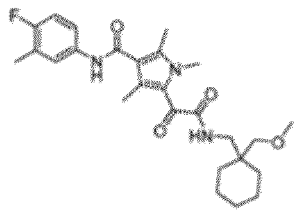,

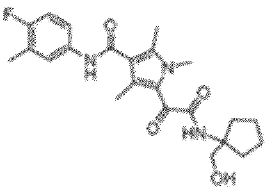,
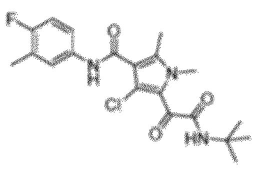,
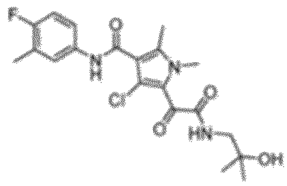,

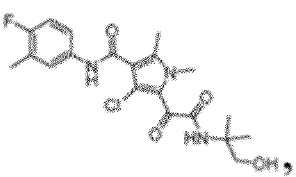,
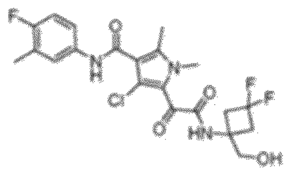,
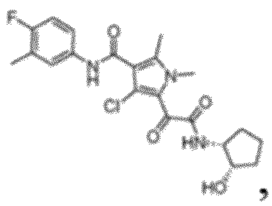,

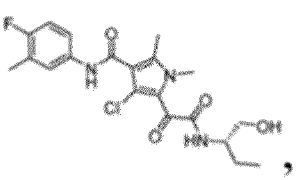,
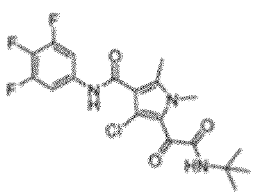,
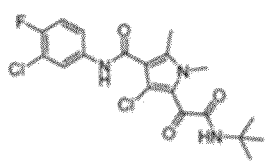,

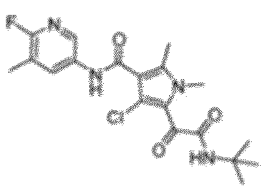,
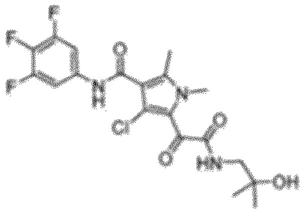,
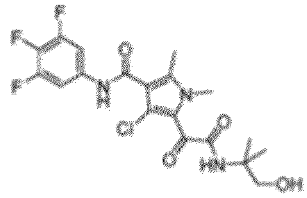,

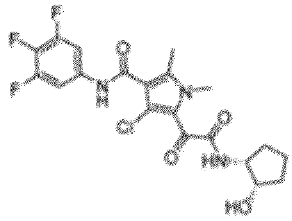,
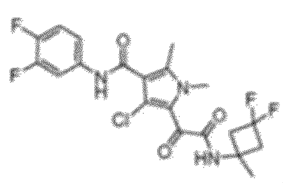,
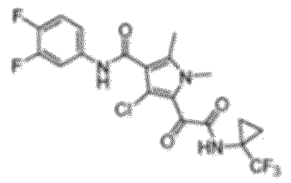,

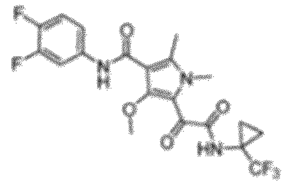,
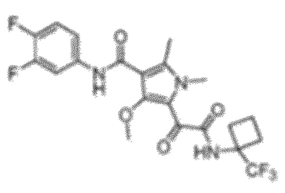,
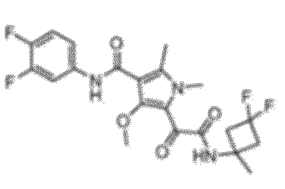,

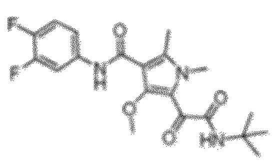,
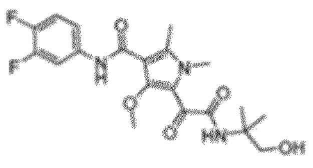,
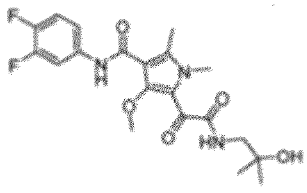,

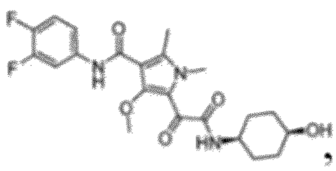,
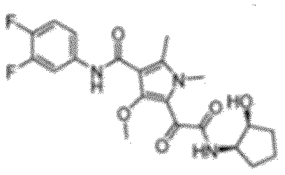,
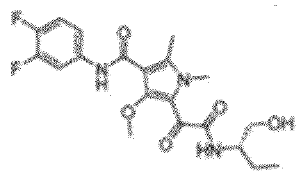,

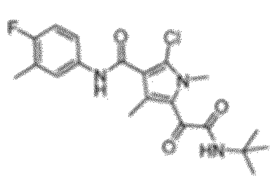,
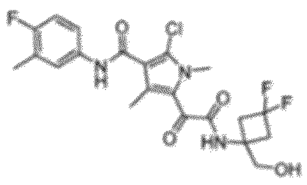,
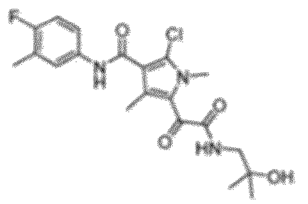,

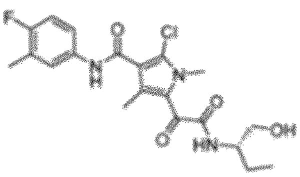,
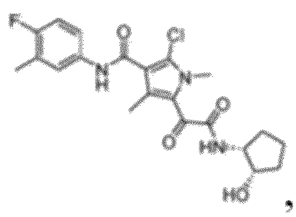,
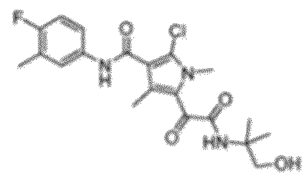,

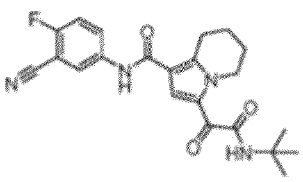,
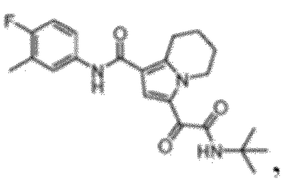,
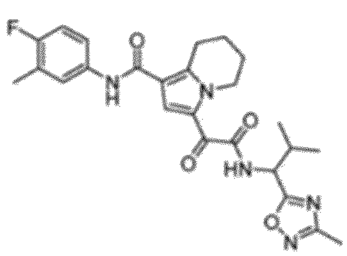,

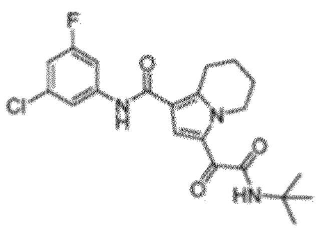,
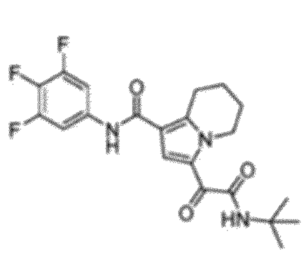,
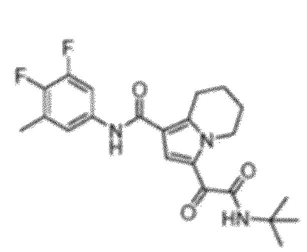,

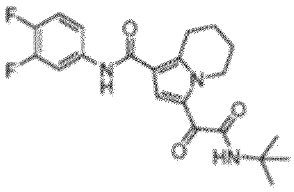, 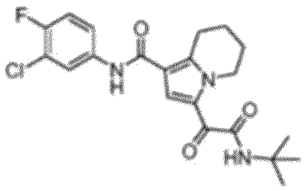, 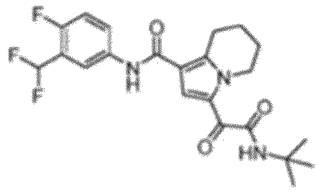,

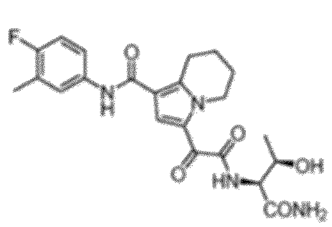, 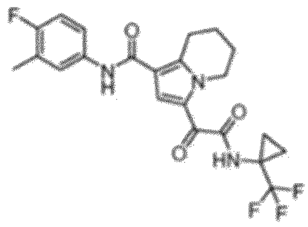, 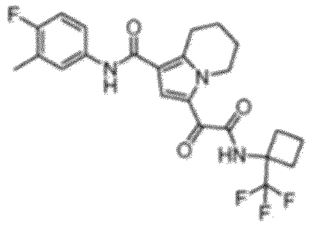,

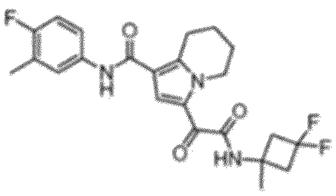, 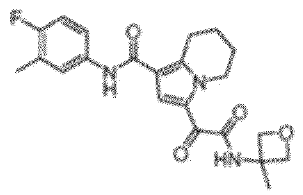, 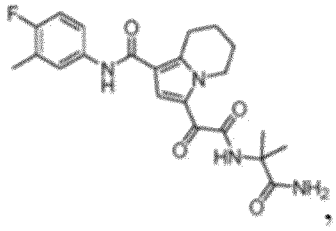,

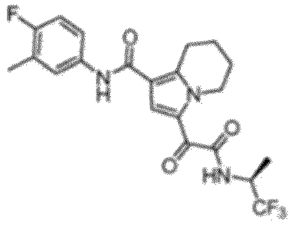, 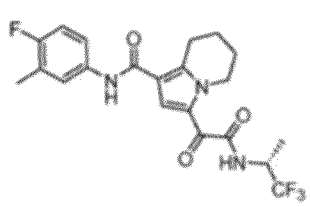, 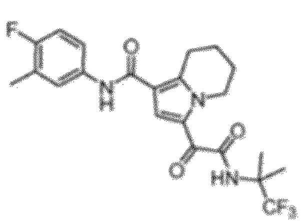,

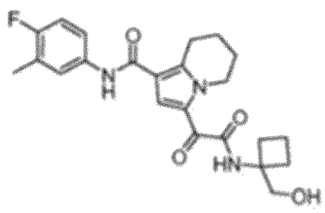, 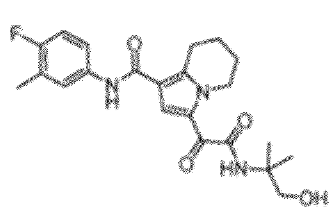, 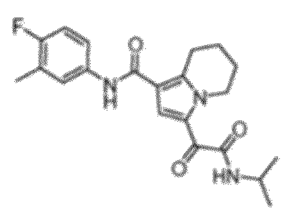,

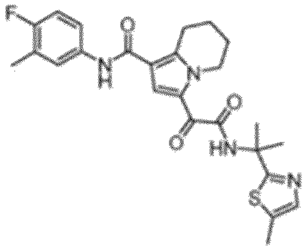, 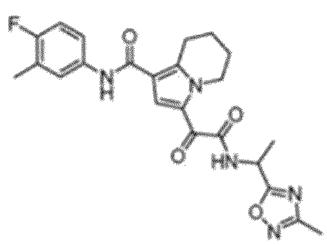, 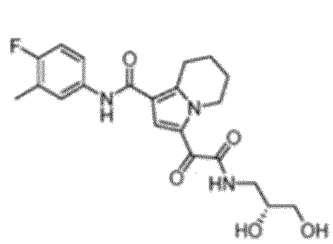,

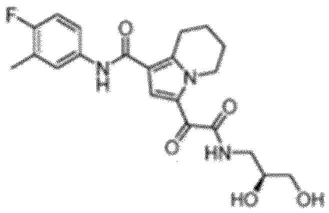 , 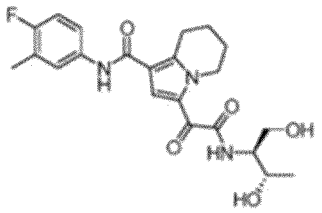 , 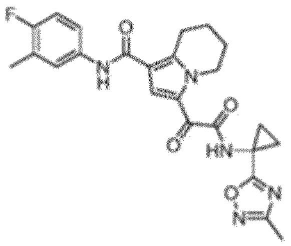 ,

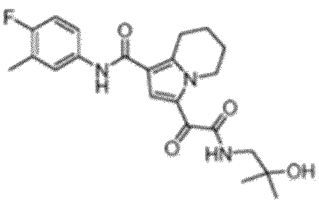 , 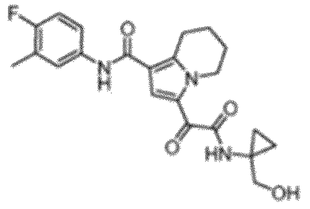 , 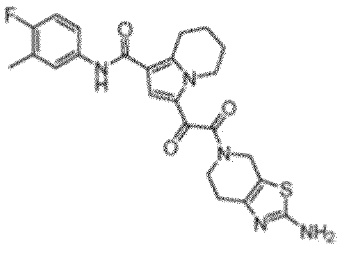 ,

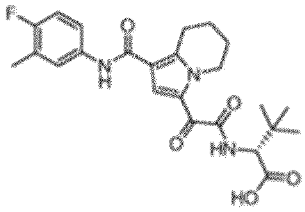 , 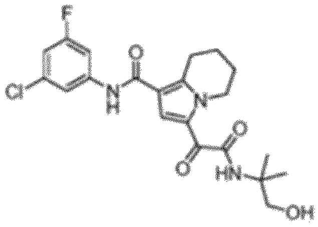 , 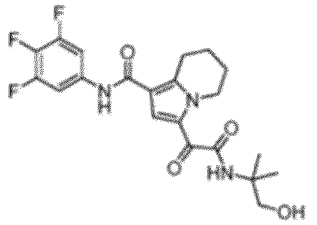 ,

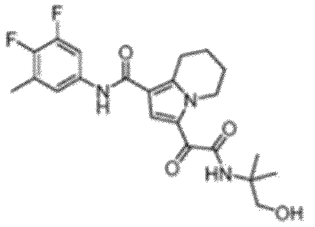 , 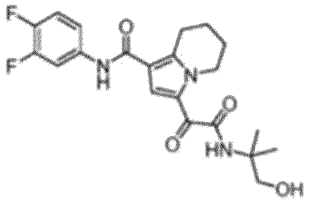 , 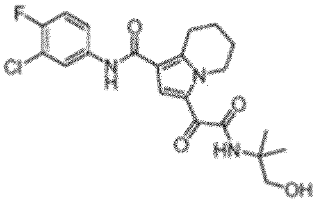 ,

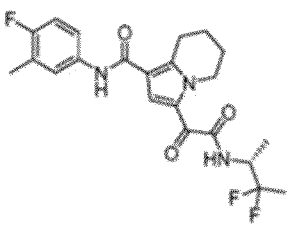 , 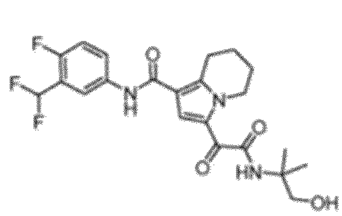 , 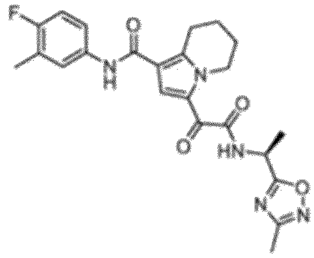 ,

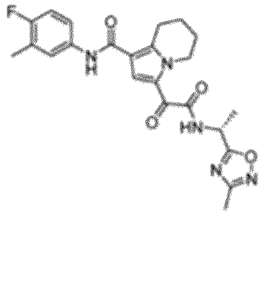 , 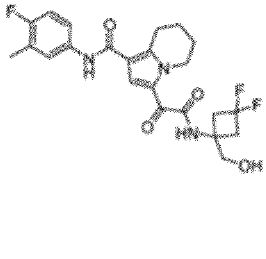 , 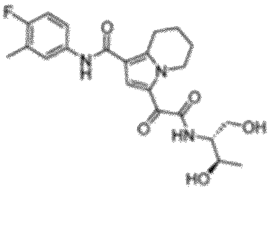 ,

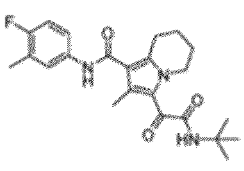 , 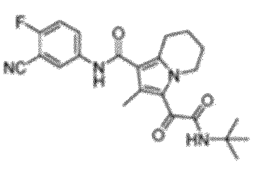 , 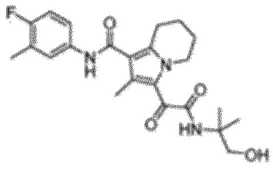 ,

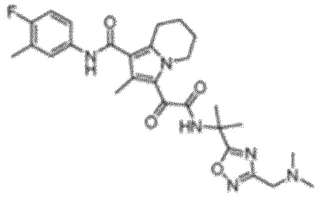 , 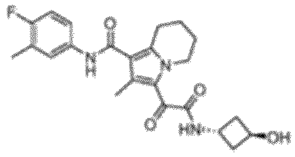 ,

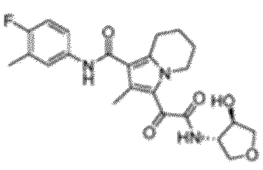 , 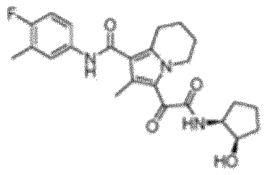 , 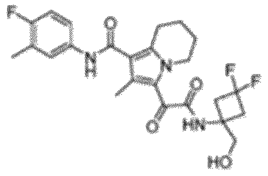 ,

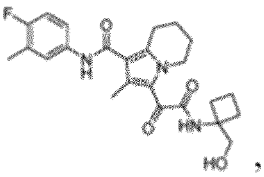 , 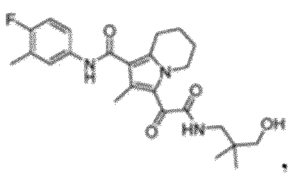 , 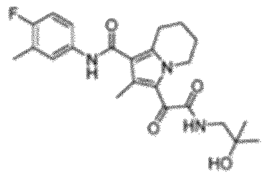 ,

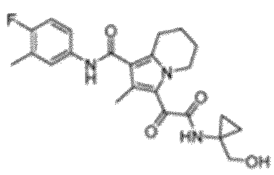 , 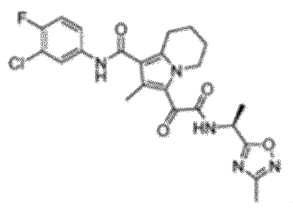 , 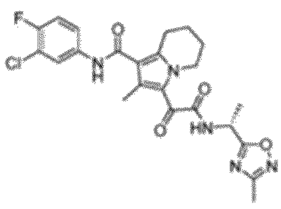 ,

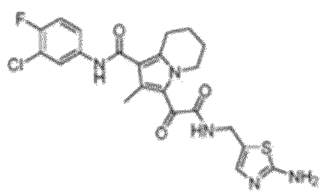 , 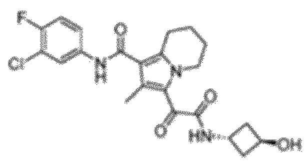 ,

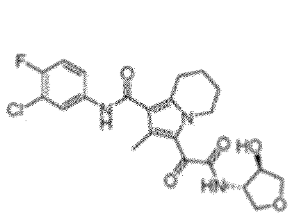 , 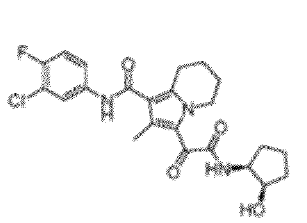 , 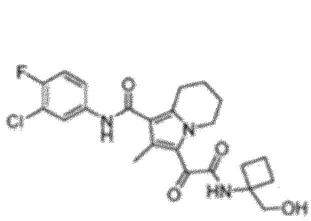 ,

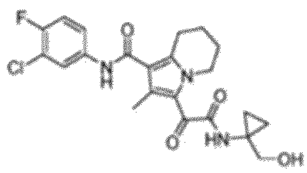,

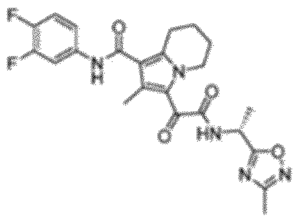,

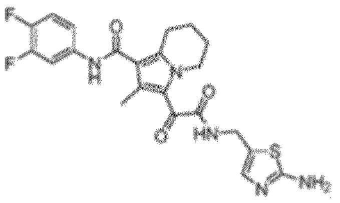,

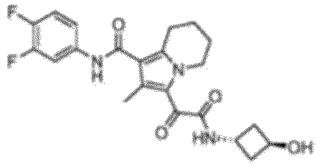,

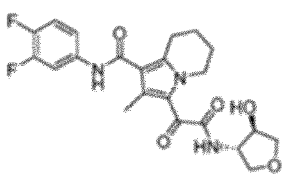,

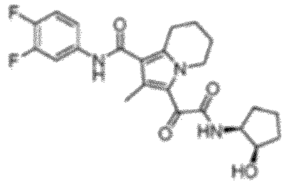,

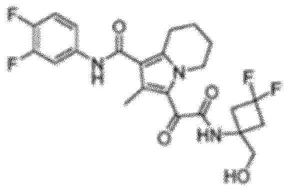,

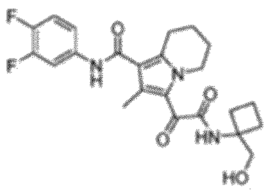,

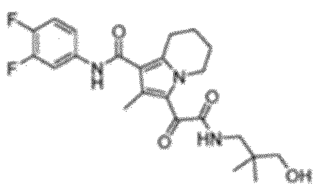,

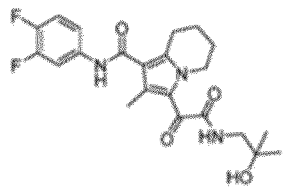,

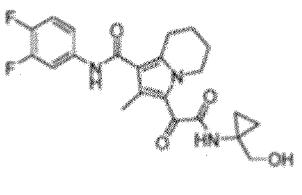,

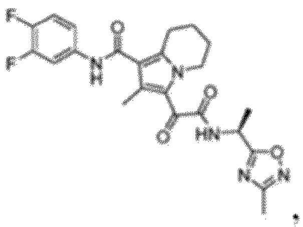,

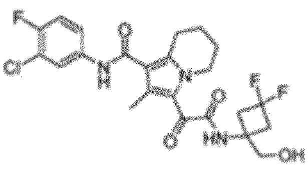,

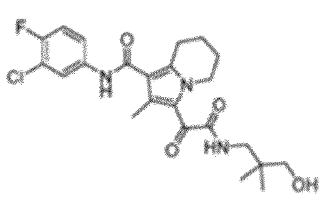,

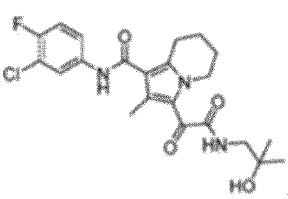,

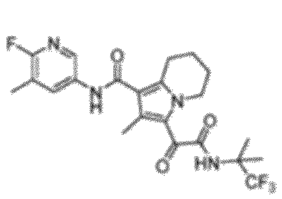,

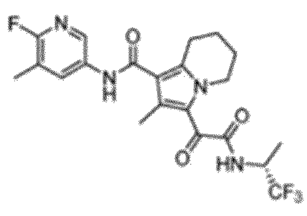,
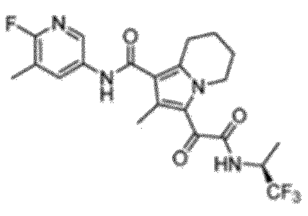,
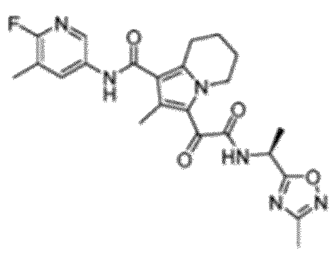,

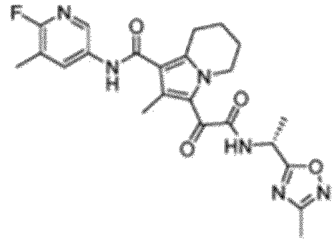,
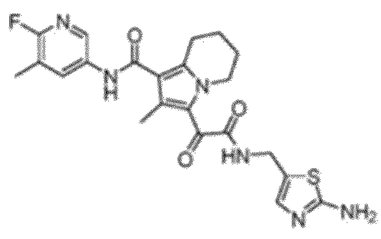,

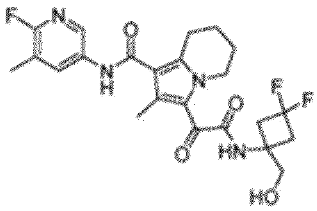,
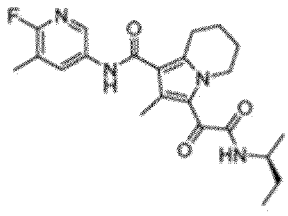,
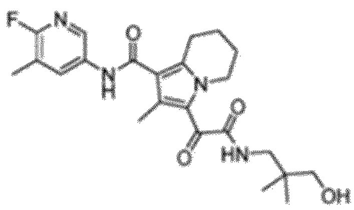,

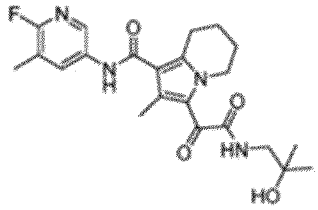,
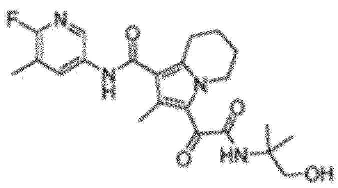,
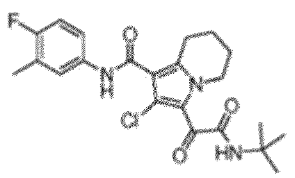,

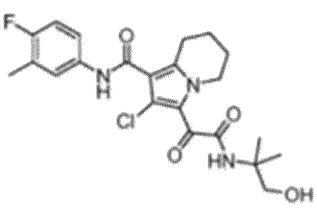,
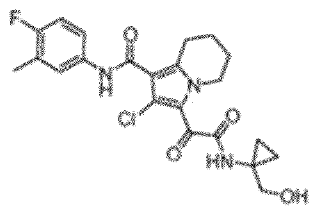,
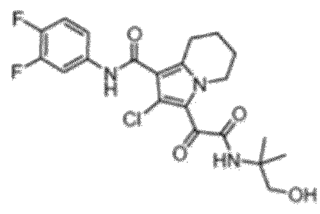,

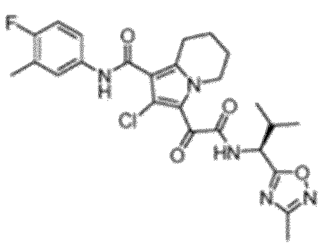,
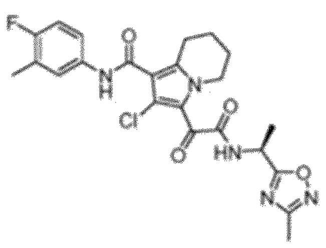,
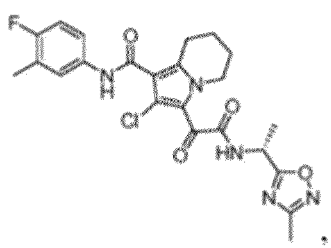,

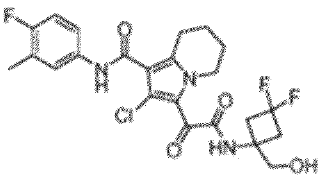 , 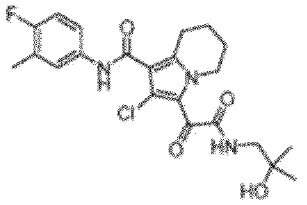 , 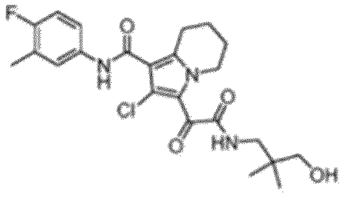 ,

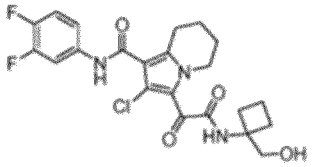 , 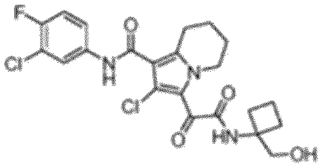 , 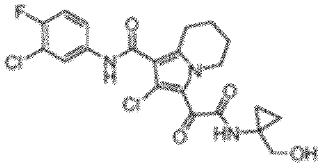 ,

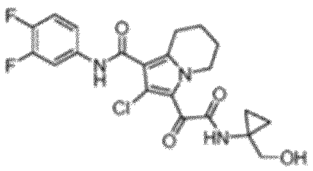 , 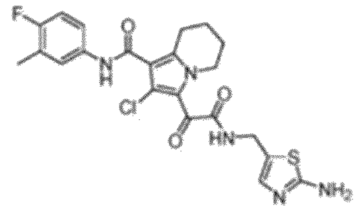 ,

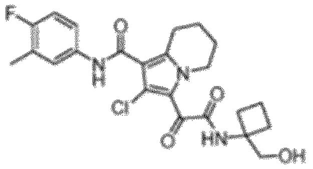 , 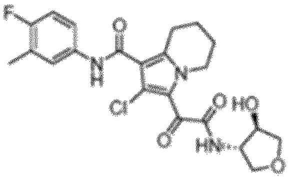 , 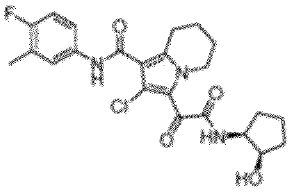 ,

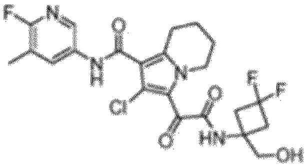 , 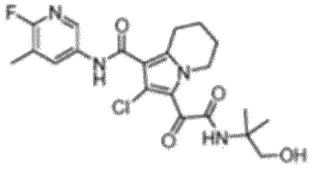 , 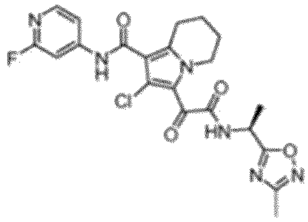 ,

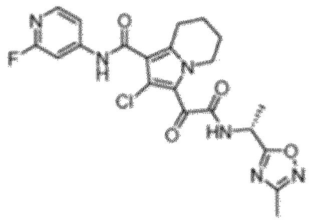 , 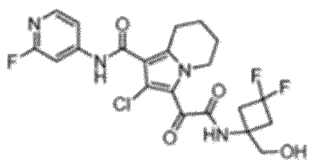 , 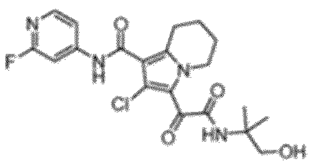 ,

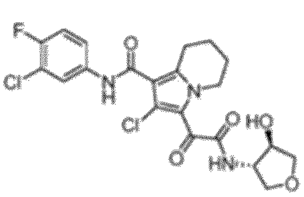 , 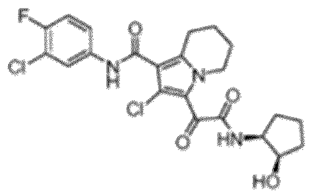 , 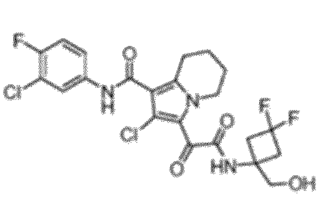 ,

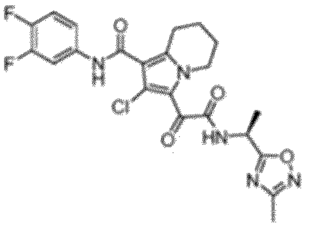 , 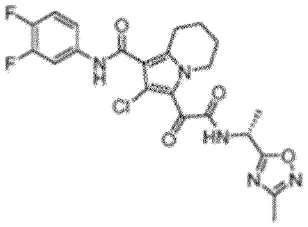 , 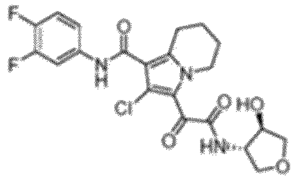 ,

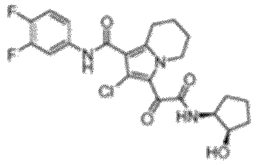 , 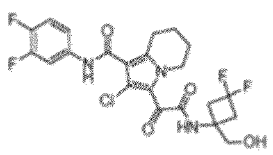 , 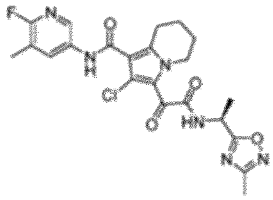 ,

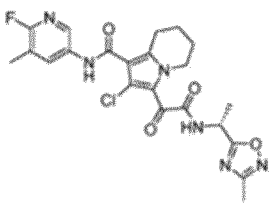 , 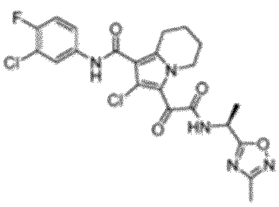 , 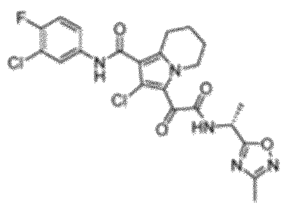 ,

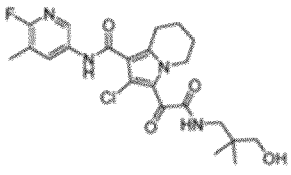 , 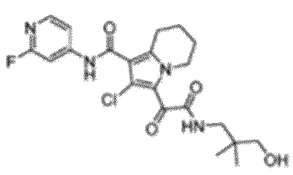 ,

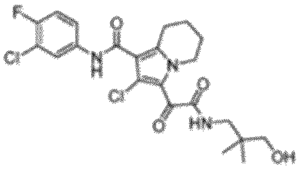 , 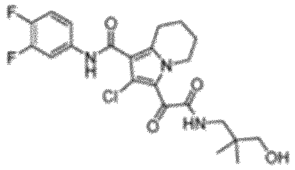 ,

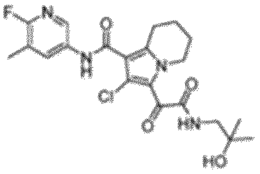 , 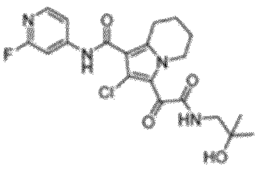 , 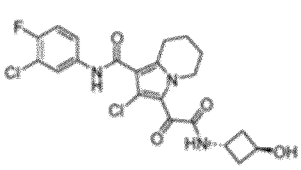 ,

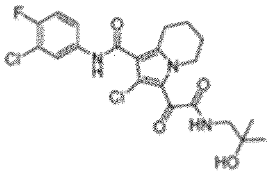 , 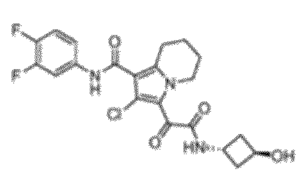 , 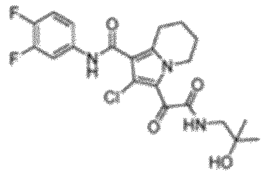 ,

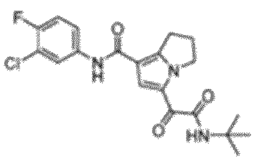, 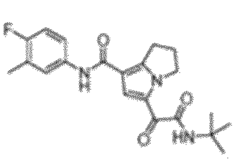, 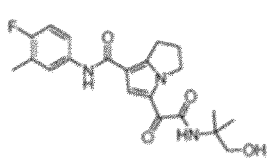,

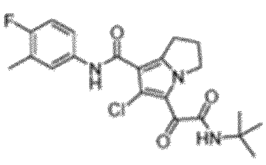, 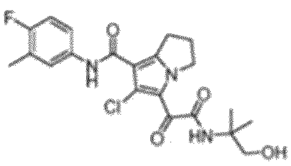, 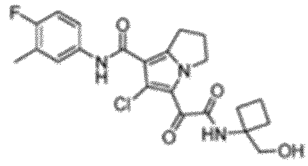,

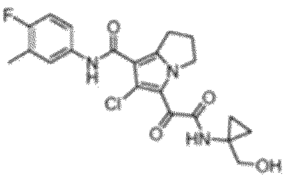, 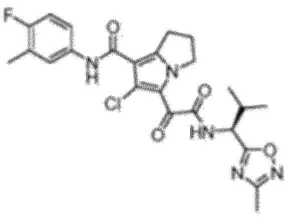, 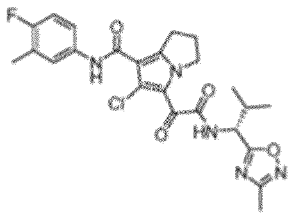,

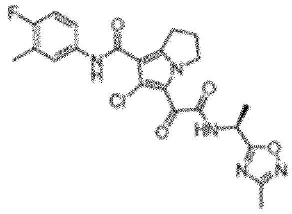, 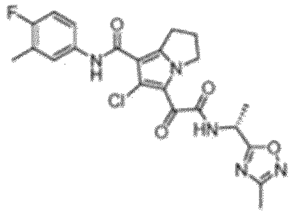, 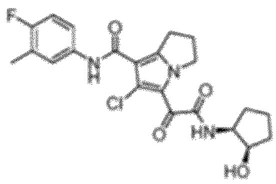,

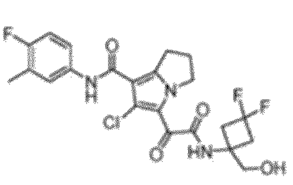, 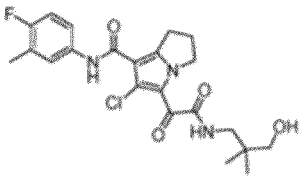, 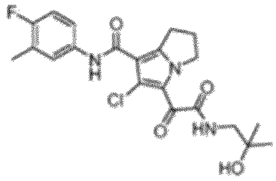,

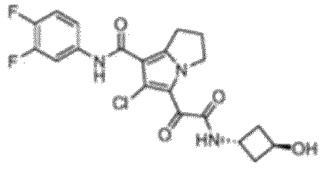, 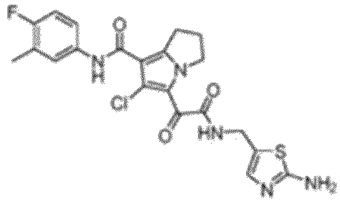,

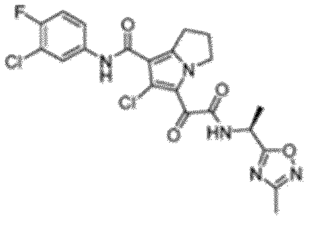, 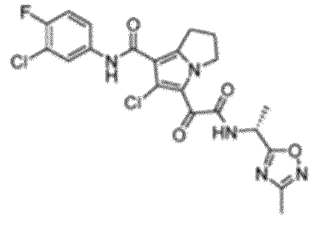,

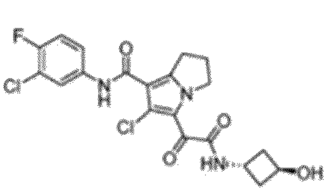 , 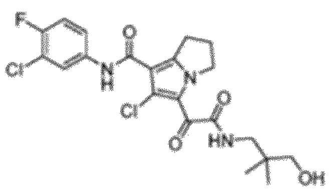 ,

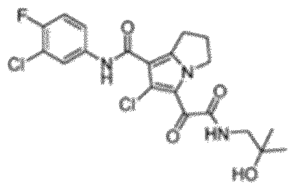 , 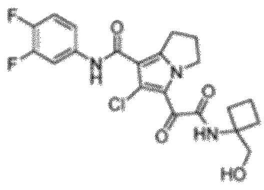 , 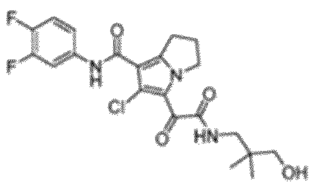 ,

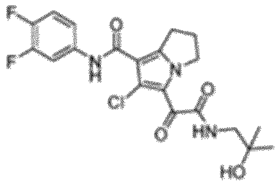 , 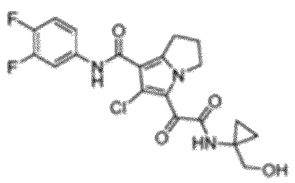 ,

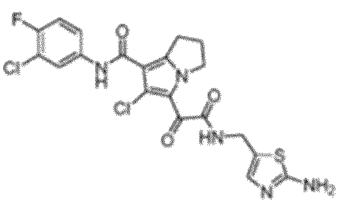 , 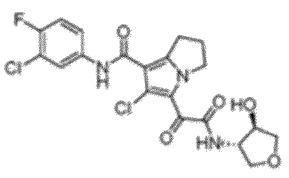 ,

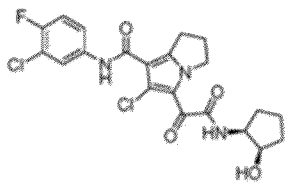 , 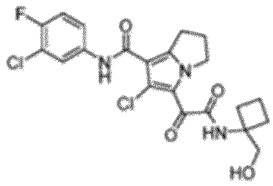 , 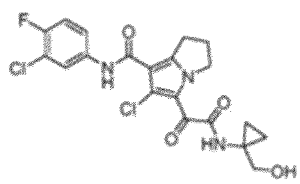 ,

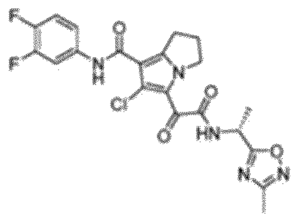 , 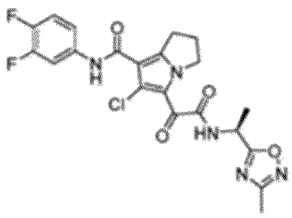 ,

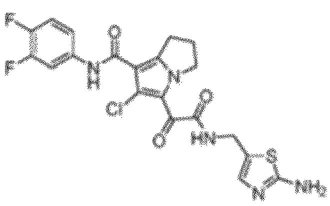 , 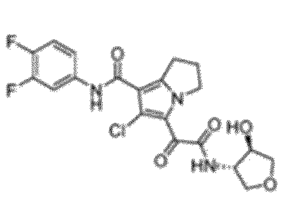 , 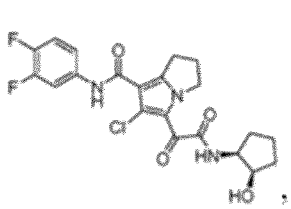 ,

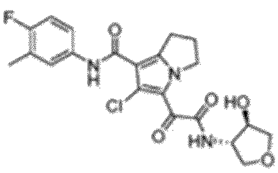,
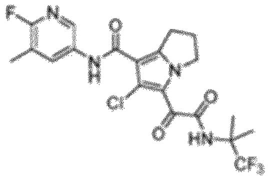,
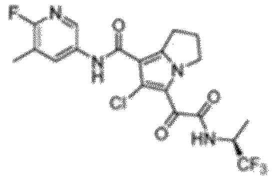,

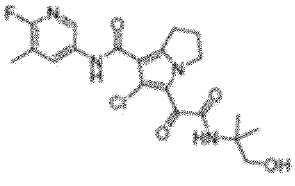,
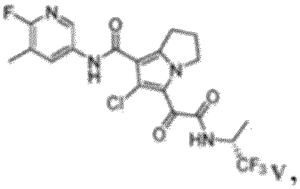,
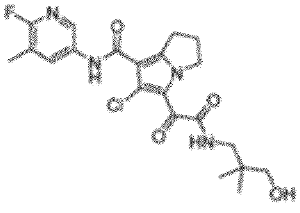,

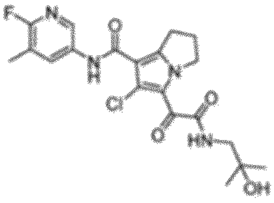,
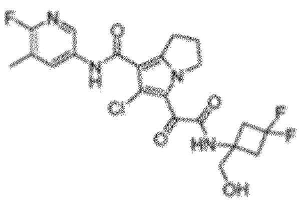,
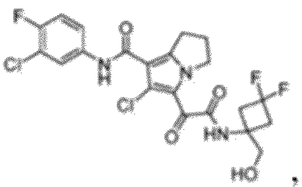,

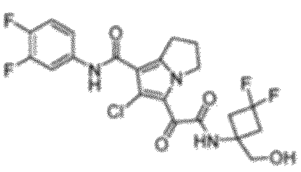,
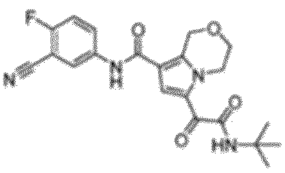,
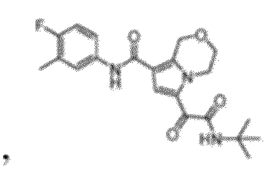,

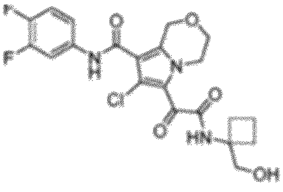,
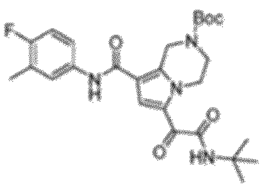,
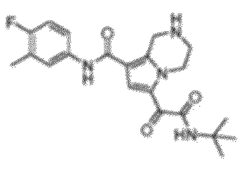,

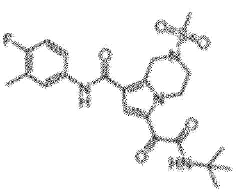,
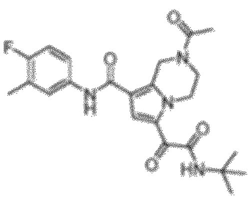,
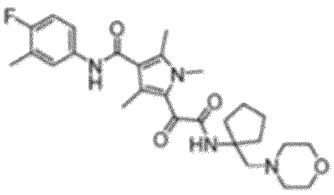,

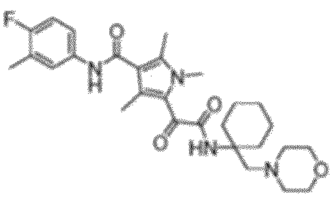,
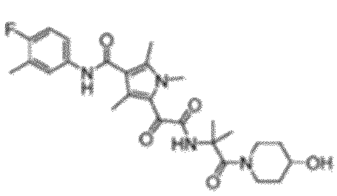,

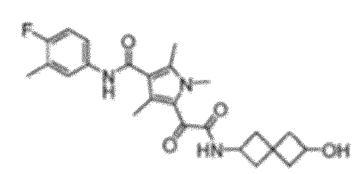,
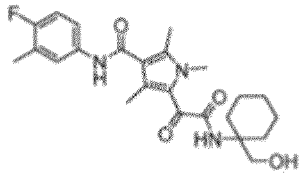,

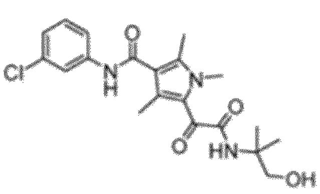,
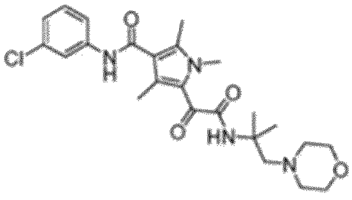,

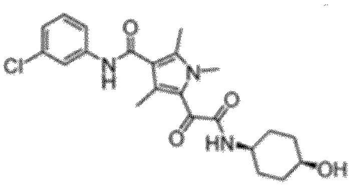,
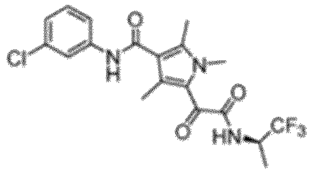,

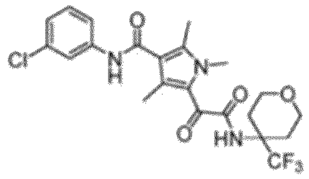,
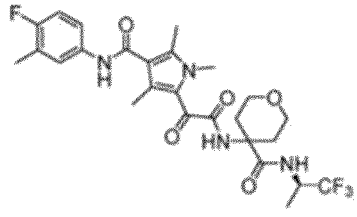,

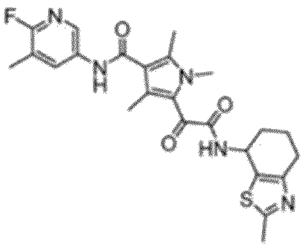,
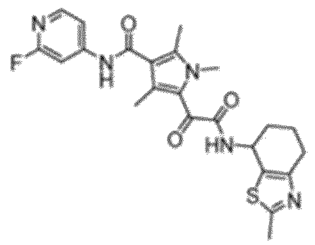,
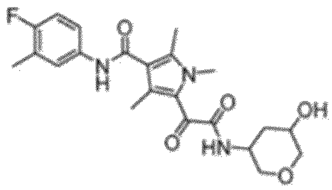,

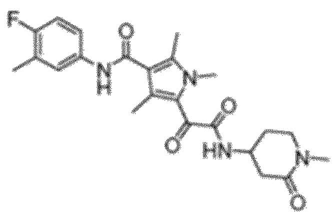,
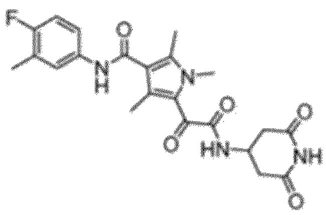,

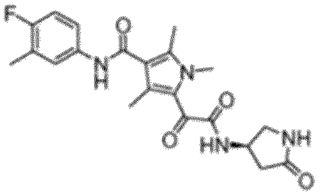,
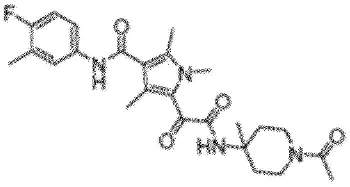,

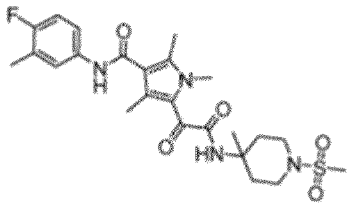 , 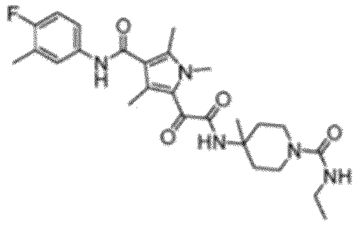 ,

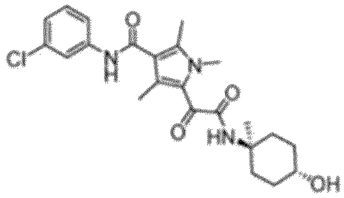 , 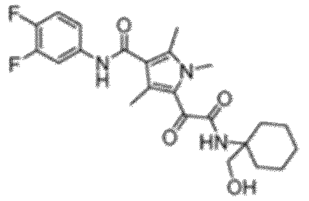 ,

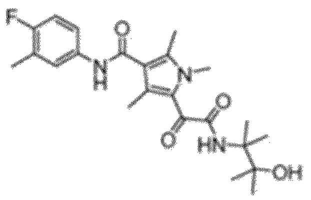 , 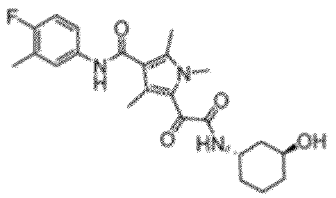 ,

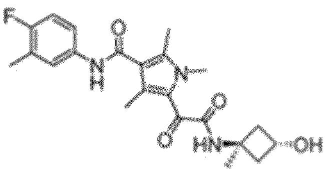 , 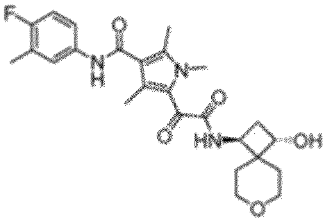 ,

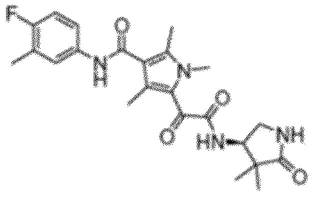 , 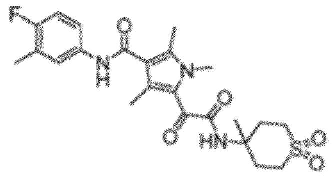 ,

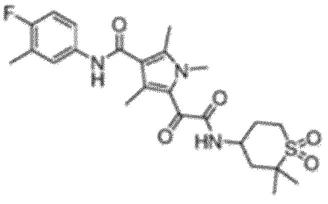 , 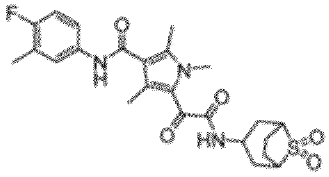 ,

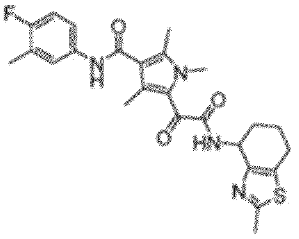 , 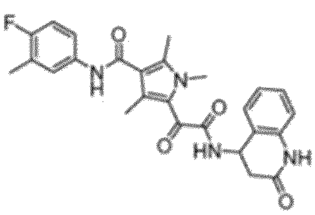 , 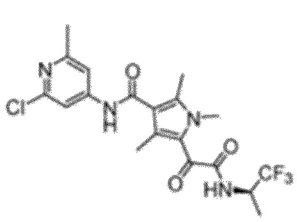 ,

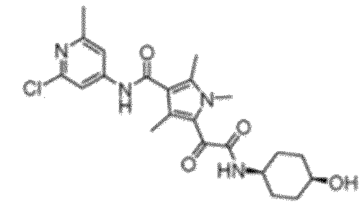,

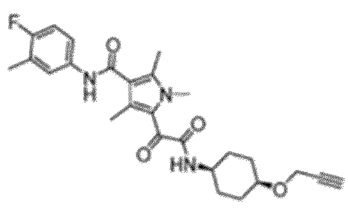,

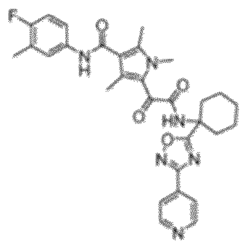,

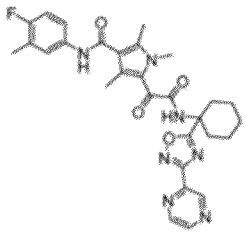,

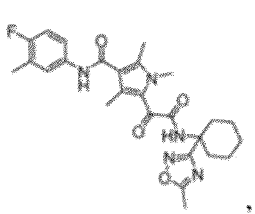,

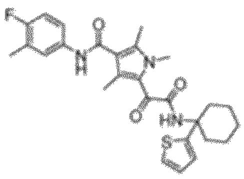,

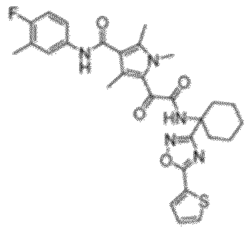,

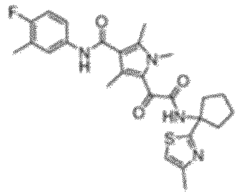,

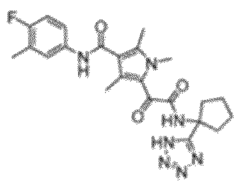,

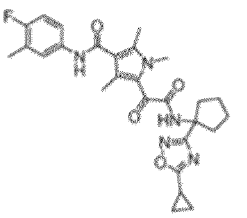,

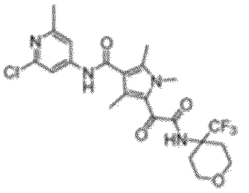,

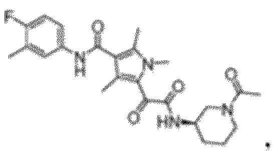,

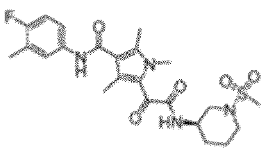,

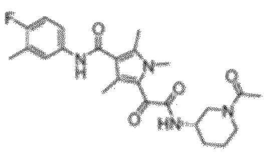,

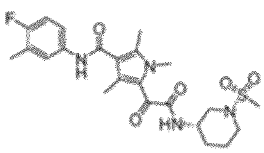,

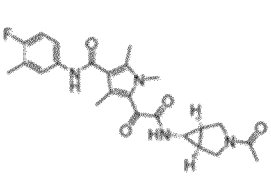,

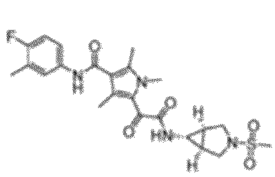,

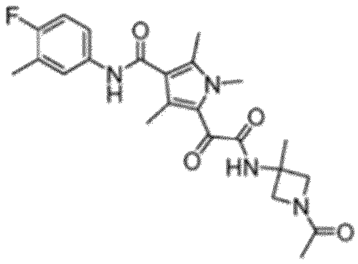,
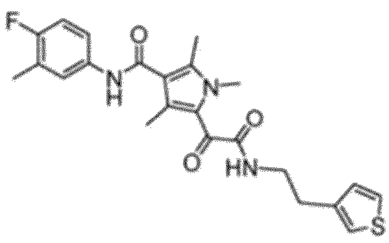,
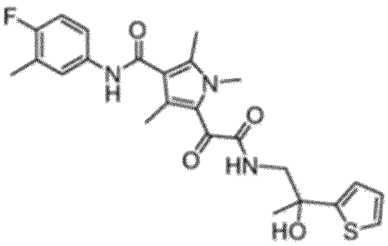,
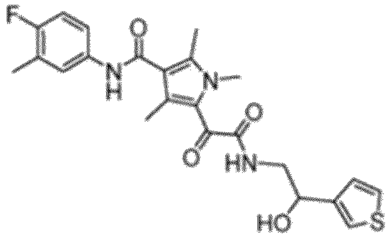,
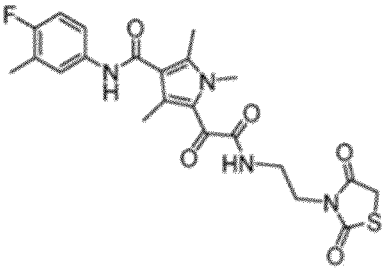,
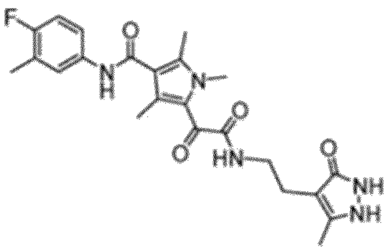,
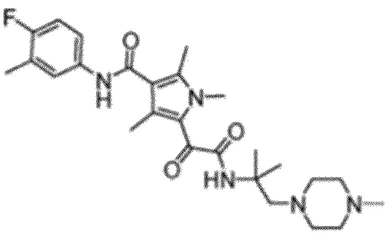,
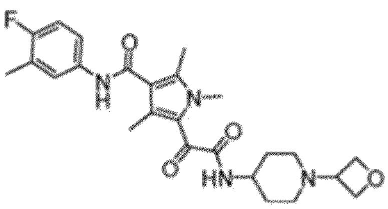,
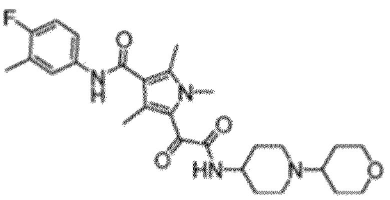,
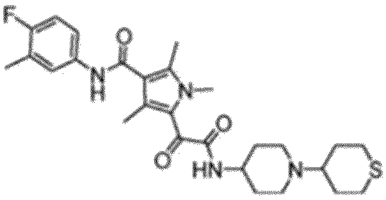,
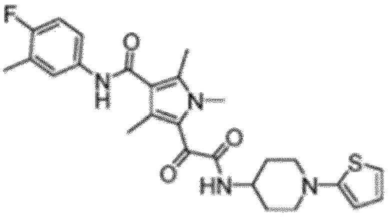,
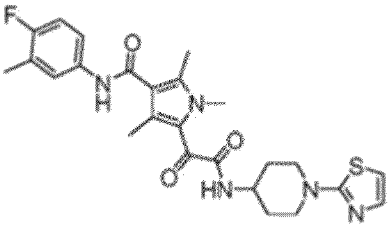,

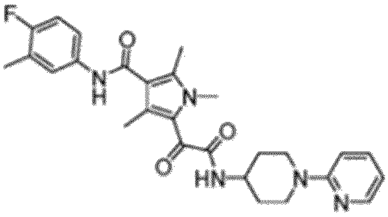, 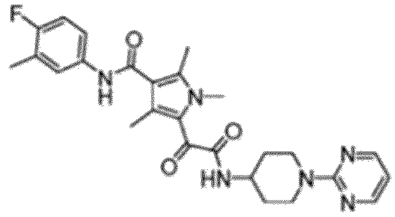,

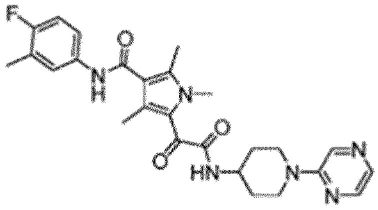, 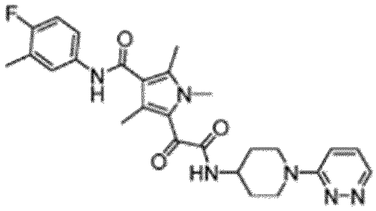,

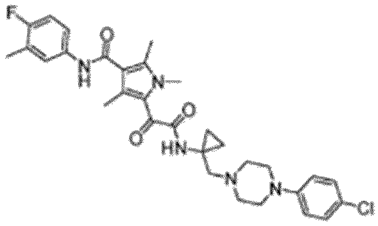, 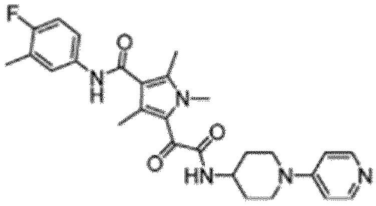,

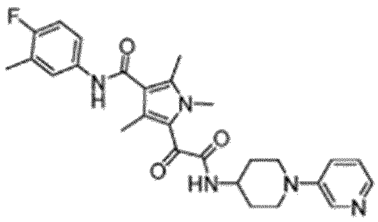, 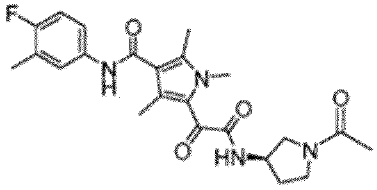,

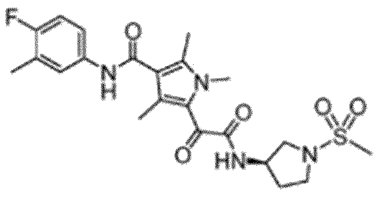, 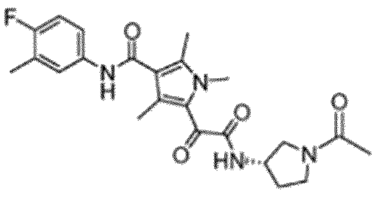,

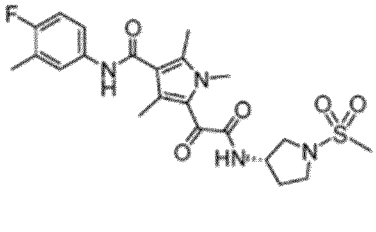, 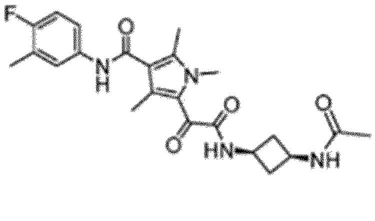,

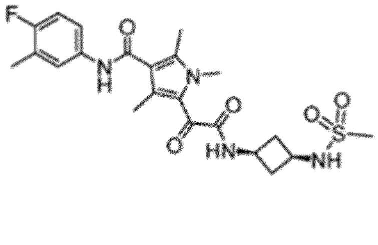, 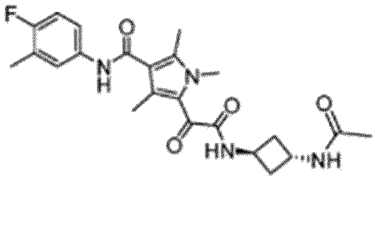,

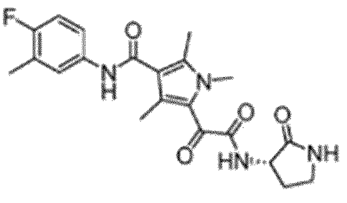 , 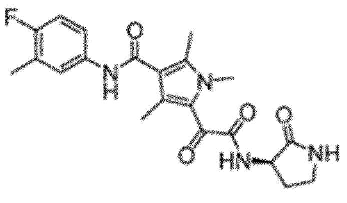 , 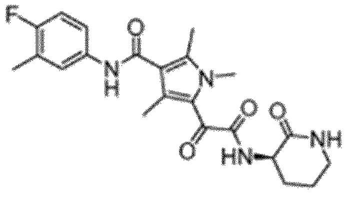 ,

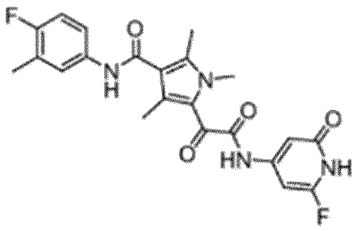 , 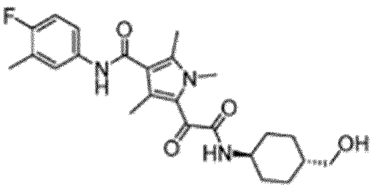 ,

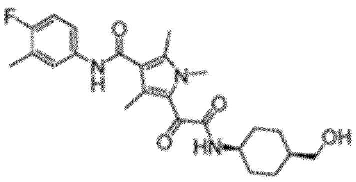 , 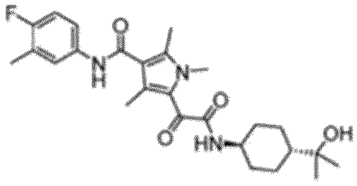 ,

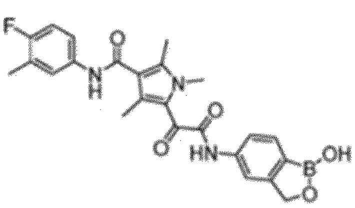 , 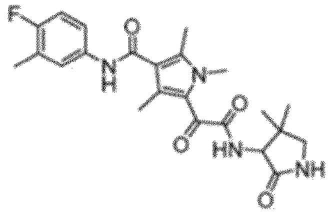 ,

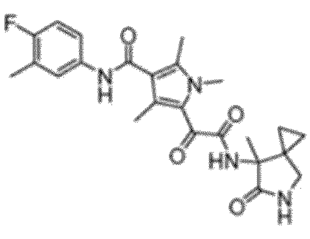 , 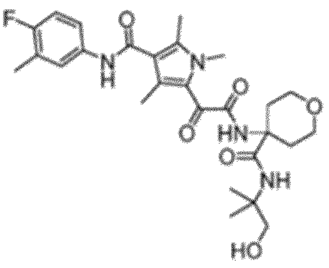 , 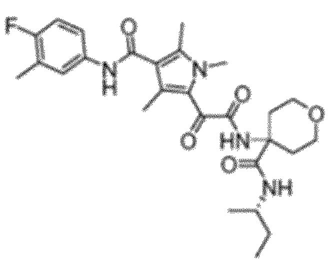 ,

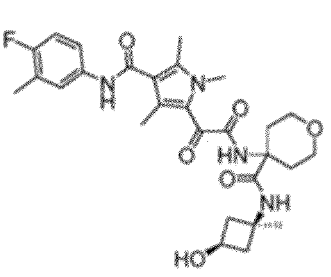 , 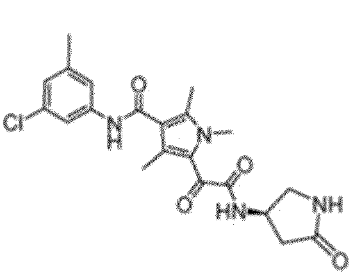 ,

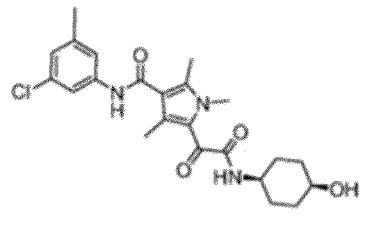,

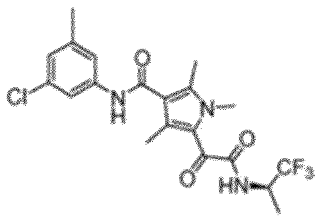,

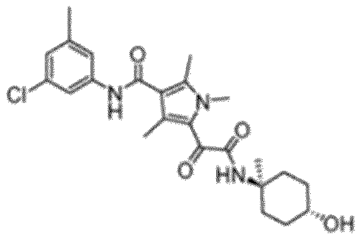,

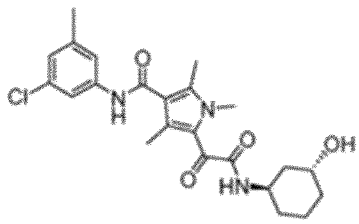,

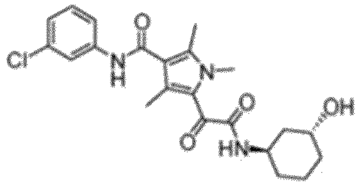,

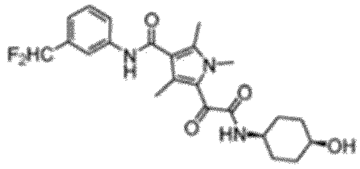,

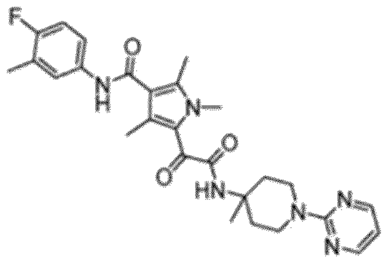,

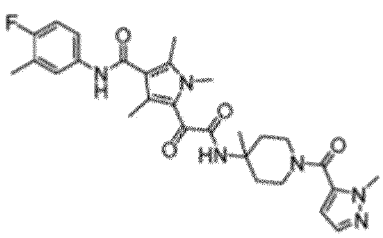,

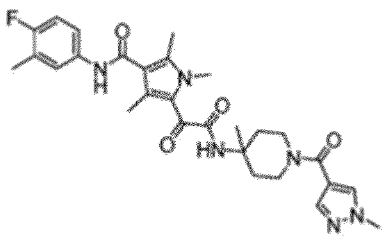,

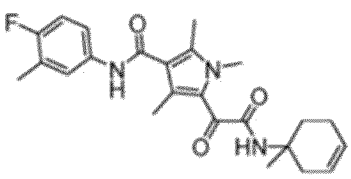,

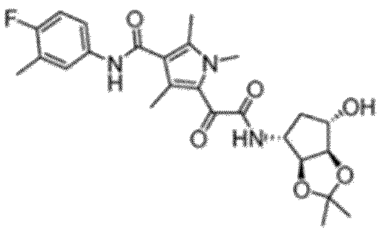,

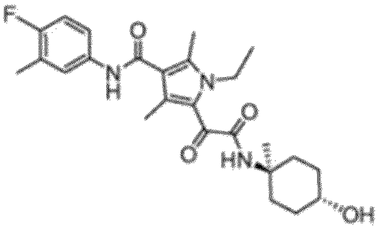,

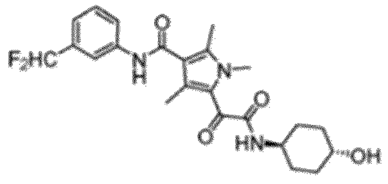,
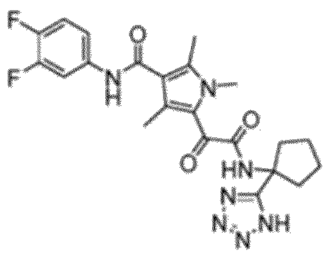,
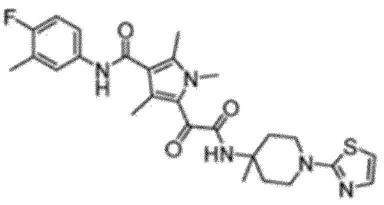,
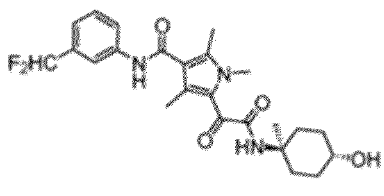,
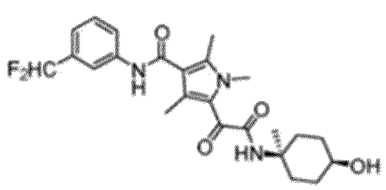,
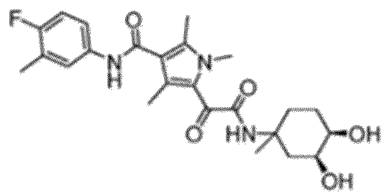,
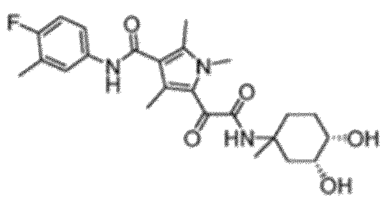,
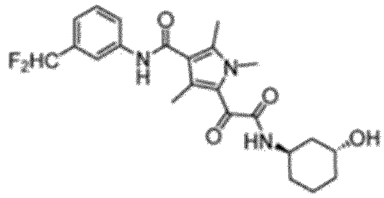,
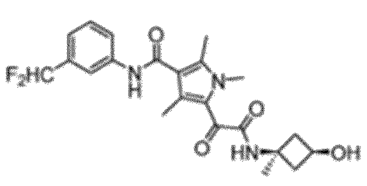,
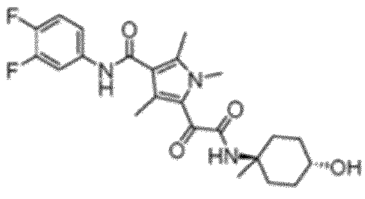,
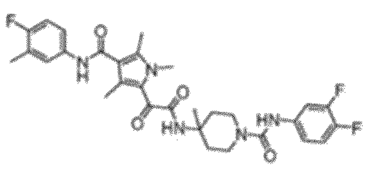,
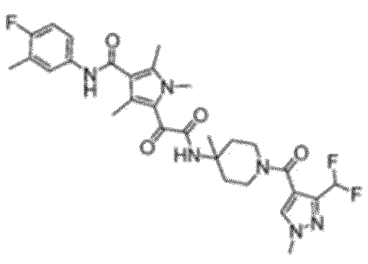,

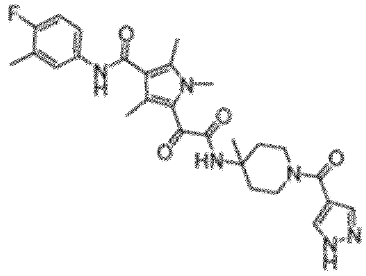,
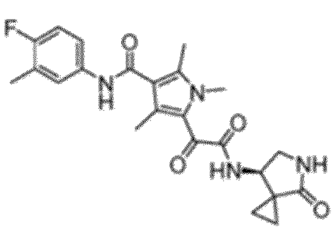,

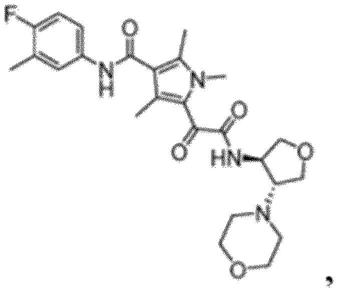,
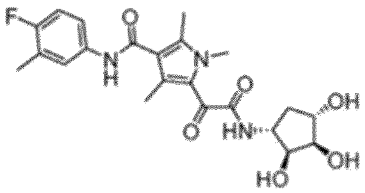,

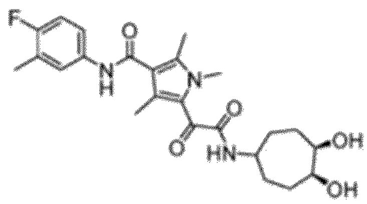,
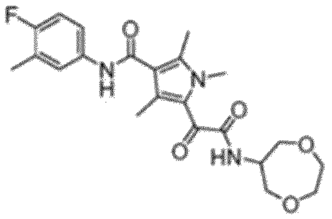,

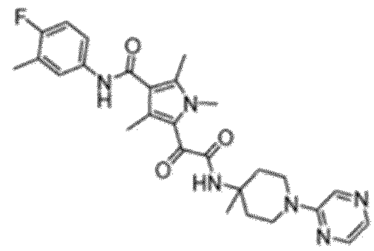,
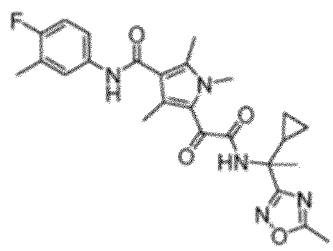,

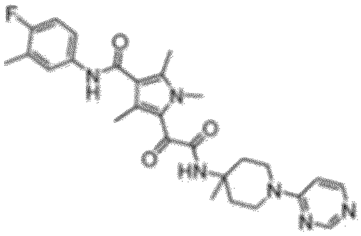,
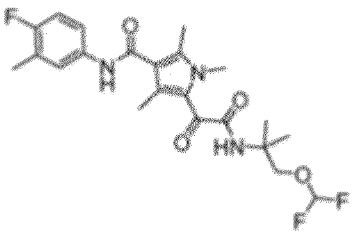,

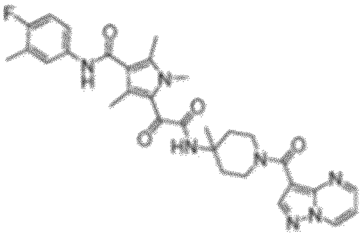,
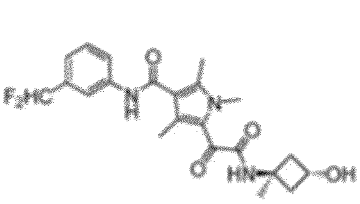,

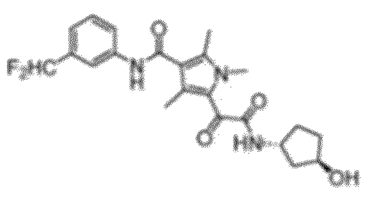,
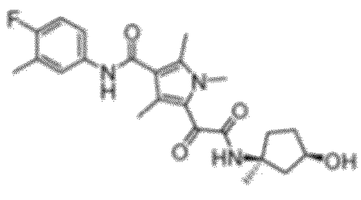,
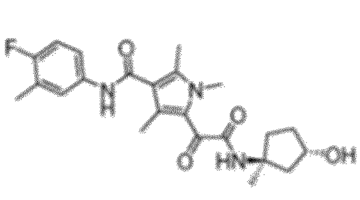,
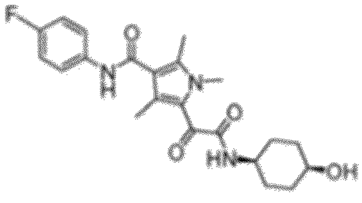,
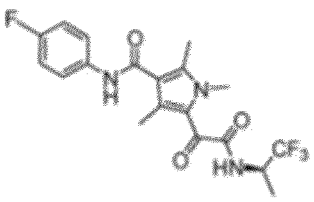,
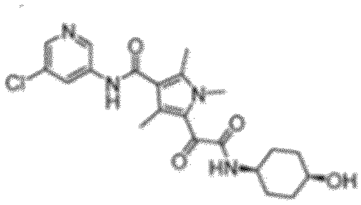,
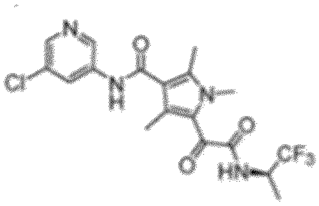,
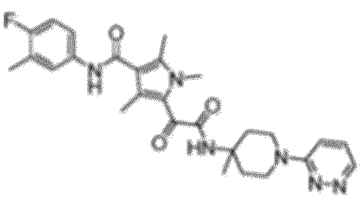,
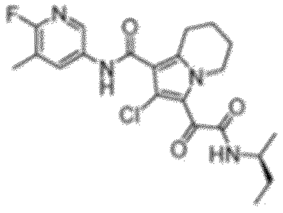,
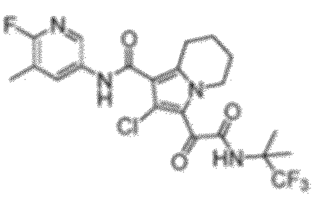,
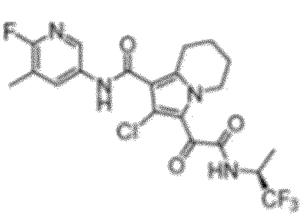,
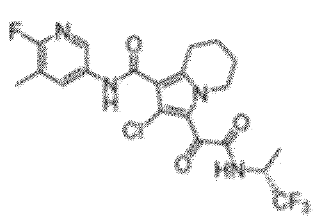,
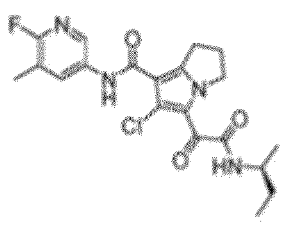,
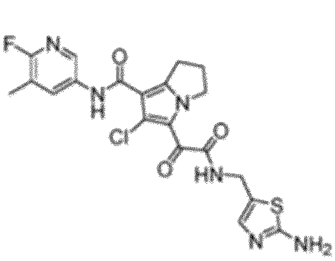,
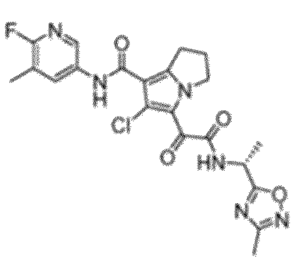,
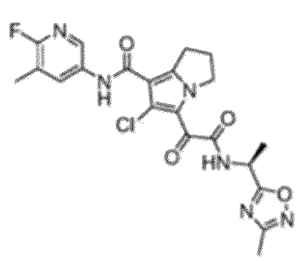,

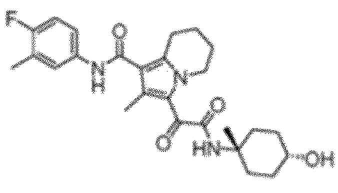, 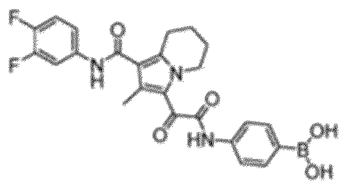,

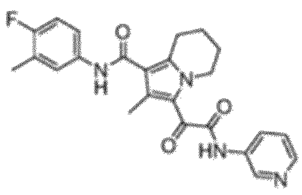, 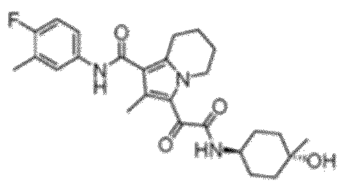,

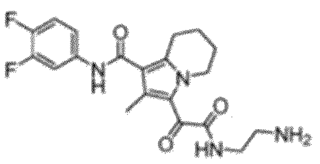, 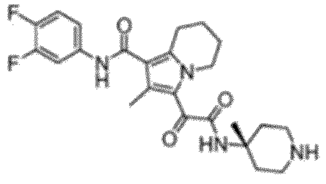, 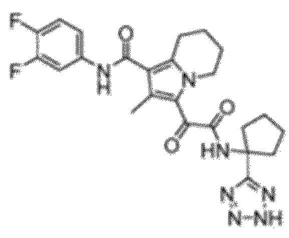,

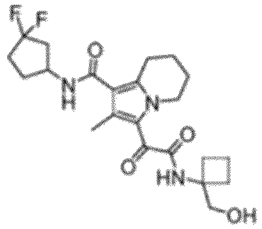, 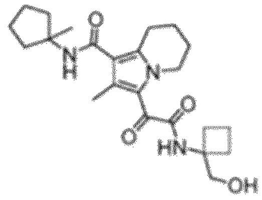, 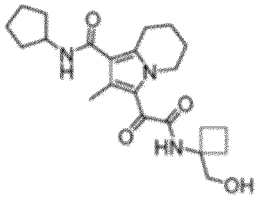,

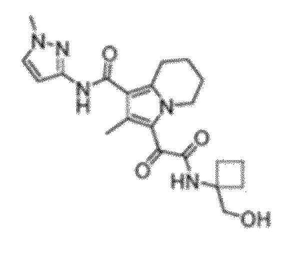, 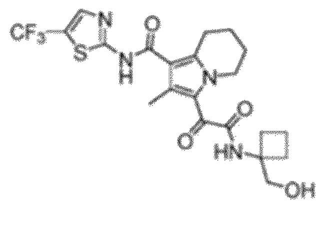, 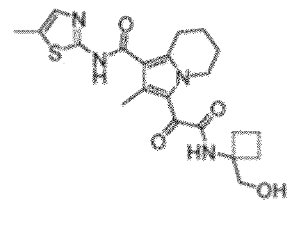,

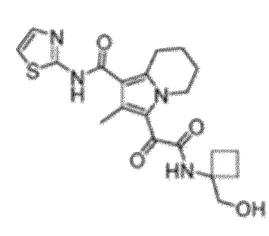, 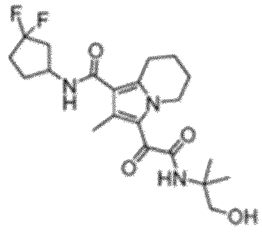, 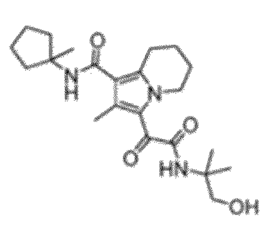,

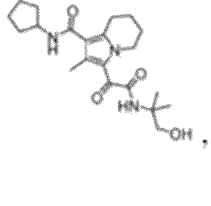, 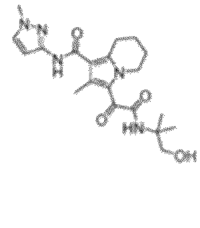, 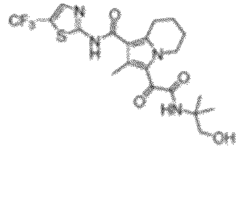,

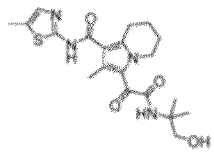 and 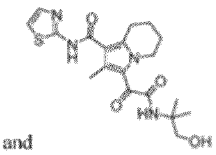 .

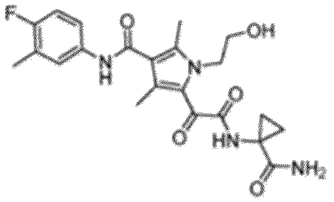 , 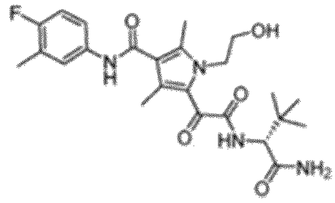 ,

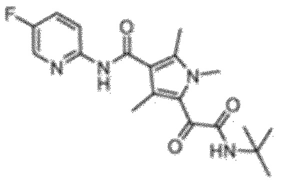 , 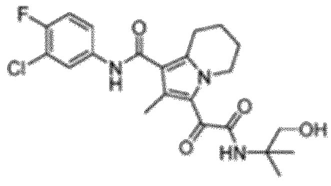 ,

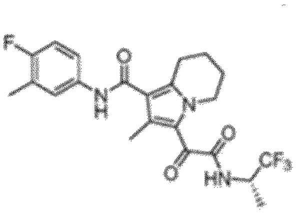 , 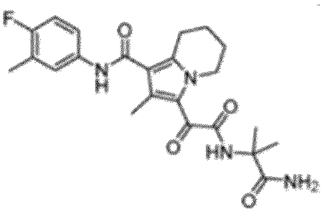 ,

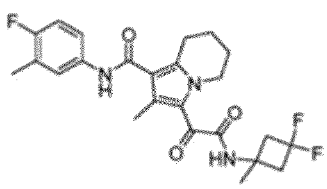 , 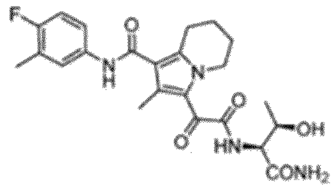 ,

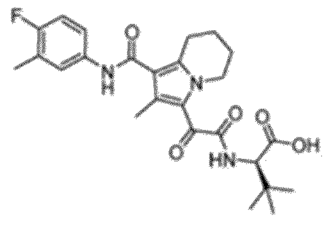 , 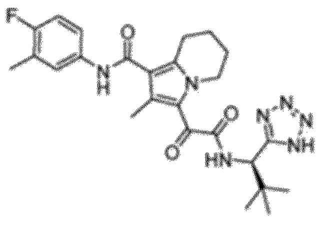 ,

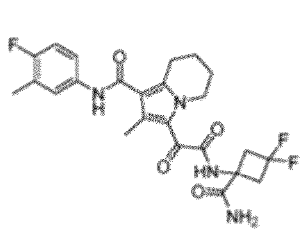 , 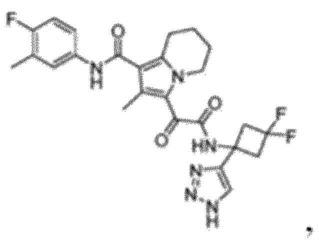 ,

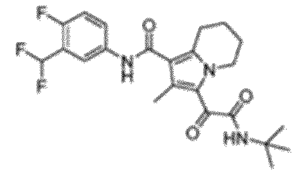,
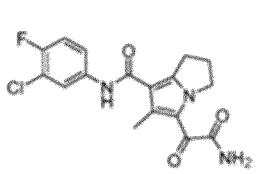,

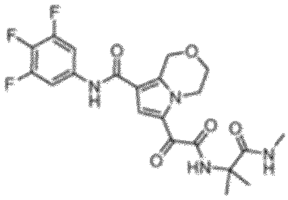,
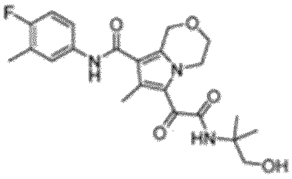,

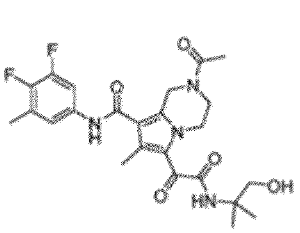,
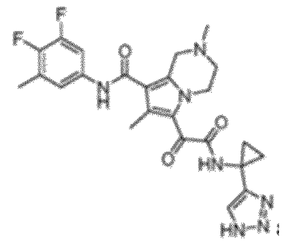;

and

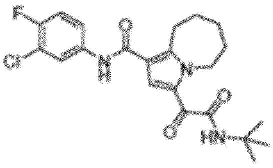,
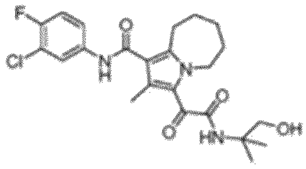.

Exemplary specific compounds (1-188) or pharmaceutically acceptable salts thereof in WO2021119081:

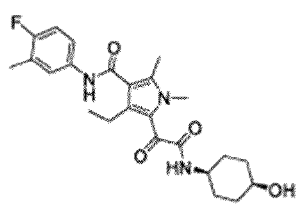,
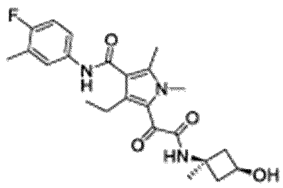,
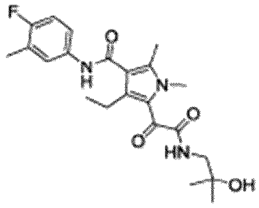,

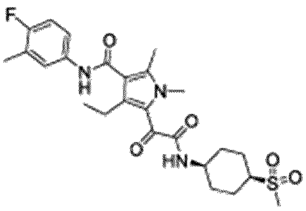,
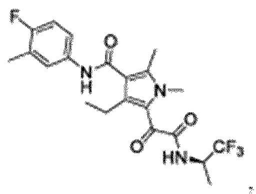,
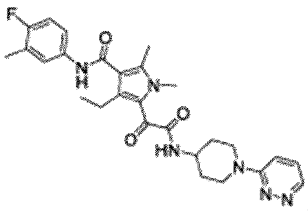,

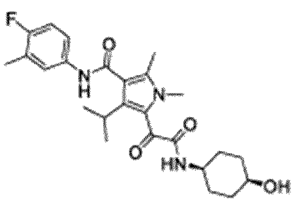,
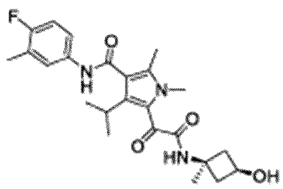,
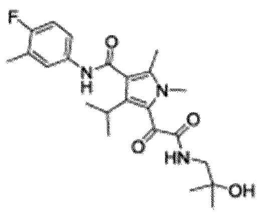,

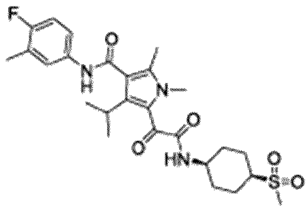,
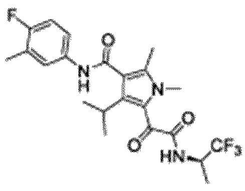,
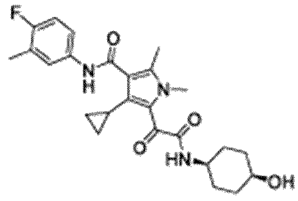,

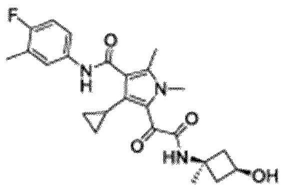,
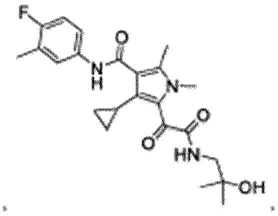,
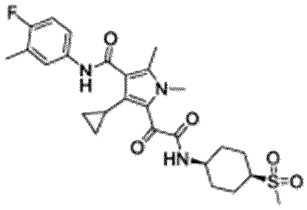,

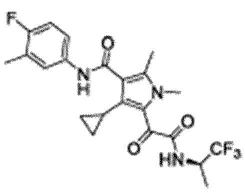,
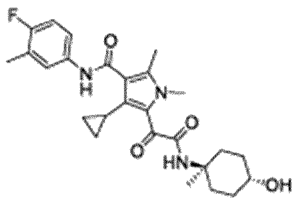,
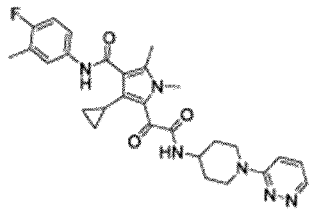,

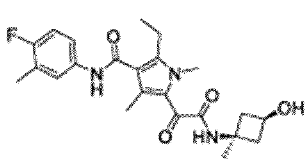,
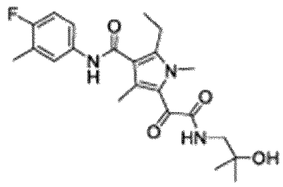,
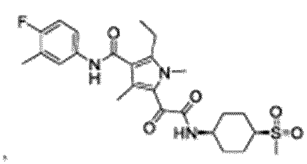,

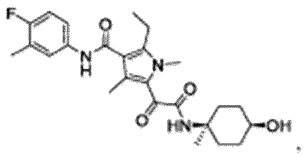,
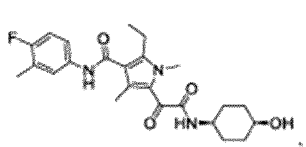,
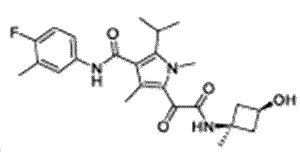,

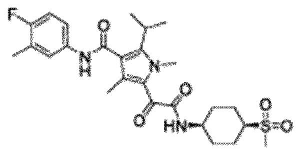,
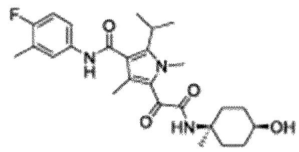,
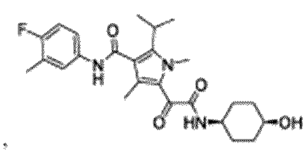,

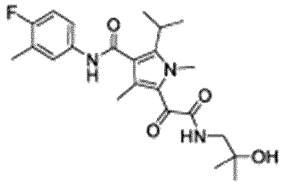,
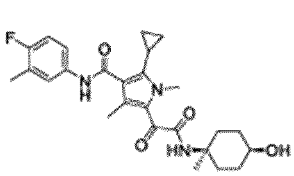,
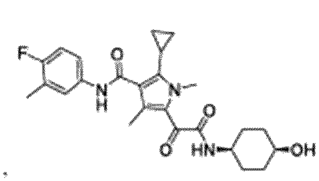,

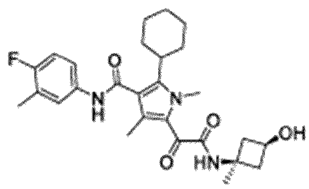,

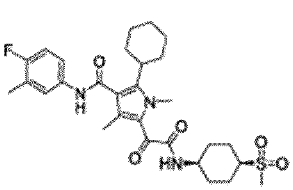,

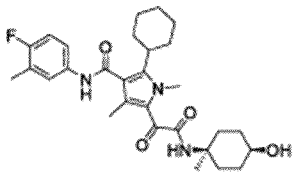,

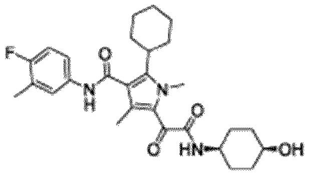,

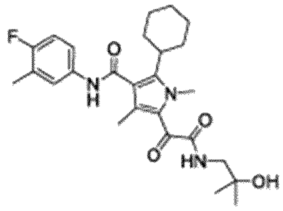,

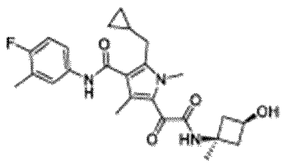,

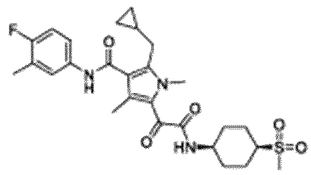,

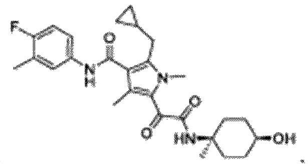,

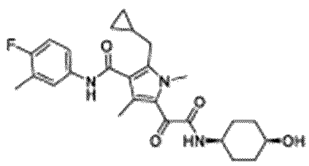,

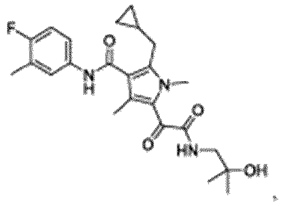,

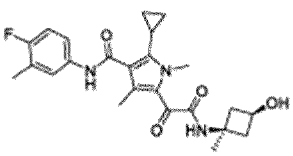,

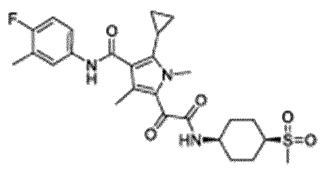,

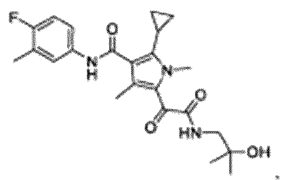,

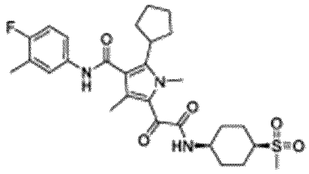,

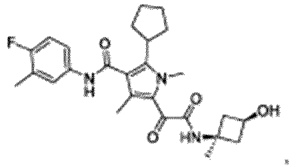,

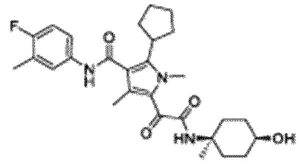,

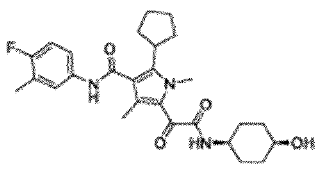,

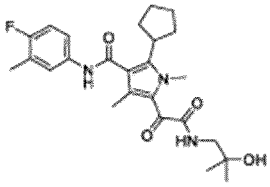,

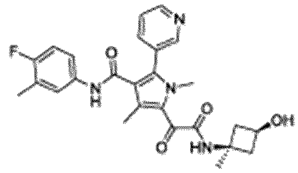,

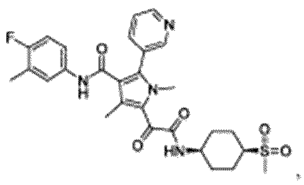, 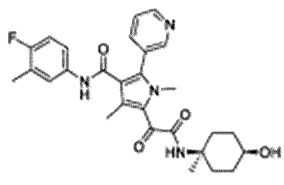, 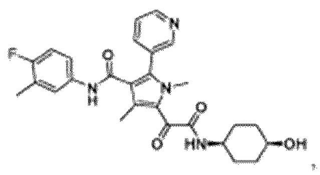,

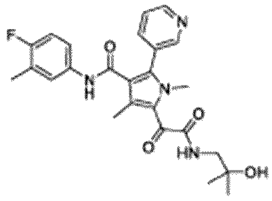, 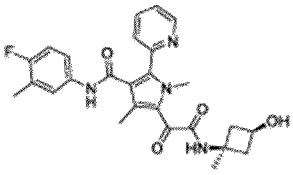, 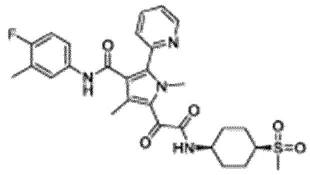,

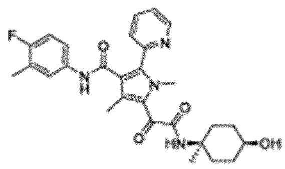, 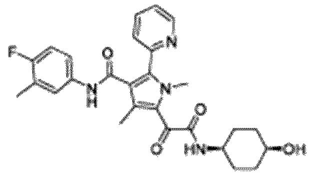, 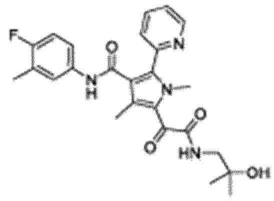,

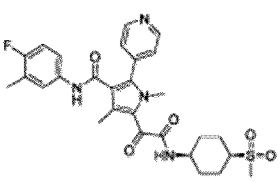, 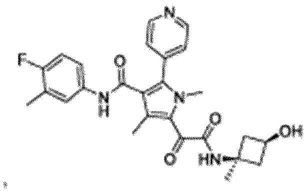, 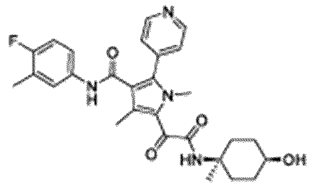,

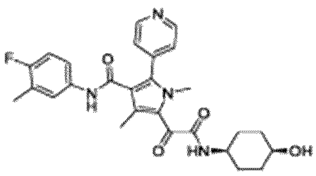, 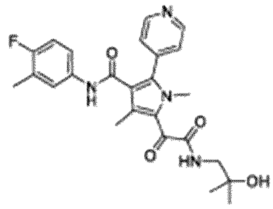, 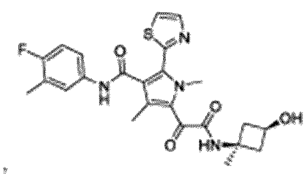,

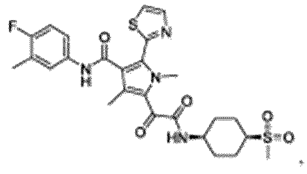, 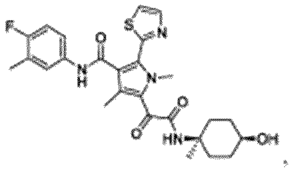, 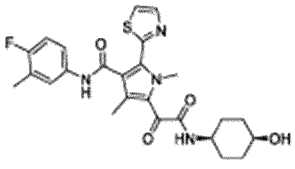,

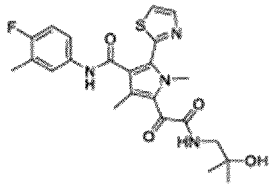, 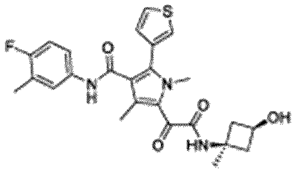, 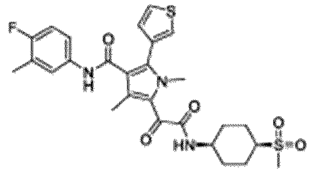,

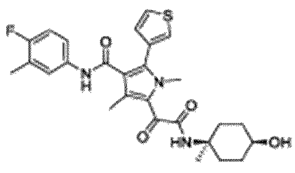,
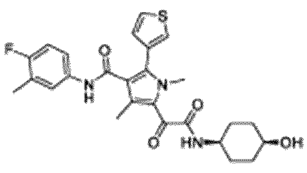;

and

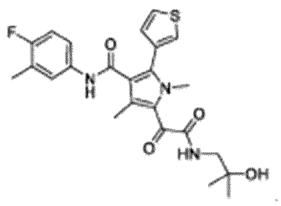

Exemplary specific compounds (1-18) or pharmaceutically acceptable salts thereof in WO2020156494:

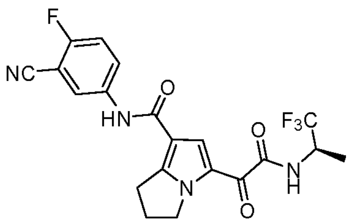
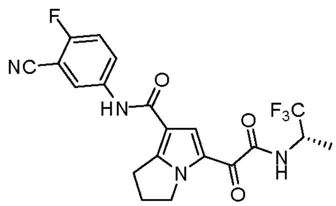
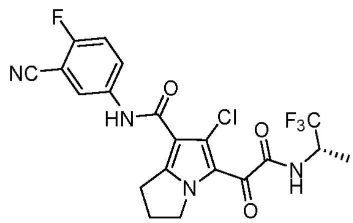
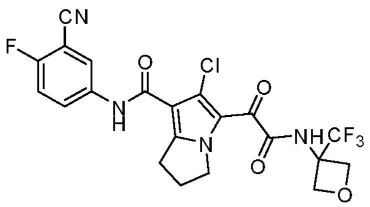
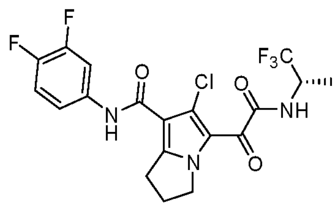
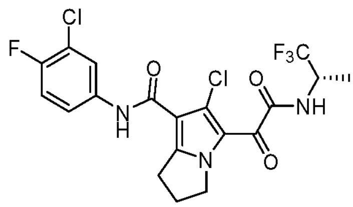
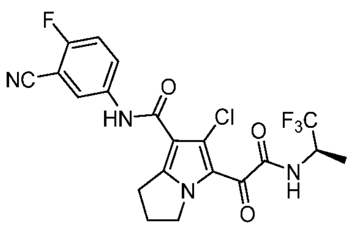
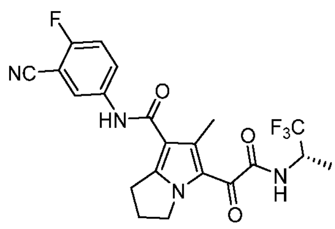
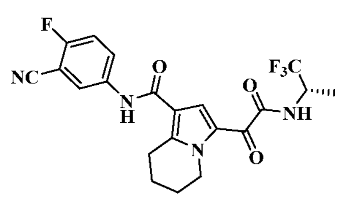
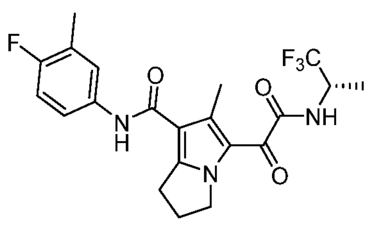
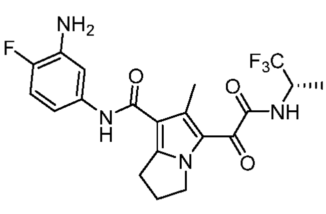
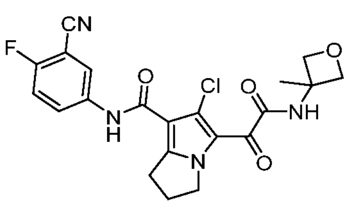

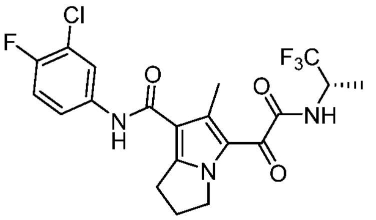,
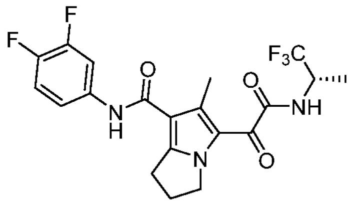,
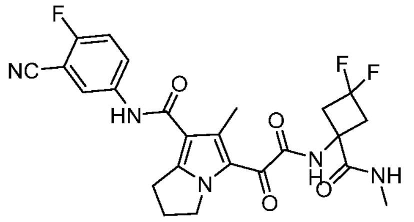,
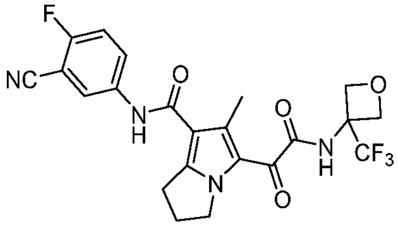,
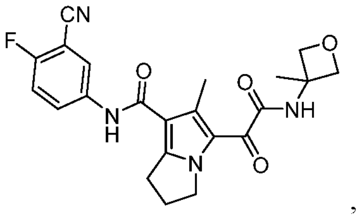, and
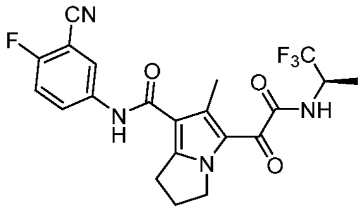,

or pharmaceutically acceptable salts thereof.

Compound of formula I or pharmaceutically acceptable salt thereof

The compound of formula I belongs to the prior art and has a chemical name of (S)-N-(3-cyano-4-fluorophenyl)-1,2,4-trimethyl-5-(2-oxo-2-((1,1,1-trifluoropropan-2-yl)amino)acetyl)-1Hpyrrole-3-carboxatnide, which has the following structural formula:

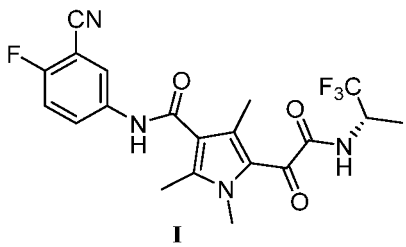

**I**

.

For the preparation method and chemical properties of the compound of formula I or the pharmaceutically acceptable salt thereof, reference can be made to WO2019185016A1.

In some embodiments, the pharmaceutical composition of the compound of formula I or the pharmaceutically acceptable salt thereof is selected from a solid pharmaceutical composition, preferably selected from the group consisting of a tablet and a capsule.

In some embodiments, the compound of formula I or the pharmaceutically acceptable salt thereof is in the form of a pharmaceutical composition in a single dose of 1 mg-1000 mg, preferably selected from a pharmaceutical composition in a single dose of 1 mg-400 mg, more preferably selected from a pharmaceutical composition in a single dose of 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg, 110 mg, 120 mg, 130 mg, 140 mg, 150 mg, 160 mg, 170 mg, 180 mg, 190 mg, 200 mg, 210 mg, 220 mg, 230 mg, 240 mg, 250 mg, 260 mg, 270 mg, 280 mg, 290 mg, 300 mg, 310 mg, 320 mg, 330 mg, 340 mg, 350 mg, 360 mg, 370 mg, 380 mg, 390 mg, 400 mg, or a range formed by any of the above values.

Entecavir or pharmaceutically acceptable salt thereof or solvate thereof, or pharmaceutical composition thereof

As used herein, entecavir has a chemical name of 2-amino-9-[(1*S*,3*R*,4*S*)-4-hydroxy-3-hydroxymethyl-2-methylenecyclopentyl]-1,9-dihydro-6H-purin-6-one, which has the following structural formula:

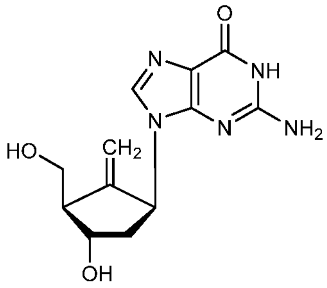

.

In some embodiments, entecavir used may be a pharmaceutically acceptable salt thereof selected from a maleate. In some embodiments, the pharmaceutically acceptable salt of entecavir is selected from a monomaleate.

In some embodiments, entecavir used may be a solvate thereof selected from an entecavir hydrate. In some embodiments, the entecavir hydrate is selected from the group consisting of entecavir hemihydrate to dihydrate. In some embodiments, the entecavir hydrate is selected from entecavir monohydrate.

In some embodiments, entecavir is selected from the group consisting of entecavir monomaleate, entecavir monohydrate, and entecavir monomaleate monohydrate.

In some embodiments, entecavir or the pharmaceutically acceptable salt thereof or the solvate thereof is in the form of a pharmaceutical composition. Preferably, the pharmaceutical composition is selected from a solid pharmaceutical composition. The solid pharmaceutical composition is preferably selected from the group consisting of a tablet and a capsule.

In some embodiments, the pharmaceutical composition of entecavir or the pharmaceutically acceptable salt thereof or the solvate thereof is selected from a pharmaceutical composition in a single dose of 0.01 mg-5 mg, preferably selected from a pharmaceutical composition in a single dose of 0.01 mg, 0.02 mg, 0.05 mg, 0.08 mg, 0.1 mg, 0.2 mg, 0.3 mg, 0.4 mg, 0.5 mg, 0.6 mg, 0.7 mg, 0.8 mg, 0.9 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, 4.0 mg, 4.1 mg, 4.2 mg, 4.3 mg, 4.4 mg, 4.5 mg, 4.6 mg, 4.7 mg, 4.8 mg, 4.9 mg, 5.0 mg, or a range formed by any of the above values.

As used herein, entecavir or the pharmaceutically acceptable salt thereof or the solvate thereof, or the pharmaceutical composition thereof may be selected from a commercially available product (e.g., entecavir dispersible tablets: Runzhong®).

<u>Tenofovir or pharmaceutically acceptable prodrug thereof or pharmaceutically acceptable salt thereof, or pharmaceutical composition thereof</u>

Tenofovir or the pharmaceutically acceptable prodrug thereof or the pharmaceutically acceptable salt thereof, or the pharmaceutical composition thereof described herein includes a free compound of tenofovir or prodrugs thereof or pharmaceutically acceptable salts thereof, or pharmaceutical compositions thereof.

As used herein, tenofovir has a chemical name of R-9-(2-phosphonomethoxypropyl)adenine, which has the following structural formula:

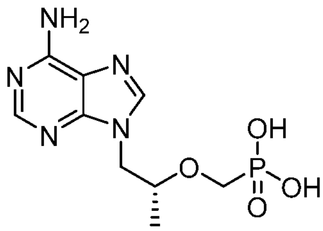

.

In some embodiments, tenofovir used may be a prodrug thereof. In some embodiments, the pharmaceutically acceptable prodrug of tenofovir is selected from the group consisting of tenofovir alafenamide, tenofovir disoproxil, and tenofovir amibufenamide.

In some embodiments, tenofovir used may be a pharmaceutically acceptable salt of a free compound or prodrug thereof. In some embodiments, the pharmaceutically acceptable salt of the free compound or prodrug of tenofovir includes a fumarate, an orotate, a disulfonate, a phosphate, a succinate, or an aspartate.

In some embodiments, tenofovir used may be a pharmaceutically acceptable salt of a prodrug thereof. In some

embodiments, the pharmaceutically acceptable salt of the prodrug of tenofovir is selected from the group consisting of tenofovir disoproxil fumarate, tenofovir alafenamide fumarate, and tenofovir amibufenamide fumarate.

In some embodiments, the pharmaceutically acceptable salt of the prodrug of tenofovir is selected from tenofovir alafenamide fumarate. In some embodiments, the ratio of the number of molecules of tenofovir alafenamide to fumaric acid in the pharmaceutically acceptable salt of the prodrug of tenofovir is 2:1. In some embodiments, the tenofovir alafenamide fumarate is selected from the following structure:

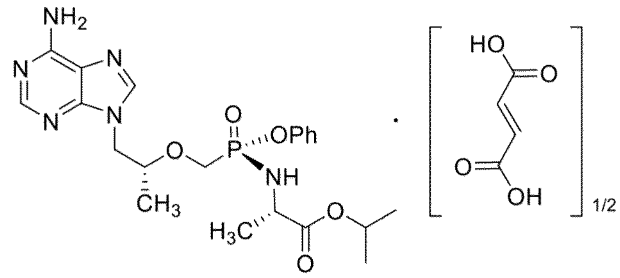

In some embodiments, the pharmaceutically acceptable salt of the prodrug of tenofovir is selected from tenofovir disoproxil fumarate. In some embodiments, the ratio of the number of molecules of tenofovir disoproxil to fumaric acid in the pharmaceutically acceptable salt of the prodrug of tenofovir is 1:1. In some embodiments, the tenofovir disoproxil fumarate is selected from the following structure:

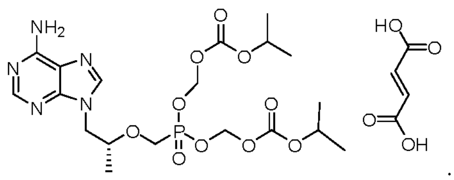

In some embodiments, the pharmaceutically acceptable salt of the prodrug of tenofovir is selected from tenofovir amibufenamide fumarate. In some embodiments, the ratio of the number of molecules of tenofovir amibufenamide to fumaric acid in the pharmaceutically acceptable salt of the prodrug of tenofovir is 1:1. In some embodiments, the tenofovir amibufenamide fumarate is selected from the following structure:

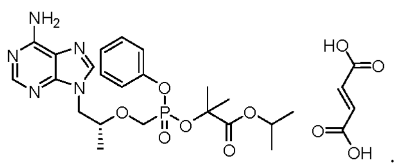

In some embodiments, the pharmaceutically acceptable salt of the prodrug of tenofovir is selected from tenofovir disoproxil orotate. In some embodiments, the ratio of the number of molecules of tenofovir disoproxil to orotic acid in the pharmaceutically acceptable salt of the prodrug of tenofovir is 1:1. In some embodiments, the tenofovir disoproxil orotate is selected from the following structure:

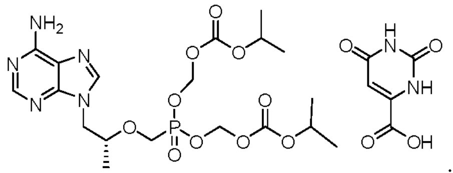

In some embodiments, the pharmaceutically acceptable salt of the prodrug of tenofovir is selected from tenofovir disoproxil succinate. In some embodiments, the ratio of the number of molecules of tenofovir disoproxil to succinic acid in the pharmaceutically acceptable salt of the prodrug of tenofovir is 1:1. In some embodiments, the tenofovir disoproxil succinate is selected from the following structure:

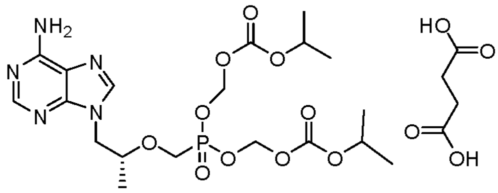
.

In some embodiments, tenofovir or the pharmaceutically acceptable prodrug thereof or the pharmaceutically acceptable salt thereof is in the form of a pharmaceutical composition. Preferably, the pharmaceutical composition is selected from a solid pharmaceutical composition. The solid pharmaceutical composition is preferably selected from the group consisting of a tablet and a capsule.

In some embodiments, tenofovir or the pharmaceutically acceptable prodrug thereof or the pharmaceutically acceptable salt thereof is selected from a pharmaceutical composition in a single dose of 0.05 mg-500 mg, preferably selected from a pharmaceutical composition in a single dose of 0.1 mg, 0.5 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 35 mg, 40 mg, 45 mg, 50 mg, 55 mg, 60 mg, 65 mg, 70 mg, 75 mg, 80 mg, 85 mg, 90 mg, 95 mg, 100 mg, 105 mg, 110 mg, 115 mg, 120 mg, 125 mg, 130 mg, 135 mg, 140 mg, 145 mg, 150 mg, 155 mg, 160 mg, 165 mg, 170 mg, 175 mg, 180 mg, 185 mg, 190 mg, 200 mg, 250 mg, 300 mg, 350 mg, 400 mg, 450 mg, 500 mg, or a range formed by any of the above values (calculated based on a free compound of tenofovir).

In some embodiments, the pharmaceutical composition of tenofovir disoproxil fumarate is selected from a pharmaceutical composition in a single dose of 300 mg.

In some embodiments, the pharmaceutical composition of tenofovir alafenamide is selected from a pharmaceutical composition in a single dose of 25 mg.

In some embodiments, the pharmaceutical composition of tenofovir alafenamide fumarate is selected from a pharmaceutical composition in a single dose of 28 mg.

As used herein, tenofovir or the pharmaceutically acceptable prodrug thereof or the pharmaceutically acceptable salt thereof may be selected from a commercially available product (e.g., tenofovir disoproxil fumarate tablets: Qingzhong®, or tenofovir alafenamide fumarate tablets: Vemlidy®).

In some embodiments, the individual is selected from the group consisting of an individual who has previously received treatment and an individual who has not previously received treatment. In some embodiments, the individual is selected from an individual who has previously received treatment with entecavir or the pharmaceutically acceptable salt thereof or the solvate thereof, or tenofovir or the pharmaceutically acceptable prodrug thereof or the pharmaceutically acceptable salt thereof. In some embodiments, the individual is selected from an individual who has not previously received treatment. In some embodiments, the individual is selected from an individual who has previously received treatment.

In some embodiments, the individual is selected from an individual who meets the following serological virology criteria: having positive serum HBsAg for 6 consecutive months or more or evidence of chronic hepatitis B for 6 months, with quantitative HbsAg≥ 1000 IU/mL and ≤ 40000 IU/mL.

In some embodiments, the individual is selected from an individual who has previously received treatment and should meet the following criteria:

1) the subject must have received the same NA treatment (ETV/TDF) at the same dose for 12 months or more prior to screening;

2) medical history records of HBV DNA inhibition less than the lower limit of normal 6 months prior to enrollment, with HBV DNA inhibition during screening defined as < LLOQ; and 3)

$$ALT \leq 5 \times ULN.$$

In some embodiments, the individual is selected from an individual who has not previously received treatment and should meet the following criteria:

1) the subject must not have received any CHB treatment at the time of screening, i.e.,

1.1 the subject has never received treatment with an HBV antiviral drug (NA) or an interferon (IFN) drug, or

1.2 the subject has received treatment with an HBV antiviral drug (NA) or an IFN drug (used irregularly), with no more than 3 months from the first use of the study drug, or

1.3 the subject has not received treatment with an HBV antiviral drug (NA) or an IFN drug within 6 months prior to screening;

2) for HBeAg-positive chronic hepatitis B patients, HBV DNA > $10^5$ copies/mL (or > 20,000 IU/mL); or for HBeAg-negative patients, HBV DNA > $10^4$ copies/mL (or > 2,000 IU/mL); and

3)

$$1.0 \times \text{ULN} \leq \text{ALT} \leq 5 \times \text{ULN}.$$

Definitions and explanations

Unless otherwise stated, the following terms used in the present application shall have the following meanings. A certain term, unless otherwise specifically defined, should not be considered ambiguous or unclear, but construed according to its common meaning in the art.

As used herein, the compound of formula I includes non-salt forms thereof (e.g., free acids or free bases) and further includes pharmaceutically acceptable salts thereof. The non-salts or salts fall within the scope of protection of the present application.

As used herein, entecavir includes non-solvate forms thereof and further includes solvate forms thereof. The non-solvates or solvates fall within the scope of protection of the present application. When entecavir is selected from solvate forms, the molar ratio of the compound to the solvent may be selected from the group consisting of 1:0.5, 1:1, 1:1.5 and 1:2 or from a range formed by any of the ratios, e.g., 1:0.5 to 1:2, 1:0.5 to 1:1.5, or 1:1 to 1:1.5. For example, entecavir is in the form of a non-solvate. For example, entecavir is in the form of a hydrate. For example, entecavir is in the form of a monohydrate.

As used herein, tenofovir includes prodrug forms thereof and further includes salt forms of prodrugs thereof. Tenofovir, the prodrugs thereof or the salts thereof fall within the scope of protection of the present application.

As used herein, unless otherwise specified, "calculated based on weight" refers to calculated based on the weight of the compound of formula I, entecavir, or tenofovir in its free form.

The terms "administer", "administration" and "administering" refer to physically introducing the composition comprising a therapeutic agent to an entity using any of a variety of methods and delivery systems known to those skilled in the art.

The term "treat", "treating" or "treatment" usually refers to acquiring needed pharmacological effects and/or physiological effects. In terms of partially or fully stabilizing or curing a disease and/or an adverse effect of the disease, the effect can be therapeutic. As used herein, "treat", "treating", or "treatment" encompasses any treatment of a disease in a patient, including: (a) inhibiting a symptom of the disease, i.e., blocking the progression of the disease; or (b) alleviating a symptom of the disease, i.e., causing the regression of the disease or the symptom.

The term "effective amount" refers to an amount of the compound of the present application for (i) treating or preventing a specific disease, condition or disorder; (ii) alleviating, improving or eliminating one or more symptoms of a specific disease, condition or disorder, or (iii) preventing or delaying onset of one or more symptoms of a specific disease, condition or disorder. The amount of the compound of the present application composing the "therapeutically effective amount" varies depending on the compound, the disease state and its severity, the administration regimen, and the age of the mammal to be treated, but can be determined routinely by those skilled in the art in accordance with their knowledge and the present disclosure.

The term "individual" or "subject" includes mammals. In some embodiments, the "individual" or subject is a mouse. In some embodiments, the "individual" or subject is a human.

As used herein, the compound of formula I or the pharmaceutically acceptable salt thereof may be administered by any applicable route and method, e.g., oral administration or parenteral (e.g., intravenous) administration. The therapeutically effective amount of the compound of formula I or the pharmaceutically acceptable salt thereof includes, but is not limited to, from about 0.0001 to 20 mg/kg body weight/day, e.g., from 0.001 to 10 mg/kg body weight/day. The dosing frequency (e.g., once, twice, or more times daily) of the compound of formula I or the pharmaceutically acceptable salt thereof can be determined according to the needs of an individual patient, including the severity of the disease, the response to the disease, any treatment-related toxicity, and the age and health status of the patient. Administration can be intermittent, e.g., in a period of several days, the subject receives a daily dose of the compound of formula I or the pharmaceutically acceptable salt thereof, and in the following period of several days or more days, the patient does not receive the daily dose of the compound of formula I or the pharmaceutically acceptable salt thereof.

Entecavir or the pharmaceutically acceptable salt thereof or the solvate thereof can be administered through

various routes including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular, intraperitoneal and intrathecal administrations. In some specific embodiments, the drug is administered orally. The amount of entecavir administered can be determined according to the severity of the disease, the response to the disease, any treatment-related toxicity, and the age and health status of the patient. For example, entecavir may be administered at a daily dose of 0.005 mg to 10.0 mg. Entecavir may be administered once or more times daily. In some embodiments, entecavir is administered once daily in the form of an oral solid formulation.

Tenofovir or the pharmaceutically acceptable prodrug thereof or the pharmaceutically acceptable salt thereof can be administered through various routes including, but not limited to, oral, parenteral, intraperitoneal, intravenous, intra-arterial, transdermal, sublingual, intramuscular, rectal, transbuccal, intranasal, inhalational, vaginal, intraocular, topical, subcutaneous, intra-adipose, intra-articular, intraperitoneal and intrathecal administrations. In some specific embodiments, the drug is administered orally. The amount of tenofovir or the pharmaceutically acceptable prodrug thereof or the pharmaceutically acceptable salt thereof administered can be determined according to the severity of the disease, the response to the disease, any treatment-related toxicity, and the age and health status of the patient. For example, tenofovir or the pharmaceutically acceptable prodrug thereof or the pharmaceutically acceptable salt thereof may be administered at a daily dose of 0.05 mg to 500 mg. Tenofovir or the pharmaceutically acceptable prodrug thereof or the pharmaceutically acceptable salt thereof may be administered once or more times daily. In some embodiments, tenofovir or the pharmaceutically acceptable prodrug thereof or the pharmaceutically acceptable salt thereof is administered once daily in the form of an oral solid formulation.

The term "about" shall be interpreted as including a range of three standard deviations from the mean value or the standard tolerance range in a specific field. In some embodiments, "about" shall be interpreted as a variation not exceeding 0.5. The term "about" modifies all listed values thereafter. For example, "about 1, 2 and 3" represents "about 1", "about 2" and "about 3".

The term "pharmaceutical combination" refers to combined use of two or more active components in a simultaneous, parallel or sequential manner.

The term "fixed combination" refers to the simultaneous administration of the active components (e.g., the compound of formula I or entecavir or tenofovir) to a subject at a fixed total dose or in a fixed dose proportion, or in the form of a single entity, pharmaceutical composition or formulation. In some embodiments, for example, the active ingredients are present in one tablet, one capsule, or one sachet.

The term "non-fixed combination" refers to the simultaneous, parallel or sequential (without specific time limitation) administration of two or more active components as independent entities (for example, a pharmaceutical composition and a pharmaceutical formulation) to an individual, wherein the active ingredients administered to the individual reach therapeutically effective amounts. Examples of the non-fixed combination can include cocktail therapy, e.g., administration of 2, 3, or more active components. In a non-fixed combination, each of the active components can be packaged, sold, or administered as a fully independent pharmaceutical composition. The "non-fixed combination" further includes combined use of "fixed combinations", or a "fixed combination" and an independent entity of any one or more active components.

The term "pharmaceutical composition" refers to a mixture consisting of one or more of the compounds or the pharmaceutical combinations thereof or the salts thereof of the present application and a pharmaceutically acceptable excipient. The pharmaceutical composition is intended to facilitate the administration of the compound or the pharmaceutical combination thereof of the present application to a subject.

The term "pharmaceutically acceptable excipient" refers to those that do not have a significant irritating effect on an organism and do not impair the biological activity and properties of the active compound. Suitable excipients are well known to those skilled in the art, such as carbohydrate, wax, water-soluble and/or water-swellable polymers, hydrophilic or hydrophobic material, gelatin, oil, solvent, water, and the like.

The pharmaceutical composition of the present application can be prepared by combining the compound of the present application with a suitable pharmaceutically acceptable excipient, and can be formulated, for example, into a solid formulation such as a tablet, a pill, a capsule, and the like.

The pharmaceutical composition of the present application can be manufactured using methods well known in the art, such as conventional mixing, dissolving, granulating, dragee-making, levigating, emulsifying, lyophilizing, and the like.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

A solid oral composition can be prepared by conventional mixing, filling or tableting. For example, it can be obtained by the following method: mixing the active compounds with solid excipients, optionally grinding the resulting mixture, adding additional suitable excipients if desired, and processing the mixture into granules to get the core parts

of tablets or dragees. Suitable excipients include, but are not limited to: binders, diluents, disintegrants, lubricants, glidants, sweeteners or flavoring agents, and the like.

The term "pharmaceutically acceptable salt" refers to salts of the compounds of the present application that are within the definition of "pharmaceutically acceptable".

Unless otherwise specified, terms in the singular shall be deemed to include the plural and vice versa. Unless otherwise specified, the word "a" or "an" refers to "at least one". Unless otherwise stated, "or" is used to mean "and/or".

As used herein, the terms "comprise", "comprises" and "comprising" or equivalents thereof are open-ended statements and mean that elements, components and steps that are not specified may be included in addition to those listed, unless otherwise stated.

The term "single dose" refers to the smallest unit of packaging containing a certain quantity of pharmaceutical product; for example, in a box of seven capsules, each capsule is a single dose; or a vial of injection is a single dose. The term "multiple doses" consists of multiple single doses.

The term "free compound" refers to the compounds of the present application existing in a free state, specifically

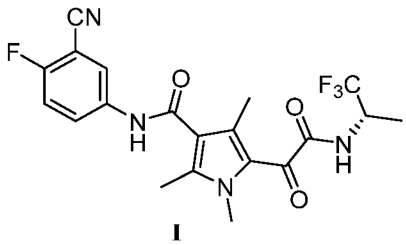

I

,

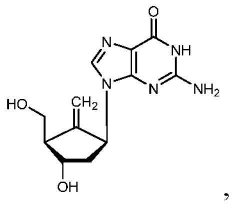

, and

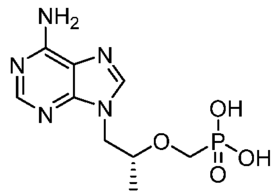

.

The term "solvate" refers to a substance formed by combining the compound of the present application with a pharmaceutically acceptable solvent. The pharmaceutically acceptable solvent includes water, alcohol, and the like. The solvate includes both stoichiometric and non-stoichiometric amounts of solvates.

The term "prodrug" is intended to include any covalently bonded carrier that, when administered to a mammalian subject, releases the active parent drug of the present invention *in vivo.* The prodrug of the present invention is prepared by modifying a functional group present in the compound in such a way that the modification is cleaved into the parent compound in a conventional operation or *in vivo.*

The term "nucleos(t)ide analogue" (NA) or "nucleos(t)ide drug" can be used for the treatment of hepatitis B. Illustratively, NA includes, but is not limited to, entecavir, tenofovir, lamivudine, adefovir, telbivudine, and the like. All patents, patent applications and other identified publications are explicitly incorporated herein by reference for the purpose of description and disclosure. These publications are provided solely because they were disclosed prior to the filing date of the present application. All statements as to the dates of these documents or descriptions as to the contents of these documents are based on the information available to the applicant and do not constitute any admission as to the correctness of the dates or the content of these documents. Moreover, in any country or region, any reference to these publications herein shall not be construed as an admission that the publications form part of the common knowledge in the art.

Administration

The content below is not intended to limit the administration of the pharmaceutical combination of the present application.

The active components in the pharmaceutical combination of the present application can be formulated separately, or some or all of the active components are co-formulated. In one embodiment, the pharmaceutical combination of the present application can be formulated into a pharmaceutical composition which is suitable for a single dose or multiple doses.

The active components in the pharmaceutical combination of the present application can be administered separately, or some or all of the active components are co-administered. The components in the pharmaceutical combination of the present application can be administered in a substantially asynchronous manner, or some or all of the components are administered in a substantially synchronous manner.

The active components in the pharmaceutical combination of the present application can be administered independently, or some or all of the active components are co-administered in various proper routes, including, but not limited to, oral administration or parenteral administration (intravenous, intramuscular, topical or subcutaneous routes). In some embodiments, the active components in the pharmaceutical combination of the present application can be administered independently, or some or all of the active components are co-administered by means of oral administration

or injection, e.g., intravenous injection or intraperitoneal injection.

The active components in the pharmaceutical combination of the present application can be in independent suitable dosage forms, or some or all of the active components are co-formulated in a suitable dosage form, including, but not limited to, tablet, lozenge, pill, capsule (e.g., hard capsule, soft capsule, enteric capsule, and microcapsule), elixir, granule, syrup, injection (intramuscular, intravenous, and intraperitoneal), powder, emulsion, suspension, solution, dispersion, and dosage forms of sustained-release formulations for oral or non-oral administration.

The active components in the pharmaceutical combination of the present application can be formulated independently with a pharmaceutically acceptable carrier and/or excipient, or some or all of the active components are co-formulated with a pharmaceutically acceptable carrier and/or excipient.

The pharmaceutical combination of the present application may further comprise an additional therapeutic agent. In one embodiment, the additional therapeutic agent can be a therapeutic agent known in the art for treating hepatitis B virus infection.

In some embodiments, the effective amounts of the compound of formula I or the pharmaceutically acceptable salt thereof and entecavir or the pharmaceutically acceptable salt thereof or the solvate thereof of the present application can be administered to an individual in need simultaneously, sequentially, or at intervals.

In some embodiments, the effective amounts of the compound of formula I or the pharmaceutically acceptable salt thereof and entecavir or the pharmaceutically acceptable salt thereof or the solvate thereof can be administered to an individual in need by following the same or different administration regimens.

In some embodiments, the compound of formula I or the pharmaceutically acceptable salt thereof is administered thrice every day, twice every day, once every day, once every two days, once every three days, once every four days, once every five days, once every six days, once every week, once every two weeks, or once every three weeks.

In some embodiments, the compound of formula I or the pharmaceutically acceptable salt thereof is administered at a dose of 1-1500 mg, 2-1000 mg, 3-800 mg, 3-600 mg, 4-550 mg, 5-500 mg, 5-450 mg, 5-400 mg, 5-480 mg, 5-460 mg, 5-450 mg, 5-430 mg, 6-400 mg, 7-380 mg, 8-360 mg, 9-340 mg, or 10-320 mg each time, preferably a dose of 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg, 120 mg, 140 mg, 160 mg, 180 mg, 200 mg, 220 mg, 240 mg, 260 mg, 280 mg, 300 mg, 320 mg, 340 mg, 360 mg, 380 mg, 400 mg, or a range formed by any of the above values.

In some embodiments, the dose of the compound of formula I or the pharmaceutically acceptable salt thereof is a dose of 1-1500 mg, 2-1000 mg, 3-800 mg, 3-600 mg, 4-550 mg, 5-500 mg, 5-450 mg, 5-400 mg, 5-480 mg, 5-460 mg, 5-450 mg, 5-430 mg, 6-400 mg, 7-380 mg, 8-360 mg, 9-340 mg, or 10-320 mg per administration, preferably a dose of 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 31 mg, 32 mg, 33 mg, 34 mg, 35 mg, 36 mg, 37 mg, 38 mg, 39 mg, 40 mg, 41 mg, 42 mg, 43 mg, 44 mg, 45 mg, 46 mg, 47 mg, 48 mg, 49 mg, 50 mg, 51 mg, 52 mg, 53 mg, 54 mg, 55 mg, 56 mg, 57 mg, 58 mg, 59 mg, 60 mg, 61 mg, 62 mg, 63 mg, 64 mg, 65 mg, 66 mg, 67 mg, 68 mg, 69 mg, 70 mg, 71 mg, 72 mg, 73 mg, 74 mg, 75 mg, 76 mg, 77 mg, 78 mg, 79 mg, 80 mg, 81 mg, 82 mg, 83 mg, 84 mg, 85 mg, 86 mg, 87 mg, 88 mg, 89 mg, 90 mg, 91 mg, 92 mg, 93 mg, 94 mg, 95 mg, 96 mg, 97 mg, 98 mg, 99 mg, 100 mg, 120 mg, 140 mg, 160 mg, 180 mg, 200 mg, 220 mg, 240 mg, 260 mg, 280 mg, 300 mg, 320 mg, 340 mg, 360 mg, 380 mg, 400 mg, or a range formed by any of the above values.

In some embodiments, the compound of formula I or the pharmaceutically acceptable salt thereof is administered once every day and at a dose of 1-320 mg each time.

In some embodiments, entecavir or the pharmaceutically acceptable salt thereof or the solvate thereof is administered thrice every day, twice every day, once every day, once every two days, once every three days, once every four days, once every five days, once every six days, once every week, once every two weeks, or once every three weeks.

In some embodiments, entecavir or the pharmaceutically acceptable salt thereof or the solvate thereof is administered at a dose of 0.005 mg to 5.0 mg each time, preferably a dose of 0.01 mg, 0.05 mg, 0.1 mg, 0.2 mg, 0.3 mg, 0.4 mg, 0.5 mg, 0.6 mg, 0.7 mg, 0.8 mg, 0.9 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, 4.0 mg, 4.1 mg, 4.2 mg, 4.3 mg, 4.4 mg, 4.5 mg, 4.6 mg, 4.7 mg, 4.8 mg, 4.9 mg, 5.0 mg, or a range formed by any of the above values. In some embodiments, entecavir or the pharmaceutically acceptable salt thereof or the solvate thereof is administered at a dose of 0.05 mg to 5.0 mg, 0.10 mg to 2.0 mg, 0.25 mg to 2.0 mg, or 0.5 mg to 1.0 mg each time.

In some embodiments, the dose of entecavir or the pharmaceutically acceptable salt thereof or the solvate thereof is a dose of 0.005 mg to 5.0 mg per administration, preferably a dose of 0.01 mg, 0.05 mg, 0.1 mg, 0.2 mg, 0.3

mg, 0.4 mg, 0.5 mg, 0.6 mg, 0.7 mg, 0.8 mg, 0.9 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, 4.0 mg, 4.1 mg, 4.2 mg, 4.3 mg, 4.4 mg, 4.5 mg, 4.6 mg, 4.7 mg, 4.8 mg, 4.9 mg, 5.0 mg, or a range formed by any of the above values. In some embodiments, the dose of entecavir or the pharmaceutically acceptable salt thereof or the solvate thereof is a dose of 0.05 mg to 5.0 mg, 0.10 mg to 2.0 mg, 0.25 mg to 2.0 mg, or 0.5 mg to 1.0 mg per administration.

In some embodiments, entecavir or the pharmaceutically acceptable salt thereof or the solvate thereof is administered once every day and at a dose of 0.10 mg to 2.0 mg each time. In some embodiments, entecavir or the pharmaceutically acceptable salt thereof or the solvate thereof can be administered once every day and at a dose of 0.5 mg each time. In some embodiments, the compound of formula I is administered once every day and at a dose of 1 mg to 100 mg each time; entecavir or the pharmaceutically acceptable salt thereof or the solvate thereof is administered once every day and at a dose of 0.10 mg to 2.0 mg each time.

In some embodiments, tenofovir or the pharmaceutically acceptable prodrug thereof or the pharmaceutically acceptable salt thereof is administered once every day, twice every day, or once every two days, or once every three days, or once every four days, or once every five days, or once every six days, or once every seven days. In some embodiments of the present application, tenofovir or the pharmaceutically acceptable prodrug thereof or the pharmaceutically acceptable salt thereof in the pharmaceutical combination can be administered once every day.

In some embodiments, tenofovir or the pharmaceutically acceptable prodrug thereof or the pharmaceutically acceptable salt thereof is administered at a dose of 0.05 mg to 500 mg each time, preferably a dose of 0.1 mg, 0.5 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 35 mg, 40 mg, 45 mg, 50 mg, 55 mg, 56 mg, 57 mg, 58 mg, 59 mg, 60 mg, 65 mg, 66 mg, 67 mg, 68 mg, 70 mg, 71 mg, 72 mg, 73 mg, 74 mg, 75 mg, 80 mg, 85 mg, 90 mg, 91 mg, 92 mg, 93 mg, 94 mg, 95 mg, 100 mg, 101 mg, 102 mg, 103 mg, 104 mg, 105 mg, 110 mg, 111 mg, 112 mg, 113 mg, 114 mg, 115 mg, 120 mg, 125 mg, 130 mg, 131 mg, 132 mg, 133 mg, 134 mg, 135 mg, 136 mg, 137 mg, 138 mg, 139 mg, 140 mg, 145 mg, 150 mg, 155 mg, 160 mg, 165 mg, 170 mg, 175 mg, 180 mg, 181 mg, 182 mg, 183 mg, 184 mg, 185 mg, 190 mg, 200 mg, 250 mg, 300 mg, 350 mg, 400 mg, 450 mg, 500 mg, or a range formed by any of the above values. In some embodiments, tenofovir or the pharmaceutically acceptable prodrug thereof or the pharmaceutically acceptable salt thereof is administered at a dose of 0.05 mg to 200 mg, 0.10 mg to 190 mg, 1 mg to 160 mg, or 10 mg to 150 mg each time.

In some embodiments, the dose of tenofovir or the pharmaceutically acceptable prodrug thereof or the pharmaceutically acceptable salt thereof is a dose of 0.05 mg to 500 mg per administration, preferably a dose of 0.1 mg, 0.5 mg, 1 mg, 2 mg, 3 mg, 4 mg, 5 mg, 6 mg, 7 mg, 8 mg, 9 mg, 10 mg, 11 mg, 12 mg, 13 mg, 14 mg, 15 mg, 16 mg, 17 mg, 18 mg, 19 mg, 20 mg, 21 mg, 22 mg, 23 mg, 24 mg, 25 mg, 26 mg, 27 mg, 28 mg, 29 mg, 30 mg, 35 mg, 40 mg, 45 mg, 50 mg, 55 mg, 56 mg, 57 mg, 58 mg, 59 mg, 60 mg, 65 mg, 66 mg, 67 mg, 68 mg, 70 mg, 71 mg, 72 mg, 73 mg, 74 mg, 75 mg, 80 mg, 85 mg, 90 mg, 91 mg, 92 mg, 93 mg, 94 mg, 95 mg, 100 mg, 101 mg, 102 mg, 103 mg, 104 mg, 105 mg, 110 mg, 111 mg, 112 mg, 113 mg, 114 mg, 115 mg, 120 mg, 125 mg, 130 mg, 131 mg, 132 mg, 133 mg, 134 mg, 135 mg, 136 mg, 137 mg, 138 mg, 139 mg, 140 mg, 145 mg, 150 mg, 155 mg, 160 mg, 165 mg, 170 mg, 175 mg, 180 mg, 181 mg, 182 mg, 183 mg, 184 mg, 185 mg, 190 mg, 200 mg, 250 mg, 300 mg, 350 mg, 400 mg, 450 mg, 500 mg, or a range formed by any of the above values. In some embodiments, the dose of tenofovir or the pharmaceutically acceptable prodrug thereof or the pharmaceutically acceptable salt thereof is a dose of 0.05 mg to 200 mg, 0.10 mg to 190 mg, 1 mg to 160 mg, or 10 mg to 150 mg per administration.

In some embodiments, tenofovir or the pharmaceutically acceptable prodrug thereof or the pharmaceutically acceptable salt thereof is administered once every day and at a dose of 10 mg to 150 mg each time. In some embodiments, tenofovir or the pharmaceutically acceptable prodrug thereof or the pharmaceutically acceptable salt thereof can be administered once every day and at a dose of 15 mg or 135.5 mg each time.

In some embodiments, tenofovir disoproxil fumarate is administered once every day and at a dose of 300 mg each time.

In some embodiments, tenofovir alafenamide is administered once every day and at a dose of 25 mg each time.

In some embodiments, tenofovir alafenamide fumarate is administered once every day and at a dose of 28 mg each time.

In some embodiments, the compound of formula I is administered once every day and at a dose of 1 mg to 320 mg each time; tenofovir or the pharmaceutically acceptable prodrug thereof or the pharmaceutically acceptable salt thereof is administered once every day and at a dose of 10 mg to 150 mg each time.

## Technical effects

The pharmaceutical combination of the present application can significantly reduce the HBV DNA level or other HBV indicators. In addition, compared to monotherapies, the pharmaceutical combination of the present application

demonstrates an enhanced effect and has good tolerability. These indicate that the pharmaceutical combination of the present application has good pharmaceutical value.

## DETAILED DESCRIPTION

For clarity, the present application is further described with the following examples, which are, however, not intended to limit the scope of the present application. All reagents used in the present application are commercially available and can be used without further purification.

### Example 1. HepG2.2.15 Cell Assay

1.1. Methodology

1) HepG2.2.15 cells were plated into a 96-well cell culture plate at $2 \times 10^4$ cells/well, with 100 μL of cell complete medium per well.
2) The cells were then incubated overnight in a cell incubator at 37 °C with 5% $CO_2$.
3) On day 0, fresh complete media containing different compounds were added to a new 96-well plate.
4) Compound formulation and cell treatment:
Monotherapy administration process: Each compound was diluted with DMSO to 100 times the maximum test concentration, then serially 3-fold diluted with DMSO to give 8 points, and added at 2 μL/well, and the mixture was mixed well. The old medium was discarded, and then 100 μL of the well mixed complete medium containing the compound was added.

Combined therapy administration process: The compounds were each diluted with DMSO to 200 times the maximum test concentration and then serially 3-fold diluted with DMSO to give 8 points. Each of the two compounds at the corresponding concentrations was added at 1 μL/well, and the mixture was mixed well. The old medium was discarded, and then 100 μL of the well mixed complete medium containing the compounds was added.
5) On day 6, the cell supernatant was collected, and 5 μL of the supernatant was taken for qPCR detection of viral DNA. A CCK8 reaction solution was added to the original 96-well plate (with each well containing 100 μL of culture medium + 10 μL of CCK) at 110 μL/well. The plate was then incubated in a cell incubator at 37 °C for 0.5 h. The OD values were measured at wavelengths of 450 nm and 630 nm.
6) The administration concentrations are shown in Table 1.

Table 1. Compound concentration distribution (nM)

| Compound | Group | | | | | | | |
|---|---|---|---|---|---|---|---|---|
| | 1 | 2 | 3 | 4 | 5 | 6 | 7 | 8 |
| Compound of formula I (a) | 64.8 | 21.6 | 7.20 | 2.40 | 0.800 | 0.267 | 0.0889 | 0.0296 |
| Entecavir (b) | 8.10 | 2.70 | 0.900 | 0.300 | 0.100 | 0.0333 | 0.0111 | 0.00370 |
| Compound of formula I in combination with entecavir | a1b1 | a2b2 | a3b3 | a4b4 | a5b5 | a6b6 | a7b7 | a8b8 |
| Note: a1b1 represents that in a group of the compound of formula I in combination with entecavir, the administration concentration of the compound of formula I (a) in group 1 and the administration concentration of entecavir (b) in group 1 were used, and so on for others. | | | | | | | | |

7) Supernatant DNA detection

Firstly, an HBV DNA quantitative assay kit (one-step method) from Sansure Biotech Inc. was taken and equilibrated to room temperature. After the reagents in the kit were completely melted, they were mixed well and then centrifuged instantaneously.

Next, 5 μL of a nucleic acid release agent was added to each well of an 8-tube strip, and then a quality control standard, a quantitative standard, and the cell culture supernatant sample treated with the drug for 6 days were each added at 5 μL/well. After the addition, the mixture was mixed well by pipetting 5 times, and then left to stand at room

temperature for 10 min.

Then, a PCR mixed solution (38 μL of a PCR reaction solution + 2 μL of an enzyme mixed solution + 0.2 μL of an internal standard) was prepared according to the number of the wells and added into the wells at 40 μL/well.

The mixture was instantaneously centrifuged and then placed into a Tianlong TL988 qPCR instrument for qPCR detection.

Reaction system: 5 μL of the extraction solution + 5 μL of the sample + 40 μL of the PCR mixed solution (38 μL of the PCR reaction solution + 2 μL of the enzyme mixed solution + 0.2 μL of the internal standard).

Detection channels: FAM channel (sample detection channel, Reportere: FAM, Quencher: None), VIC channel (internal standard detection channel, Reportere: VIC, Quencher: None), and ROX channel (reference detection channel: Passive Reference: ROX).

8) Data processing

HBV DNA inhibition rate (%)=(1 - compound administration group (iU/mL)/vehicle control group (iU/mL))× 100

DAS software was used to analyze the experimental data and calculate the combination indices. The combination indices for 50%, 75%, 90% and 95% inhibition rates were calculated separately, and the average combination index (CIwt) was calculated.

$$\text{Average combination index (CIwt)} = [1 \times CI50 + 2 \times CI75 + 3 \times CI90 + 4 \times CI95] / 10$$

An average combination index of 0.9-1.1 indicates an additive effect, an average combination index of less than 0.9 indicates a synergistic effect, and an average combination index of greater than 1.1 indicates an antagonistic effect.

9) The results are shown in Table 2.

Table 2. CI indices for combination therapy

| Molar ratio of compound of formula **I**/ entecavir | Combination index (CI) | | | | | Average combination index |
|---|---|---|---|---|---|---|
| | 50% Inhibition rate | 75% Inhibition rate | 90% Inhibition rate | 95% Inhibition rate | $R^2$ | (CIwt) |
| 8 | 0.893 | 0.743 | 0.669 | 0.642 | 0.999 | 0.695 |

The average combination index for the combination therapy with the compound of formula I and entecavir against HBV was 0.695, indicating a synergistic effect.

**Example 2. AAV/HBV Mouse Model Experiment**

2.1. Experimental materials

2.1.1. Animals

rAAV8-1.3HBV (source: Beijing FivePlus Molecular Medicine Institute, titer: 1 × $10^{12}$ v.g/mL) was used to establish an AAV/HBV mouse model (species: male, C57BL/6), and the mice were randomized into groups.

2.1.2. Test compound treatment

Solvent: 1% DMSO + 99% PEG-400.

Compounds: a compound of formula I and entecavir.

The compound of formula I was dissolved in DMSO, and PEG-400 was added to prepare a 1 mg/mL clear solution, which was then gradually diluted with the vehicle to 0.3 mg/mL, 0.1 mg/mL, and 0.03 mg/mL. After preparation, the dilutions were stored at room temperature.

Entecavir was dissolved in DMSO to prepare a 1 mg/mL solution, which was then gradually 1000-fold diluted with normal saline (NS) to 0.001 mg/mL. The dilution was aliquoted at 1 mL/tube to serve as a stock solution and stored in a freezer at -20 °C. Each day, one tube was taken, and the solution in the tube was 10-fold diluted with NS to achieve the required administration concentration of 0.0001 mg/mL for daily administration.

2.1.3. rAAV8-1.3HBV

rAAV8-1.3HBV (type D, ayw) was purchased from Beijing FivePlus Gene Technology Co., Ltd., with a titer of $1 \times 10^{12}$ v.g/mL.

2.1.4. Reagents and instruments

Hepatitis B virus nucleic acid assay kit (Sansure Biotech Inc.), high-speed refrigerated centrifuge (Sorvall Legend), and real-time fluorescent quantitative PCR instrument (TL988).

2.2. Methodology

AAV-HBV mouse model: rAAV8-1.3HBV was prepared into a solution with a concentration of $5 \times 10^{11}$ v.g/mL by sterile PBS in advance before injection. Each mouse was injected with 200 μL of the solution via the tail vein, with $1 \times 10^{11}$ v.g per mouse.

Blood sampling before grouping: On day 14 after the virus injection, all infected mice were subjected to blood sampling (approximately 100 μL per mouse) via the orbital vein for serum collection. After the whole blood was left to stand at room temperature for 30 min, serum was collected by centrifugation at 3000 rpm at 4 °C for 10 min. The collected serum was stored at -80 °C for HBV DNA, HBeAg and HBsAg detection.

Compound treatment: All mice were subjected to intragastric administration with the solvent or the compound. The day of the first administration was set as day 0 of the experiment, and the duration of administration was from day 0 to day 27. Entecavir was administered once daily (QD), and the compound of formula I was administered once daily (QD, with the time interval between administrations being 8-16 hr). The administration volume was 10 mL/kg.

Table 3. Administration regimen

| Group | Dose (mg/kg) | Route of administration | Administration interval | Time of administration |
|---|---|---|---|---|
| i. | Solvent | i.g. | BID | Day 0 to day 27 |
| ii. | Entecavir 0.001 mg/kg | | QD | |
| iii. | Compound of formula **I** 1 mg/kg | | QD | |
| iv. | Compound of formula **I** 10 mg/kg | | QD | |
| v. | Entecavir 0.001 mg/kg | | QD | |
| | Compound of formula **I** 1 mg/kg | | QD | |
| vi. | Entecavir 0.001 mg/kg | | QD | |
| | Compound of formula **I** 10 mg/kg | | QD | |
| Note: i.g.: intragastric; QD: administration once daily; BID: administration twice daily. The administration of the combination therapy group was consistent with that of the monotherapy groups. | | | | |

Non-endpoint blood sampling: On days 7, 14, 21, 28 and 35, serum was collected by blood sampling (approximately 100 μL per mouse) via the orbital vein for HBV DNA detection.

Experimental endpoint: The mice in groups i-iii and v were euthanized on day 28. The experimental endpoint for groups iv and vi was on day 42. Serum was collected from each mouse by cardiac blood sampling for HBV DNA detection.

2.3. Sample analysis

The experimental method referred to the instructions of the hepatitis B virus nucleic acid assay kit. 5 μL of the nucleic acid release agent was added into each PCR reaction tube, and then 5 μL of the test serum, a negative control,

a positive control and a quantitative reference substance were added separately. The mixture was then lysed for 10 min, and then 40 μL of PCR-mix was added, achieving a total volume of 50 μL in the system. Reaction conditions: 50 °C for 2 min, 1 cycle; 94 °C for 5 min, 1 cycle; 94 °C for 15 s, 57 °C for 30 s, 45 cycles; 25 °C for 10 s, 1 cycle. 2.4. Results in Tables 4-1 and 4-2

Table 4-1. Detection results of HBV DNA in mouse serum

| Group No. | HBV DNA (log10, copy/μL) at a certain time (day) after administration * | | | | |
|---|---|---|---|---|---|
| | -2 | 7 | 14 | 21 | 28 |
| i. | 7.84±0.36 | 6.15±0.50 | 7.11±0.75 | 7.20±0.56 | 7.90±0.33 |
| ii. | 7.78±0.28 | 5.17±0.54** | 5.30±0.30*** | 5.22±0.46*** | 5.46±0.45*** |
| iii. | 7.71±0.27 | 6.16±0.36 | 5.48±0.34*** | 5.23±0.33*** | 5.42±0.38*** |
| v. | 7.88±0.22 | 4.66±0.24*** | 3.90±0.24*** | 3.5 6±0.31*** | 3.47±0.59*** |

Table 4-2. Detection results of HBV DNA in mouse serum

| Group No. | HBV DNA (log10, copy/μL) at a certain time (day) after administration* | | | | | | |
|---|---|---|---|---|---|---|---|
| | -2 | 7 | 14 | 21 | 28 | 35 | 42 |
| iv. | 7.97±0.22 | 4.72±0.86** | 3.97±0.45*** | 3.97±0.31*** | 3.75±0.35*** | 3.77±0.45 | 5.18±0.74 |
| vi. | 7.89±0.32 | 4.38±0.35*** | 3.59±0.24*** | 3.42±0.25*** | 3.29±0.40*** | 3.57±0.36 | 4.73±0.24 |
| Note: *Mean ± standard error. Compared to the solvent group, *P < 0.05, **P < 0.01, ***P < 0.001. | | | | | | | |

The above results show that, compared to the solvent group, the inhibitory effect of the combination therapy with the compound of formula **I** and entecavir on serum HBV DNA was remarkable and significantly superior to that of the monotherapy.

**Example 3**

1. Test compounds

Compound of formula I: prepared into capsules with strengths of 10 mg and 50 mg.
Entecavir: entecavir tablets, with a strength of 0.5 mg/tablet (e.g., Runzhong® from Chia Tai Tianqing Pharmaceutical Group Co., Ltd.).
Placebo: placebo of the compound of formula **I.**
Tenofovir: tenofovir disoproxil fumarate, with a strength of 300 mg/tablet; tenofovir alafenamide fumarate, with a strength of 25 mg/tablet.

2. Enrolled subjects

The subjects should meet the following criteria:

1) aged 18-75 years (inclusive), male or female;
2) serological virology criteria: having positive serum HBsAg for 6 consecutive months or more or evidence of chronic hepatitis B for 6 months; 1000 IU/mL ≤ quantitative HBsAg ≤ 40000 IU/mL; and
3) no significant cirrhosis as determined by a researcher.

**The treated patients should meet the following criteria:**

4) the subjects must have received the same NA treatment (ETV/TAF) at the same dose for 12 months or more prior to screening;
5) medical history records of HBV DNA inhibition less than the lower limit of normal 6 months prior to enrollment,

with HBV DNA inhibition during screening defined as < LLOQ; and
6)

$$ALT \leq 5 \times ULN.$$

**The patients to be treated for the first time should meet the following criteria:**
7) the subjects must not have received any CHB treatment at the time of screening, i.e.,

♦ the subjects had never received treatment with an HBV antiviral drug (NA) or an interferon (IFN) drug, or
♦ the subjects had received treatment with an HBV antiviral drug (NA) or an IFN drug (used irregularly), with no more than 3 months from the first use of the study drug, or
♦ the subjects had not received treatment with an HBV antiviral drug (NA) or an IFN drug within 6 months prior to screening
Note: if the subjects had received treatment with an anti-HBV drug for more than 3 months prior to screening, the sponsor should be contacted to discuss this matter.
8) for HBeAg-positive chronic hepatitis B patients, HBV DNA > $10^5$ copies/mL (or > 20,000 IU/mL); or for HBeAg-negative patients, HBV DNA > $10^4$ copies/mL (or > 2,000 IU/mL); and

$$1.0 \times ULN \leq ALT \leq 5 \times ULN.$$

3. Administration regimen

The capsules/placebo of the compound of formula I were administered once daily on an empty stomach (at least 2 hours before or after a meal), with the timing of each administration being approximately consistent.
Entecavir was administered once daily on an empty stomach (at least 2 hours before or after a meal), with the timing of each administration being approximately consistent.
Tenofovir was administered once daily under a postprandial condition (within 1 hour after a meal), with the timing of each administration being approximately consistent.

4. Efficacy indicators

The indicators include HBV markers (HBsAg, HBsAb, HBeAg, HBeAb, HBcAb, quantitative HBV DNA, HBV RNA, and HBcrAg).

## Claims

1. A pharmaceutical combination, comprising a capsid protein inhibitor or a pharmaceutically acceptable salt thereof and a reverse transcriptase inhibitor or a pharmaceutically acceptable prodrug thereof, a pharmaceutically acceptable salt thereof or a solvate thereof.

2. A pharmaceutical combination, comprising a compound of formula **I** or a pharmaceutically acceptable salt thereof and a reverse transcriptase inhibitor or a pharmaceutically acceptable prodrug thereof, a pharmaceutically acceptable salt thereof or a solvate thereof:

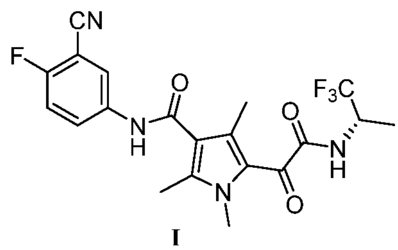

I

.

3. The pharmaceutical combination according to claim 2, wherein the reverse transcriptase inhibitor or the pharmaceutically acceptable prodrug thereof, the pharmaceutically acceptable salt thereof or the solvate thereof is selected from the group consisting of entecavir or a pharmaceutically acceptable salt thereof or a solvate thereof, and tenofovir or a pharmaceutically acceptable prodrug thereof or a pharmaceutically acceptable salt thereof.

**4.** The pharmaceutical combination according to claim 3, wherein entecavir or the pharmaceutically acceptable salt thereof or the solvate thereof is selected from the group consisting of entecavir maleate, entecavir monomaleate, entecavir hydrate, entecavir hemihydrate to dihydrate, and entecavir monohydrate; optionally, entecavir or the pharmaceutically acceptable salt thereof or the solvate thereof is selected from entecavir monomaleate monohydrate.

**5.** The pharmaceutical combination according to claim 3 or 4, wherein the average daily dose ratio of the compound of formula **I** or the pharmaceutically acceptable salt thereof to entecavir or the pharmaceutically acceptable salt thereof or the solvate thereof, calculated based on the weight of free compounds, is selected from 1000:1 to 1:1000; optionally, the average daily dose ratio of the compound of formula **I** or the pharmaceutically acceptable salt thereof to entecavir or the pharmaceutically acceptable salt thereof or the solvate thereof, calculated based on the weight of free compounds, is selected from the group consisting of 1000:1, 950:1, 900:1, 850:1, 800:1, 750:1, 700:1, 650:1, 640:1, 630:1, 620:1, 610:1, 600:1, 550:1, 500:1, 450:1, 400:1, 350:1, 300:1, 250:1, 200:1, 150:1, 100:1, 90:1, 80:1, 70:1, 60:1, 50:1, 40:1, 30:1, 20:1, 10:1, 1:0.01, 1:0.02, 1:0.03, 1:0.04, 1:0.05, 1:0.06, 1:0.07, 1:0.08, 1:0.09, 1:0.1, 1:0.5, 1:0.6, 1:0.7, 1:0.8, 1:0.9, 1:1, 1:10, 1:20, 1:30, 1:40, 1:50, 1:60, 1:70, 1:80, 1:90, 1:100, 1:150, 1:200, 1:250, 1:300, 1:350, 1:400, 1:450, 1:500, 1:550, 1:600, 1:650, 1:700, 1:750, 1:800, 1:850, 1:900, 1:950 and 1:1000 or from a range formed by any of the above ratios.

**6.** The pharmaceutical combination according to any one of claims 3-5, wherein entecavir or the pharmaceutically acceptable salt thereof or the solvate thereof is capable of being administered thrice every day, twice every day, once every day, once every two days, once every three days, once every four days, once every five days, once every six days, once every week, once every two weeks, or once every three weeks.

**7.** The pharmaceutical combination according to any one of claims 3-6, wherein entecavir or the pharmaceutically acceptable salt thereof or the solvate thereof is administered at a dose of 0.005 mg to 5.0 mg each time; preferably, entecavir or the pharmaceutically acceptable salt thereof or the solvate thereof is administered at a dose of 0.01 mg, 0.05 mg, 0.1 mg, 0.2 mg, 0.3 mg, 0.4 mg, 0.5 mg, 0.6 mg, 0.7 mg, 0.8 mg, 0.9 mg, 1.0 mg, 1.1 mg, 1.2 mg, 1.3 mg, 1.4 mg, 1.5 mg, 1.6 mg, 1.7 mg, 1.8 mg, 1.9 mg, 2.0 mg, 2.1 mg, 2.2 mg, 2.3 mg, 2.4 mg, 2.5 mg, 2.6 mg, 2.7 mg, 2.8 mg, 2.9 mg, 3.0 mg, 3.1 mg, 3.2 mg, 3.3 mg, 3.4 mg, 3.5 mg, 3.6 mg, 3.7 mg, 3.8 mg, 3.9 mg, 4.0 mg, 4.1 mg, 4.2 mg, 4.3 mg, 4.4 mg, 4.5 mg, 4.6 mg, 4.7 mg, 4.8 mg, 4.9 mg, 5.0 mg, or a range formed by any of the above values each time.

**8.** The pharmaceutical combination according to claim 3, wherein the pharmaceutically acceptable prodrug of tenofovir comprises tenofovir disoproxil, tenofovir alafenamide, or tenofovir amibufenamide;

optionally, the pharmaceutically acceptable salt of tenofovir or the pharmaceutically acceptable prodrug thereof is selected from the group consisting of a fumarate, an orotate, a disulfonate, a phosphate, a succinate, and an aspartate;
optionally, tenofovir or the pharmaceutically acceptable prodrug thereof or the pharmaceutically acceptable salt thereof is selected from the group consisting of tenofovir, tenofovir alafenamide fumarate, tenofovir disoproxil fumarate, tenofovir disoproxil orotate, tenofovir disoproxil hemidisulfonate, tenofovir disoproxil phosphate, tenofovir disoproxil succinate, tenofovir disoproxil aspartate, and tenofovir amibufenamide fumarate.

**9.** The pharmaceutical combination according to claim 3 or 8, wherein the average daily dose ratio of the compound of formula I or the pharmaceutically acceptable salt thereof to tenofovir or the pharmaceutically acceptable prodrug thereof or the pharmaceutically acceptable salt thereof, calculated based on the weight of corresponding free compounds, is selected from 500:1 to 1:1000; optionally, the average daily dose ratio of the compound of formula **I** or the pharmaceutically acceptable salt thereof to tenofovir or the pharmaceutically acceptable prodrug thereof or the pharmaceutically acceptable salt thereof, calculated based on the weight of corresponding free compounds, is selected from the group consisting of 500:1, 450:1, 400:1, 350:1, 300:1, 250:1, 200:1, 150:1, 100:1, 95:1, 90:1, 85:1, 80:1, 75:1, 70:1, 65:1, 60:1, 55:1, 50:1, 45:1, 40:1, 39:1, 38:1, 37:1, 36:1, 35:1, 34:1, 33:1, 32:1, 31:1, 30:1, 29:1, 28:1, 27:1, 26:1, 25:1, 24:1, 23:1, 22:1, 21:1, 20:1, 19:1, 18:1, 17:1, 16:1, 15:1, 10:1, 5:1, 4:1, 3:1, 2:1, 1:1, 1:2, 1:3, 1:4, 1:5, 1:6, 1:7, 1:8, 1:9, 1:10, 1:11, 1:12, 1:13, 1:14, 1:15, 1:16, 1:17, 1:18, 1:19, 1:20, 1:30, 1:40, 1:50, 1:60, 1:70, 1:80, 1:90, 1:100, 1:150, 1:200, 1:250, 1:300, 1:350, 1:400, 1:450, 1:500, 1:550, 1:600, 1:650, 1:700, 1:750, 1:800, 1:850, 1:900, 1:950 and 1:1000 or from a range formed by any of the above ratios.

**10.** The pharmaceutical combination according to any one of claims 3, 8 and 9, wherein tenofovir or the pharmaceutically acceptable prodrug thereof or the pharmaceutically acceptable salt thereof is capable of being administered once every day, twice every day, or once every two days, or once every three days, or once every four days, or once every five days, or once every six days, or once every seven days.

**11.** The pharmaceutical combination according to any one of claims 3 and 8-10, wherein the average daily dose of tenofovir or the pharmaceutically acceptable prodrug thereof or the pharmaceutically acceptable salt thereof is 0.05 mg to 500 mg, or 0.1 mg to 400 mg, or 1 mg to 300 mg, or 5 mg to 200 mg, or 8 mg to 190 mg, or 10 mg to 185 mg, or 14 mg to 140 mg;

optionally, tenofovir disoproxil fumarate is administered at a dose of 300 mg each time;
or tenofovir alafenamide is administered at a dose of 25 mg each time;
or tenofovir alafenamide fumarate is administered at a dose of 28 mg each time.

**12.** The pharmaceutical combination according to any one of claims 1-11, wherein the compound of formula I or the pharmaceutically acceptable salt thereof is capable of being administered once every day, twice every day, once every two days, once every three days, once every four days, once every five days, once every six days, once every week, once every two weeks, or once every three weeks.

**13.** The pharmaceutical combination according to any one of claims 1-12, wherein the compound of formula I or the pharmaceutically acceptable salt thereof is administered at a dose of 1-1500 mg, 2-1000 mg, 3-800 mg, 3-600 mg, 4-550 mg, 5-500 mg, 5-450 mg, 5-400 mg, 5-480 mg, 5-460 mg, 5-450 mg, 5-430 mg, 6-400 mg, 7-380 mg, 8-360 mg, 9-340 mg, or 10-320 mg each time.

**14.** The pharmaceutical combination according to any one of claims 1-13, wherein the pharmaceutical combination is a fixed combination; optionally, the fixed combination is in the form of a solid pharmaceutical composition; optionally, the compound of formula **I** or the pharmaceutically acceptable salt thereof and entecavir or the pharmaceutically acceptable salt thereof or the solvate thereof or tenofovir or the pharmaceutically acceptable prodrug thereof or the pharmaceutically acceptable salt thereof in the fixed combination are present in the same solid pharmaceutical composition.

**15.** The pharmaceutical combination according to any one of claims 1-14, wherein the pharmaceutical combination is a non-fixed combination;

optionally, the compound of formula **I** or the pharmaceutically acceptable salt thereof and entecavir or the pharmaceutically acceptable salt thereof or the solvate thereof or tenofovir or the pharmaceutically acceptable prodrug thereof or the pharmaceutically acceptable salt thereof in the non-fixed combination are each in the form of a solid pharmaceutical composition;
optionally, the compound of formula **I** or the pharmaceutically acceptable salt thereof and entecavir or the pharmaceutically acceptable salt thereof or the solvate thereof or tenofovir or the pharmaceutically acceptable prodrug thereof or the pharmaceutically acceptable salt thereof in the non-fixed combination are each in the form of a solid pharmaceutical composition, and the solid pharmaceutical composition of the compound of formula **I** or the pharmaceutically acceptable salt thereof and the solid pharmaceutical composition of entecavir or the pharmaceutically acceptable salt thereof or the solvate thereof or the solid pharmaceutical composition of tenofovir or the pharmaceutically acceptable prodrug thereof or the pharmaceutically acceptable salt thereof are present in the same sachet;
optionally, the compound of formula I or the pharmaceutically acceptable salt thereof and entecavir or the pharmaceutically acceptable salt thereof or the solvate thereof or tenofovir or the pharmaceutically acceptable prodrug thereof or the pharmaceutically acceptable salt thereof in the non-fixed combination are each in the form of a solid pharmaceutical composition, and the solid pharmaceutical composition of the compound of formula **I** or the pharmaceutically acceptable salt thereof and the solid pharmaceutical composition of entecavir or the pharmaceutically acceptable salt thereof or the solvate thereof or the solid pharmaceutical composition of tenofovir or the pharmaceutically acceptable prodrug thereof or the pharmaceutically acceptable salt thereof are not present in the same sachet.

**16.** Use of the pharmaceutical combination according to any one of claims 1-15 for preparing a medicament for treating hepatitis B virus infection.

**17.** A method for treating hepatitis B virus infection, comprising administering to an individual in need an effective amount of the pharmaceutical combination according to any one of claims 1-15.

**18.** A pharmaceutical combination for use in treating hepatitis B virus infection, wherein the pharmaceutical combination is selected from the pharmaceutical combination according to any one of claims 1-15.

**19.** Use of the pharmaceutical combination according to any one of claims 1-15 for treating hepatitis B virus infection.

**20.** A kit for use in treating hepatitis B virus infection, comprising: (a) a first pharmaceutical composition comprising a compound of formula I or a pharmaceutically acceptable salt thereof as an active ingredient; and (b) a second pharmaceutical composition comprising a reverse transcriptase inhibitor or a pharmaceutically acceptable prodrug thereof, a pharmaceutically acceptable salt thereof or a solvate thereof as an active ingredient; and optionally (c) a package insert for combined use of the compound of formula I or the pharmaceutically acceptable salt thereof and the reverse transcriptase inhibitor or the pharmaceutically acceptable prodrug thereof, the pharmaceutically acceptable salt thereof or the solvate thereof:

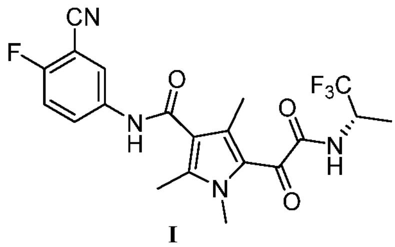

I

.

**21.** The kit according to claim 20, wherein the reverse transcriptase inhibitor or the pharmaceutically acceptable prodrug thereof, the pharmaceutically acceptable salt thereof or the solvate thereof is selected from the group consisting of: entecavir or a pharmaceutically acceptable salt thereof or a solvate thereof, and tenofovir or a pharmaceutically acceptable prodrug thereof or a pharmaceutically acceptable salt thereof.

**INTERNATIONAL SEARCH REPORT**

International application No. **PCT/CN2022/123799**

**A. CLASSIFICATION OF SUBJECT MATTER**

A61K 31/40(2006.01)i; A61P 31/12(2006.01)i; A61K 31/513(2006.01)i; A61K 31/675(2006.01)i

According to International Patent Classification (IPC) or to both national classification and IPC

**B. FIELDS SEARCHED**

Minimum documentation searched (classification system followed by classification symbols)

A61K, A61P

Documentation searched other than minimum documentation to the extent that such documents are included in the fields searched

Electronic data base consulted during the international search (name of data base and, where practicable, search terms used)

WPABSC; CNTXT; ENTXTC; STN; ISI Web of science, CNKI: wo2020156494, wo2021058002, wo2019185016, 恩替卡韦, 替诺福韦, 乙肝, 病毒, 逆转录酶

**C. DOCUMENTS CONSIDERED TO BE RELEVANT**

| Category* | Citation of document, with indication, where appropriate, of the relevant passages | Relevant to claim No. |
|---|---|---|
| X | WO 2021058002 A1 (CHIA TAI TIANQING PHARMACEUTICAL GROUP CO., LTD.) 01 April 2021 (2021-04-01)<br>description, page 1, paragraph 2 to page 2, paragraph 3, and embodiment 2 | 1-2, 12, 13, 16-20 |
| A | WO 2019185016 A1 (CHIA TAI TIANQING PHARMACEUTICAL GROUP CO., LTD.) 03 October 2019 (2019-10-03)<br>abstract, description, page 1, paragraphs 3-4; and claims 1-21 | 1-21 |
| A | WO 2020156494 A1 (CHIA TAI TIANQING PHARMACEUTICAL GROUP CO., LTD.) 06 August 2020 (2020-08-06)<br>abstract, description, paragraphs 2-4, and claims 1-15 | 1-21 |
| A | CN 102389400 A (XIE ANYUN) 28 March 2012 (2012-03-28)<br>abstract, and claims 1-10 | 1-21 |
| A | CN 101756890 A (TIANJIN PACIFIC PHARMACEUTICAL CO., LTD.) 30 June 2010 (2010-06-30)<br>abstract, and claims 1-6 | 1-21 |
| A | CN 103705478 A (ZHEJIANG HUAHAI PHARMACEUTICAL CO., LTD.) 09 April 2014 (2014-04-09)<br>abstract, and claims 1-9 | 1-21 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 December 2022** | **15 December 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

International application No.
**PCT/CN2022/123799**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **17, 19**
because they relate to subject matter not required to be searched by this Authority, namely:

[1] Claims 17 and 19 respectively relate to a method for or use of treatment of hepatitis B virus infection that comprises a process of treating a disease by a drug, belong to a disease treatment method and do not comply with PCT Rule 39.1(iv). The present search was formed on the basis of a use thereof in the preparation of a drug.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (January 2015)

**INTERNATIONAL SEARCH REPORT**
**Information on patent family members**

International application No.
**PCT/CN2022/123799**

| Patent document cited in search report | Publication date (day/month/year) | Patent family member(s) | Publication date (day/month/year) |
|---|---|---|---|
| WO 2021058002 A1 | 01 April 2021 | CA 3156070 A1 | 01 April 2021 |
| | | US 2022363634 A1 | 17 November 2022 |
| | | CN 114430736 A | 03 May 2022 |
| | | AU 2020355384 A1 | 05 May 2022 |
| | | EP 4036078 A1 | 03 August 2022 |
| WO 2019185016 A1 | 03 October 2019 | EP 3778569 A1 | 17 February 2021 |
| | | PH 12020551616 A1 | 16 August 2021 |
| | | CN 111868026 A | 30 October 2020 |
| | | AU 2019241321 A1 | 08 October 2020 |
| | | SG 11202009150 R A | 29 October 2020 |
| | | US 2021017154 A1 | 21 January 2021 |
| | | JP 2021519325 A | 10 August 2021 |
| | | KR 20200136994 A | 08 December 2020 |
| | | CA 3094022 A1 | 03 October 2019 |
| | | EP 3778569 A4 | 29 December 2021 |
| | | CN 111868026 B | 25 November 2022 |
| WO 2020156494 A1 | 06 August 2020 | CN 113365999 A | 07 September 2021 |
| CN 102389400 A | 28 March 2012 | CN 102389400 B | 27 March 2013 |
| CN 101756890 A | 30 June 2010 | None | |
| CN 103705478 A | 09 April 2014 | CN 103705478 B | 07 February 2020 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111172126
- WO 2019185016 A
- WO 2019165374 A
- WO 2019241292 A
- WO 2021119081 A
- WO 2020156494 A
- WO 2019185016 A1